# EUROPEAN PATENT APPLICATION

(11) **EP 2 255 830 A1**
(43) Date of publication of application: **01.12.2010**
(21) Application number: 09717592.1
(22) Date of filing: 02.03.2009
(51) Int. Cl.: A61K 45/06, A61K 31/436, A61K 31/4545, A61K 31/519, A61K 31/5377, A61P 35/00, A61P 43/00

(54) **CONCOMITANT DRUG**

(30) Priority: 03.03.2008 JP 2008052615
(71) Applicant: Takeda Pharmaceutical Company Limited, Osaka 541-0045 (JP)
(72) Inventor: OHTA, Yoshikazu, Tsukuba-shi Ibaraki 300-4293 (JP); TAKAGI, Shinji, Tsukuba-shi Ibaraki 300-4293 (JP); YAGUCHI, Masahiro, Tsukuba-shi Ibaraki 300-4293 (JP)
(74) Representative: Jones, Nicholas Andrew
(86) International application number: PCT/JP2009/053832
(87) International publication number: WO 2009/110415

(57) **Abstract**

Provided is a combination drug. The present invention provides a pharmaceutical agent comprising (1) a HER2 inhibitor having a pyrrolopyrimidine skeleton or pyrazolopyrimidine skeleton, and (2) not less than one pharmaceutical agent selected from an mTOR inhibitor, a PI3 kinase inhibitor and a cMet inhibitor in combination.

## Description

### Technical Field

The present invention relates to a pharmaceutical agent comprising (1) a HER2 inhibitor having a pyrrolopyrimidine skeleton or pyrazolopyrimidine skeleton, and (2) not less than one pharmaceutical agent selected from an mTOR inhibitor, a PI3 kinase inhibitor and a cMet inhibitor in combination, and a thymidine synthase production inhibitor comprising a compound having a particular pyrrolopyrimidine skeleton or pyrazolopyrimidine skeleton.

### (Background of the Invention)

Patent document 1 describes a HER2 inhibitor having a pyrrolopyrimidine skeleton or pyrazolopyrimidine skeleton.
In addition, patent document 2 describes a combined use of a compound represented by the formula:

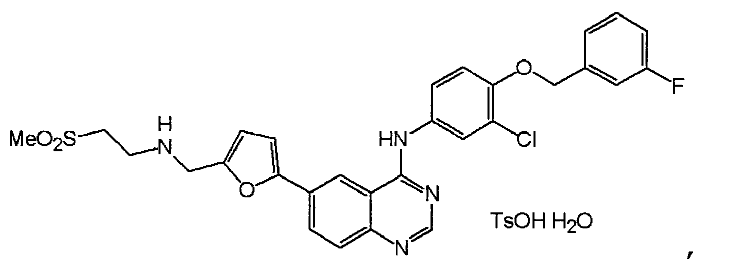

which is a HER2 inhibitor, trastuzumab and the like for the treatment of breast cancer.
patent document 1: W02005/010451
patent document 2: W02005/120512

### Disclosure of the Invention

### Problems to be Solved by the Invention

The present invention aims to provide a pharmaceutical agent comprising (1) a HER2 inhibitor having a pyrrolopyrimidine skeleton or pyrazolopyrimidine skeleton, and (2) not less than one pharmaceutical agent selected from an mTOR inhibitor, a PI3 kinase inhibitor and a cMet inhibitor in combination, which is useful as an agent for the prophylaxis or treatment of cancer, (hereinafter sometimes to be abbreviated as the combination drug of the present invention), and a thymidine synthase production inhibitor comprising a compound having a particular pyrrolopyrimidine skeleton or pyrazolopyrimidine skeleton (hereinafter sometimes to be abbreviated as the thymidine synthase production inhibitor of the present invention).

### Means of Solving the Problems

As a result of the intensive studies, the present inventors have found that a combined use of (1) a HER2 inhibitor having a pyrrolopyrimidine skeleton or pyrazolopyrimidine skeleton, and (2) not less than one pharmaceutical agent selected from an mTOR inhibitor, a PI3 kinase inhibitor and a cMet inhibitor shows a more significant anti-cancer action than use thereof as a single agent and other combination pharmaceutical agents, and that a compound having a particular pyrrolopyrimidine skeleton or pyrazolopyrimidine skeleton shows a thymidine synthase production inhibitory action. Further studies have resulted in the completion of the present invention.

Accordingly, the present invention relates to
[1] a pharmaceutical agent comprising (1) a HER2 inhibitor having a pyrrolopyrimidine skeleton or pyrazolopyrimidine skeleton and (2) not less than one pharmaceutical agent selected from an mTOR inhibitor, a PI3 kinase inhibitor and a cMet inhibitor in combination;
[2] the pharmaceutical agent of the above-mentioned [1], wherein the HER2 inhibitor having a pyrrolopyrimidine skeleton or pyrazolopyrimidine skeleton is a compound represented by the formula:

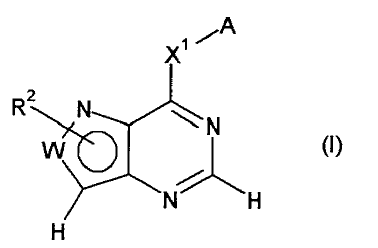

wherein
W is C(R¹) or N,
A is an optionally substituted aryl group or an optionally substituted heteroaryl group,
X¹ is -NR³-Y¹-, -O-, -S-, -SO-, -SO₂- or -CHR³- wherein R³ is a hydrogen atom or an optionally substituted aliphatic hydrocarbon group, or R³ is optionally bonded to a carbon atom or a hetero atom on the aryl group or the heteroaryl group for A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and
R² is a hydrogen atom or an optionally substituted group bonded via a carbon atom or a sulfur atom,
or R¹ and R², or R² and R³ are optionally bonded to each other to form an optionally substituted ring structure, except compounds represented by the formulas

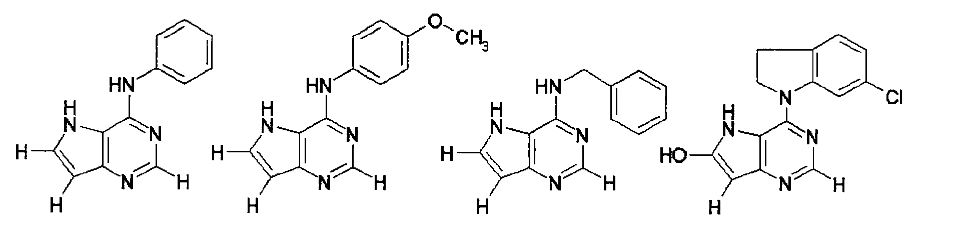

(sometimes to be referred to as compound (I) in the present specification) or a salt thereof or a prodrug thereof;
[3] the pharmaceutical agent of the above-mentioned [1], wherein the HER2 inhibitor having a pyrrolopyrimidine skeleton or pyrazolopyrimidine skeleton is a compound represented by the formula:

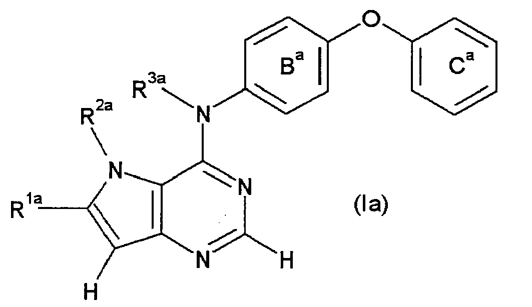

wherein
R^{1a} is a hydrogen atom or an optionally substituted group bonded via a carbon atom, a nitrogen atom or an oxygen atom,
R^{2a} is an optionally substituted group bonded via a carbon atom or a sulfur atom,
or R^{1a} and R^{2a}, or R^{2a} and R^{3a} are optionally bonded to each other to form an optionally substituted ring structure,
R^{3a} is a hydrogen atom or an optionally substituted aliphatic hydrocarbon group, or R^{3a} is optionally bonded to a carbon atom of the adjacent phenyl group to form an optionally substituted ring structure,
B^{a} is an optionally substituted benzene ring, and
C^{a} is an optionally substituted C₆₋₁₈ aryl group, (sometimes to be referred to as compound (Ia) in the present specification) or a salt thereof or a prodrug thereof;
[4] the pharmaceutical agent of the above-mentioned [1], wherein the HER2 inhibitor having a pyrrolopyrimidine skeleton or pyrazolopyrimidine skeleton is N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutaneamide or a salt thereof;
[5] the pharmaceutical agent of the above-mentioned [1], wherein the mTOR inhibitor is rapamycin;
[6] the pharmaceutical agent of the above-mentioned [1], wherein the PI3 kinase inhibitor is PI-103;
[7] the pharmaceutical agent of the above-mentioned [1], wherein the cMet inhibitor is PF2341066;
[8] the pharmaceutical agent of the above-mentioned [1], which is an agent for the prophylaxis or treatment of cancer;
[9] the pharmaceutical agent of the above-mentioned [8], wherein the cancer is breast cancer, ovarian cancer, prostate cancer, lung cancer, pancreatic cancer, kidney cancer, colorectal cancer, small intestinal cancer, esophagus cancer or gastric cancer;
[10] a method for the prophylaxis or treatment of cancer in a mammal, comprising administering (1) an effective amount of a HER2 inhibitor having a pyrrolopyrimidine skeleton or pyrazolopyrimidine skeleton, and (2) an effective amount of not less than one pharmaceutical agent selected from an mTOR inhibitor, a PI3 kinase inhibitor and a cMet inhibitor to the mammal;
[11] use of (1) a HER2 inhibitor having a pyrrolopyrimidine skeleton or pyrazolopyrimidine skeleton, and (2) not less than one pharmaceutical agent selected from an mTOR inhibitor, a PI3 kinase inhibitor and a cMet inhibitor, for the production of an agent for the prophylaxis or treatment of cancer;
[12] a thymidine synthase production inhibitor comprising a compound represented by the formula:

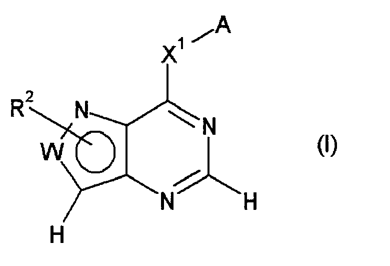

wherein
W is C(R¹) or N,
A is an optionally substituted aryl group or an optionally substituted heteroaryl group,
X¹ is -NR³-Y¹-, -O-, -S-, -SO-, -SO₂- or -CHR³-wherein R³ is a hydrogen atom or an optionally substituted aliphatic hydrocarbon group, or R³ is optionally bonded to a carbon atom or a hetero atom on the aryl group or the heteroaryl group for A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and
R² is a hydrogen atom or an optionally substituted group bonded via a carbon atom or a sulfur atom,
or R¹ and R², or R² and R³ are optionally bonded to each other to form an optionally substituted ring structure, except compounds represented by the formulas

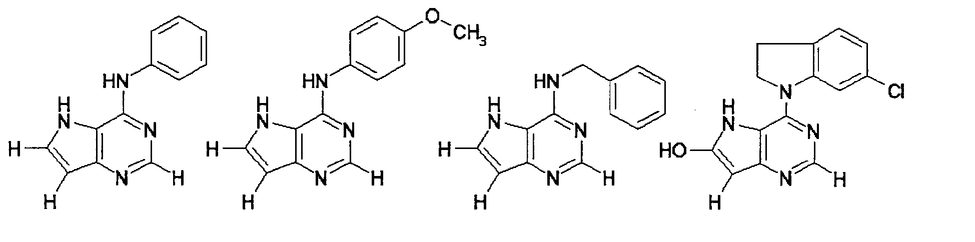

or a salt thereof or a prodrug thereof;
[13] a method of inhibiting thymidine synthase production, comprising administering an effective amount of a compound represented by the formula:

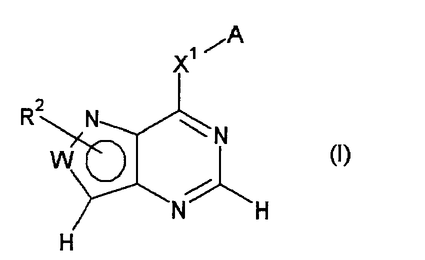

wherein
W is C(R¹) or N,
A is an optionally substituted aryl group or an optionally substituted heteroaryl group,
X¹ is -NR³-Y¹-, -O-, -S-, -SO-, -SO₂- or -CHR³-wherein R³ is a hydrogen atom or an optionally substituted aliphatic hydrocarbon group, or R³ is optionally bonded to a carbon atom or a hetero atom on the aryl group or the heteroaryl group for A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and
R² is a hydrogen atom or an optionally substituted group bonded via a carbon atom or a sulfur atom,
or R¹ and R², or R² and R³ are optionally bonded to each other to form an optionally substituted ring structure, except compounds represented by the formulas

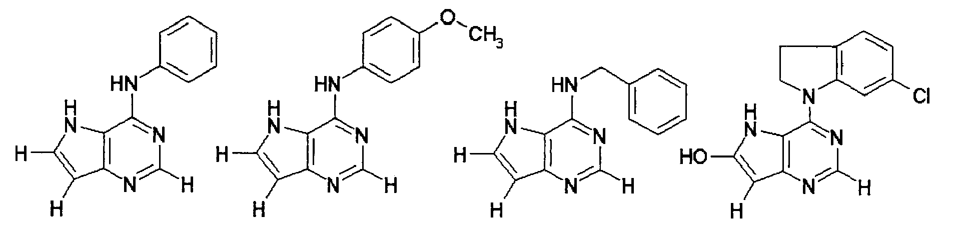

or a salt thereof or a prodrug thereof to a mammal;
[14] use of a compound represented by the formula:

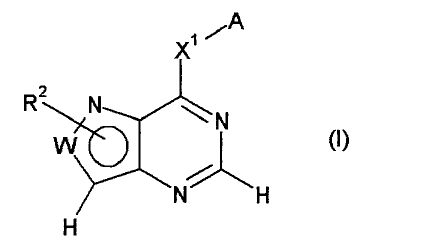

wherein
W is C(R¹) or N,
A is an optionally substituted aryl group or an optionally substituted heteroaryl group,
X¹ is -NR³-Y¹-, -O-, -S-, -SO-, -SO₂- or -CHR³-wherein R³ is a hydrogen atom or an optionally substituted aliphatic hydrocarbon group, or R³ is optionally bonded to a carbon atom or a hetero atom on the aryl group or the heteroaryl group for A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and
R² is a hydrogen atom or an optionally substituted group bonded via a carbon atom or a sulfur atom,
or R¹ and R², or R² and R³ are optionally bonded to each other to form an optionally substituted ring structure, except compounds represented by the formulas

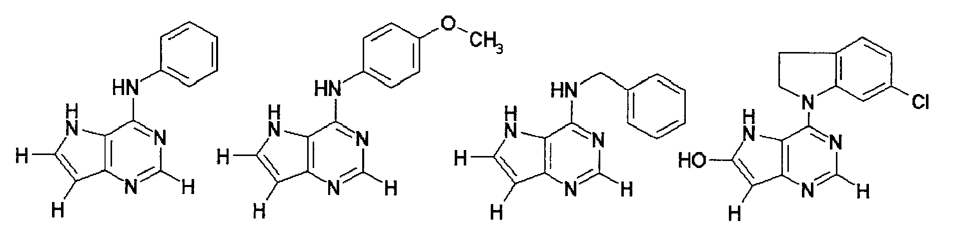

or a salt thereof or a prodrug thereof, for the production of a thymidine synthase production inhibitor; and the like.

In addition, the present invention also relates to
[15] the pharmaceutical agent of the above-mentioned [1], wherein the HER2 inhibitor having a pyrrolopyrimidine skeleton or pyrazolopyrimidine skeleton is a compound represented by the formula:

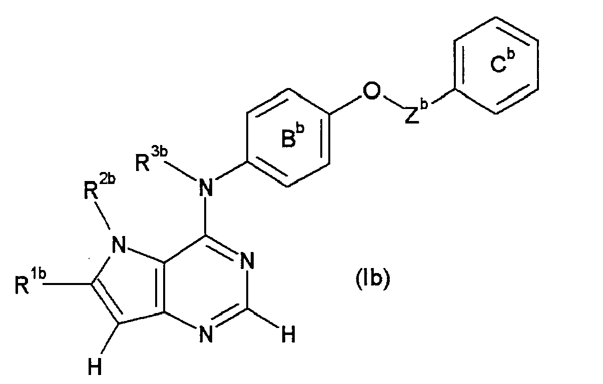

wherein
R^{1b} is a hydrogen atom or an optionally substituted group bonded via a carbon atom, a nitrogen atom or an oxygen atom,
R^{2b} is an optionally substituted group bonded via a carbon atom or a sulfur atom,
or R^{1b} and R^{2b}, or R^{2b} and R^{3b} are optionally bonded to each other to form an optionally substituted ring structure,
R^{3b} is a hydrogen atom or an optionally substituted aliphatic hydrocarbon group, or R^{3b} is optionally bonded to a carbon atom of the adjacent phenyl group to form an optionally substituted ring structure,
B^{b} is an optionally substituted benzene ring,
C^{b} is an optionally substituted C₆₋₁₈ aryl group, and
Z^{b} is an optionally substituted C₁₋₃ alkylene group (sometimes to be referred to as compound (Ib) in the present specification) or a salt thereof or a prodrug thereof;
[16] the pharmaceutical agent of the above-mentioned [1], wherein the HER2 inhibitor having a pyrrolopyrimidine skeleton or pyrazolopyrimidine skeleton is a compound represented by the formula:

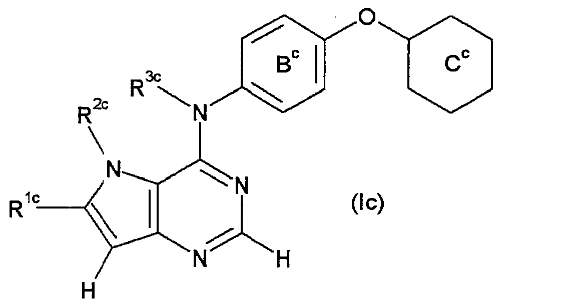

wherein
R^{1c} is a hydrogen atom or an optionally substituted group bonded via a carbon atom, a nitrogen atom or an oxygen atom,
R^{2c} is an optionally substituted group bonded via a carbon atom or a sulfur atom,
or R^{1c} and R^{2c}, or R^{2c} and R^{3c} are optionally bonded to each other to form an optionally substituted ring structure,
R^{3c} is a hydrogen atom or an optionally substituted aliphatic hydrocarbon group, or R^{3c} is optionally bonded to a carbon atom of the adjacent phenyl group to form an optionally substituted ring structure,
B^{c} is an optionally substituted benzene ring, and
C^{c} is an optionally substituted heterocyclic group (sometimes to be referred to as compound (Ic) in the present specification) or a salt thereof or a prodrug thereof;
[17] the pharmaceutical agent of the above-mentioned [1], wherein the HER2 inhibitor having a pyrrolopyrimidine skeleton or pyrazolopyrimidine skeleton is a compound represented by the formula:

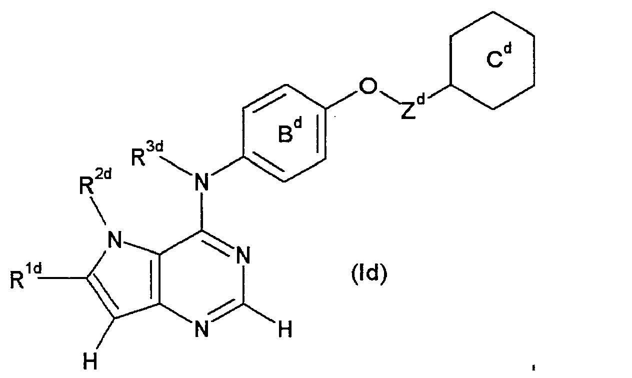

wherein
R^{1d} is a hydrogen atom or an optionally substituted group bonded via a carbon atom, a nitrogen atom or an oxygen atom,
R^{2d} is an optionally substituted group bonded via a carbon atom or a sulfur atom,
or R^{1d} and R^{2d}, or R^{2d} and R^{3d} are optionally bonded to each other to form an optionally substituted ring structure,
R^{3d} is a hydrogen atom or an optionally substituted aliphatic hydrocarbon group, or R^{3d} is optionally bonded to a carbon atom of the adjacent phenyl group to form an optionally substituted ring structure,
B^{d} is an optionally substituted benzene ring,
C^{d} is an optionally substituted heterocyclic group, and
Z^{d} is an optionally substituted C₁₋₃ alkylene group (sometimes to be referred to as compound (Id) in the present specification) or a salt thereof or a prodrug thereof;
[18] the pharmaceutical agent of the above-mentioned [1], wherein the HER2 inhibitor having a pyrrolopyrimidine skeleton or pyrazolopyrimidine skeleton is a compound represented by the formula:

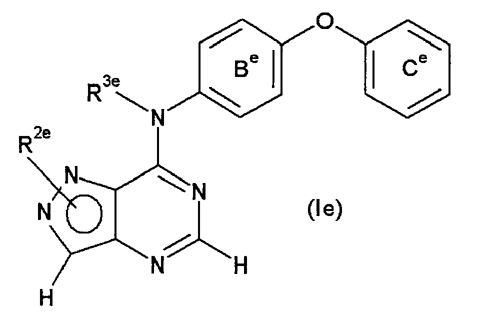

wherein
R^{2e} is an optionally substituted group bonded via a carbon atom or a sulfur atom,
or R^{2e} and R^{3e} are optionally bonded to each other to form an optionally substituted ring structure,
R^{3e} is a hydrogen atom or an optionally substituted aliphatic hydrocarbon group, or R^{3e} is optionally bonded to a carbon atom of the adjacent phenyl group to form an optionally substituted ring structure,
Be is an optionally substituted benzene ring, and
C^{e} is an optionally substituted C₆₋₁₈ aryl group (sometimes to be referred to as compound (Ie) in the present specification) or a salt thereof or a prodrug thereof;
[19] the pharmaceutical agent of the above-mentioned [1], wherein the HER2 inhibitor having a pyrrolopyrimidine skeleton or pyrazolopyrimidine skeleton is a compound represented by the formula:

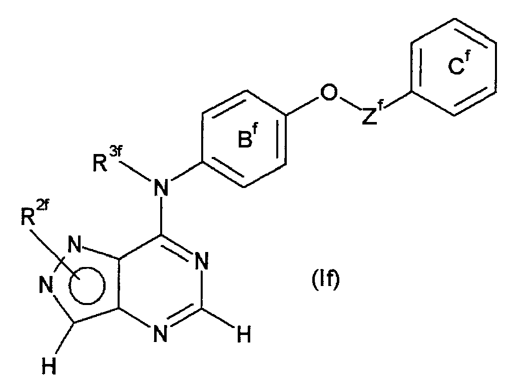

wherein
R^{2f} is an optionally substituted group bonded via a carbon atom or a sulfur atom,
or R^{2f} and R^{3f} are optionally bonded to each other to form an optionally substituted ring structure,
R^{3f} is a hydrogen atom or an optionally substituted aliphatic hydrocarbon group, or R^{3f} is optionally bonded to a carbon atom of the adjacent phenyl group to form an optionally substituted ring structure,
B^{f} is an optionally substituted benzene ring,
C^{f} is an optionally substituted C₆₋₁₈ aryl group, and
Z^{f} is an optionally substituted C₁₋₃ alkylene group (sometimes to be referred to as compound (If) in the present specification) or a salt thereof or a prodrug thereof;
[20] the pharmaceutical agent of the above-mentioned [1], wherein the HER2 inhibitor having a pyrrolopyrimidine skeleton or pyrazolopyrimidine skeleton is a compound represented by the formula:

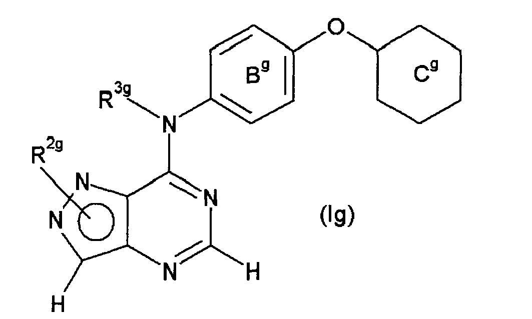

wherein
R^{2g} is an optionally substituted group bonded via a carbon atom or a sulfur atom,
or R^{2g} and R^{3g} are optionally bonded to each other to form an optionally substituted ring structure,
R^{3g} is a hydrogen atom or an optionally substituted aliphatic hydrocarbon group, or R^{3g} is optionally bonded to a carbon atom of the adjacent phenyl group to form an optionally substituted ring structure,
B^{g} is an optionally substituted benzene ring, and
C^{g} is an optionally substituted heterocyclic group (sometimes to be referred to as compound (Ig) in the present specification) or a salt thereof or a prodrug thereof; and the like.
The present invention is explained in detail in the following.

### Effect of the Invention

A pharmaceutical agent comprising (1) a HER2 inhibitor having a pyrrolopyrimidine skeleton or pyrazolopyrimidine skeleton, and (2) not less than one pharmaceutical agent selected from an mTOR inhibitor, a PI3 kinase inhibitor and a cMet inhibitor in combination shows a more significant effect than use thereof as a single agent and other combination pharmaceutical agents, and is useful as a safe agent for the prophylaxis or therapeutic of cancer.
In addition, a compound having a pyrrolopyrimidine skeleton or pyrazolopyrimidine skeleton, which is represented by the aforementioned formula (I), has a thymidine synthase production inhibitory action, and therefore, is useful as an active ingredient of a single agent or a safe agent for the prophylaxis or treatment of cancer.

### Brief Description of the Drawings

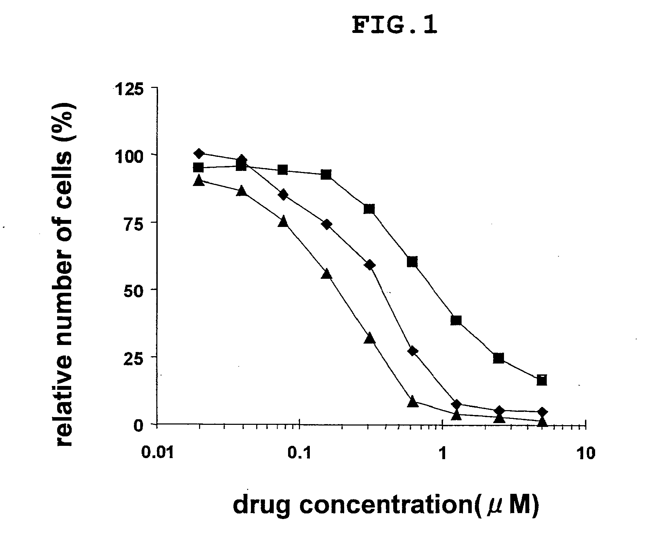

Fig. 1 is a figure showing the effect of a combined use of compound A and PI-103, wherein the horizontal axis shows the concentration of each pharmaceutical agent and the vertical axis shows the number of cells (%) when a group without addition of a pharmaceutical agent is 100%. (■; compound A alone, ◆; PI-103 alone, ▲; compound A + PI-103)

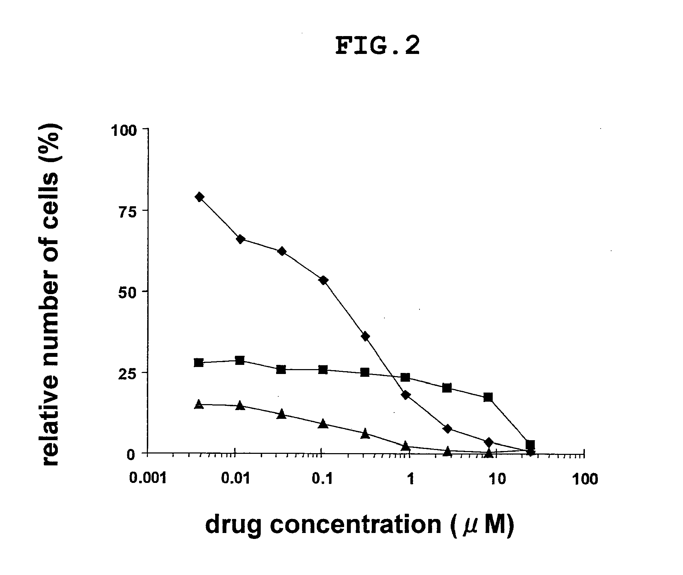

Fig. 2 is a figure showing the effect of a combined use of compound A and rapamycin, wherein the horizontal axis shows the concentration of each pharmaceutical agent and the vertical axis shows the number of cells (%) when a group without addition of a pharmaceutical agent is 100%. (◆; compound A alone, ■; rapamycin alone, ▲; compound A + rapamycin)

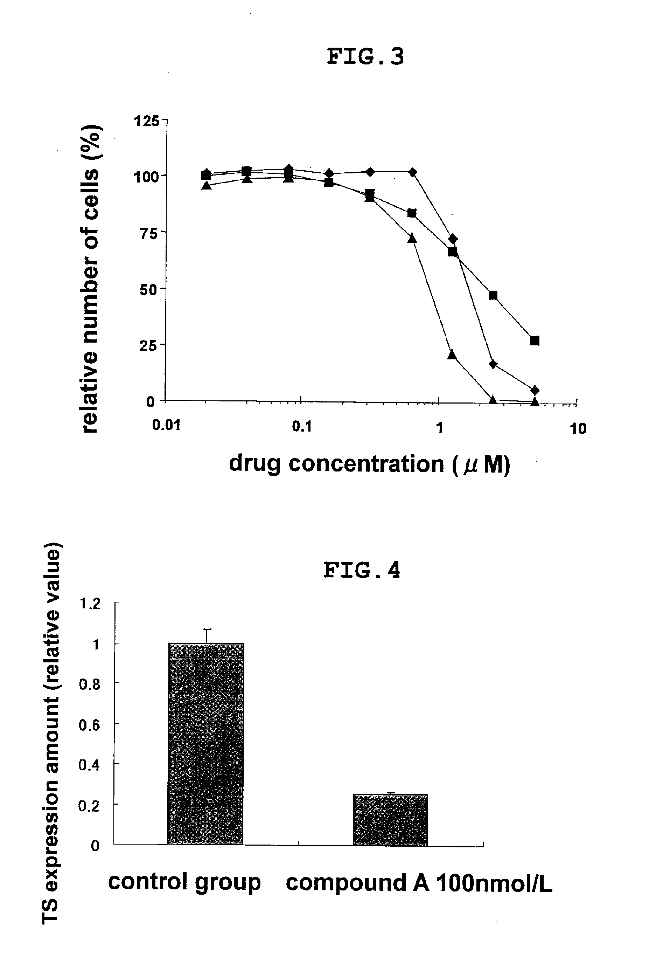

Fig. 3 is a figure showing the effect of a combined use of compound A and PF-2341066, wherein the horizontal axis shows the concentration of each pharmaceutical agent and the vertical axis shows the number of cells (%) when a group without addition of a pharmaceutical agent is 100%. (◆; PF-2341066 alone, ■; compound A alone, ▲; compound A + PF-2341066)
Fig. 4 is a figure showing an average value of TS (thymidine) gene expression amount when compound A was added to human breast cancer cell line BT-474 which is an HER2 high expression cell, wherein the vertical axis shows the relative value of TS gene expression amount.

### (Detailed Description of the Invention)

In the present specification, unless otherwise specified, the "aryl" in the "aryl group" and the substituents includes a monocyclic aryl group and a fused polycyclic aryl group. As the "aryl group", for example, a C₆₋₁₈ aryl group can be mentioned. As the "C₆₋₁₈ aryl group", for example, phenyl, biphenylyl, naphthyl, anthryl, phenanthryl and acenaphthylenyl can be mentioned.
In the present specification, unless otherwise specified, the "heterocyclyl-" in the "heterocyclic group" and substituent encompasses a heteroaryl group and a saturated or unsaturated aliphatic heterocyclic group. In the present specification, as the "heterocyclic group" (and "heterocyclyl-" in the substituents), for example, a 3- to 12-membered (preferably 5-to 8-membered) heterocyclic group (e.g., heteroaryl group or a saturated or unsaturated aliphatic heterocyclic group) containing, as an atom (ring atom) constituting a ring system, one or more (preferably 1 to 4, more preferably 1 to 3, further preferably 1 or 2) hetero atoms selected from an oxygen atom, an optionally oxidized sulfur atom and a nitrogen atom and the like (preferably, an oxygen atom, a sulfur atom and a nitrogen atom etc.) can be mentioned.
In the present specification, unless otherwise specified, as the "aliphatic hydrocarbon group", a linear or branched aliphatic hydrocarbon group having 1 to 15 carbon atom (preferably, 1 to 8 carbon atom) can be mentioned. As such "aliphatic hydrocarbon group", for example, a C₁₋₈ alkyl group, a C₂₋₈ alkenyl group, a C₂₋₈ alkynyl group, a C₃₋₈ cycloalkyl group and the like can be mentioned.

In the present specification, unless otherwise specified, as the "heteroaryl group", an aromatic monocyclic heterocyclic group (e.g., 5- or 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like) and an aromatic fused heterocyclic group (e.g., 8- to 12-membered aromatic fused heterocyclic group such as benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acrydinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like) and the like can be mentioned. As the aromatic fused heterocyclic group, a heterocycle wherein the aforementioned 5- or 6-membered aromatic monocyclic heterocyclic group is fused with a benzene ring and a heterocycle wherein the same or different two heterocycles of the aforementioned 5- or 6-membered aromatic monocyclic heterocyclic group are fused are preferable.

In the present specification, unless otherwise specified, as the "aliphatic heterocyclic group", for example, a 3- to 8-membered (preferably 5- or 6-membered) saturated or unsaturated (preferably saturated) aliphatic heterocyclic group such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, dihydro-1,2,4-oxadiazolyl and the like, and the like can be mentioned.
In the present specification, unless otherwise specified, as the "C₁₋₈ alkyl group", for example, methyl, ethyl, n-propyl, i-propyl, n-butyl, i-butyl, s-butyl, t-butyl, n-pentyl, i-pentyl, t-pentyl, neopentyl, n-hexyl, i-hexyl, n-heptyl and n-octyl and the like can be mentioned, with preference given to a C₁₋₆ alkyl group. In the present specification, moreover, unless otherwise specified, as the "C₁₋₄ alkyl group", for example, methyl, ethyl, n-propyl, i-propyl, n-butyl and i-butyl can be mentioned.
In the present specification, unless otherwise specified, as the "C₂₋₈ alkenyl group", for example, vinyl, (1- or 2-)propenyl, (1-, 2- or 3-)butenyl, pentenyl, octenyl and (1, 3-)butadienyl can be mentioned, with preference given to a C₂₋₄ alkenyl group.

In the present specification, unless otherwise specified, as the "C₂₋₈ alkynyl group", for example, ethynyl, (1- or 2-)propynyl, (1-, 2- or 3-)butynyl, pentynyl and octynyl can be mentioned, with preference given to a C₂₋₄ alkynyl group. In the present specification, unless otherwise specified, as the "C₃₋₈ cycloalkyl group", for example, cyclopropyl, cyclobutyl, cyclopentyl, cyclohexyl, cycloheptyl and cyclooctyl can be mentioned, with preference given to a C₃₋₆ cycloalkyl group. In the present specification, unless otherwise specified, as the "C₁₋₄ alkylene", for example, methylene, ethylene, trimethylene, tetramethylene and propylene and the like can be mentioned. In the present specification, unless otherwise specified, as the "-O-(C₁₋₄ alkylene)-", for example, -OCH₂-, -OCH₂CH₂-, -O(CH₂)3-, -O(CH₂)₄-, -OCH(CH₃)-, -OC(CH₃)₂-, -OCH(CH₃)CH₂-, -OCH₂CH(CH₃)-, -OC(CH₃)₂CH₂- and -OCH₂C(CH₃)₂- and the like can be mentioned.
In the present specification, unless otherwise specified, as the "C₆₋₁₈ aryl-carbonyl group", for example, benzoyl, naphthoyl, anthrylcarbonyl, phenanthrylcarbonyl and acenaphthylenylcarbonyl and the like can be mentioned.
In the present specification, unless otherwise specified, as the "C₆₋₁₈ aryl-C₁₋₄ alkyl-carbonyl group", for example, benzylcarbonyl, 3-phenylpropionyl, 2-phenylpropionyl, 4-phenylbutyryl and 5-phenylpentanoyl and the like can be mentioned.

In the present specification, unless otherwise specified, as the "halogen", fluorine, chlorine, bromine and iodine can be mentioned. As the "5- to 8-membered heterocyclyl-carbonyl group containing 1 to 3 hetero atoms selected from a nitrogen atom, an oxygen atom and a sulfur atom", "a 5- to 8-membered cyclic amino-carbonyl group optionally having 1 or 2 hetero atoms selected from a nitrogen atom, an oxygen atom and a sulfur atom" is preferable, for example, pyrrolidin-1-ylcarbonyl, piperidin-1-ylcarbonyl, piperazin-1-ylcarbonyl, morpholin-4-ylcarbonyl, thiomorpholin-4-ylcarbonyl and the like can be mentioned.

In the above-mentioned the formula, as the "aryl group" for A, a C₆₋₁₈ aryl group is preferable, and phenyl is more preferable. The "aryl group" and "heteroaryl group" for A is optionally substituted by a group represented by the formula - Y²-B wherein Y² is a single bond, -O-, -O-(C₁₋₃ alkylene)-(preferably -OCH₂-), -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted.
As Y², a single bond, -O- or -OCH₂- is preferable, and -O- or -OCH₂- is more preferable. Particularly -O- is preferable.
As the "aryl group" for B, a C₆₋₁₈ aryl group is preferable, and phenyl is more preferable.

As the "heterocyclic group" for B, the aforementioned "5-or 6-membered aromatic monocyclic heterocyclic group" is preferable, and pyridyl is more preferable.
The "aryl group", "heterocyclic group", "C₆₋₁₈ aryl-carbonyl group" or "C₆₋₁₈ aryl-C₁₋₄ alkyl-carbonyl group" for B may have, for example, 1 to 5, the same or different substituents selected from halogen, optionally halogenated C₁₋₄ alkyl, hydroxy, optionally halogenated C₁₋₄ alkoxy, C₁₋₄ alkoxymethyl, hydroxy-C₁₋₄ alkyl, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino, at any substitutable position(s). As the substituent of the "aryl group" for B, halogen, optionally halogenated C₁₋₄ alkyl, optionally halogenated C₁₋₄ alkoxy and the like are preferable and, for example, fluorine, chlorine, trifluoromethyl, trifluoromethoxy and the like can be mentioned. Of these, optionally halogenated C₁₋₄ alkyl (e.g., trifluoromethyl) and the like are preferable.
The "aryl group" and "heteroaryl group" for A may further have, besides the above-mentioned a group represented by the formula -Y²-B, 1 to 5, the same or different substituents at substitutable optional position(s). As such substituent, substituents similar to those exemplified for the "aryl group" or "heterocyclic group" for B can be mentioned. As such substituent, halogen, optionally halogenated C₁₋₄ alkyl and the like are preferable, such as chlorine, methyl and the like can be mentioned. Of these, halogen (e.g., chlorine) is preferable.
As A, for example, 3-chloro-4-[3-(trifluoromethyl)phenoxy]phenyl, 3-chloro-4-[(3-fluorobenzyl)oxy]phenyl, 3-methyl-4-[3-(trifluoromethoxy)phenoxy]phenyl, 3-chloro-4-(3-chlorophenoxy)phenyl, 3-chloro-4-[3-(trifluoromethoxy)phenoxy]phenyl and the like can be mentioned. Of these, 3-chloro-4-[3-(trifluoromethyl)phenoxy]phenyl and the like are preferable.
As the "aliphatic hydrocarbon group" for R³, a C₁₋₈ alkyl group, a C₂₋₈ alkenyl group, a C₂₋₈ alkynyl group and a C₃₋₈ cycloalkyl group are preferable.
The "aliphatic hydrocarbon group" for R³ is optionally substituted by 1 to 3 substituents selected from halogen, hydroxy, C₁₋₄ alkoxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino.

The "C₁₋₄ alkylene" and "-O-(C₁₋₄ alkylene)-" for Y¹ are optionally substituted by 1 to 3 substituents selected from halogen, hydroxy, C₁₋₄ alkoxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino.
As X¹, -NR³- wherein R³ is as defined above is preferable.
As R³, a hydrogen atom is preferable.
As W, C(R¹) is preferable.
As R¹, a hydrogen atom is preferable.
As the "optionally substituted group bonded via a carbon atom, a nitrogen atom or an oxygen atom" for R¹, a group of the formula -X²-R⁴ can be mentioned, wherein X² is a single bond, -NH- or -O-, and R⁴ is a hydrogen atom, a cyano group, or a C₁₋₈ alkyl group, a C₂₋₈ alkenyl group, a C₂₋₈ alkynyl group, a carbamoyl group, a C₁₋₈ alkyl-carbonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted.
The "C₁₋₈ alkyl group", "C₂₋₈ alkenyl group", "C₂₋₈ alkynyl group", "C₁₋₈ alkyl-carbonyl group", "C₃₋₈ cycloalkyl group", "C₆₋₁₈ aryl group", "C₆₋₁₈ aryl-C₁₋₄ alkyl group", "C₆₋₁₈ aryl-carbonyl group", "C₆₋₁₈ aryl-C₁₋₄ alkyl-carbonyl group", "heterocyclic group", "heterocyclyl-C₁₋₄ alkyl group", "heterocyclyl-carbonyl group" and "heterocyclyl-C₁₋₄ alkyl-carbonyl group" are, for example, optionally substituted by one or more (preferably 1 to 5, more preferably 1 to 3) substituent(s) selected from the group consisting of
(a) halogen,
(b) oxo,
(c) optionally halogenated C₁₋₄ alkyl,
(d) -(CH₂)ₘ-Q,
(e) -(CH2)ₘ-Z¹-(optionally halogenated C₁₋₄ alkyl),
(f) -(CH₂)ₘ-Z¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group)
   (preferably, said heterocyclic group is a 5- to 8-membered heterocyclic group having 1 to 3 hetero atoms selected from a nitrogen atom, an oxygen atom and an optionally oxidized sulfur atom)
(k) -(CH₂)ₘ-Z²-C₁₋₄ alkoxy, and
(l) -(CH₂)ₘ-Z²-(CH₂)ₙ-Z¹-(CH₂)ₙ-Z¹-C₁₋₄ alkyl (hereinafter to be sometimes to be referred to as substituent group T).

In these the formulas, m is an integer of 0 to 4, n is an integer of 1 to 4,
Q is hydroxy, carboxy, cyano, nitro, -NR⁶R⁷, -CONR⁶R⁷, -OCONH₂ or -SO₂NR⁶R⁷,
Z¹ is -O-, -CO-, -C(OH)R⁸-, -C(=N-OR⁸)-, -S-, -SO-, -SO₂-,
-N(COR⁸)-, -N(CO²R⁹)-, -N(SO₂R⁹)-, -CO-O-, -O-CO-, -CO-NR⁸-,
-NR⁸-CO-, -NR⁸-CO₂-, -NR⁸-CO-NH-, -NR₈-SO₂-, or -NR⁸-C(=NH)-NH-, and
Z² is -O-, -CO-, -C(OH)R⁸-, -C(=N-OR⁸)-, -S-, -SO-, -SO₂-,
-NR⁸-, -N(COR⁸)-, -N(CO²R⁹)-, -N(SO²R⁹)-, -CO-O-, -O-CO-, -CO-NR⁸-, -NR⁸-CO-, -NR⁸-CO²-, -NR⁸-CO-NH-, -NR⁸-C(=NH)-NH-, -NR⁸-SO₂-, or -SO₂-NR⁸-. In these the formulas, (CH₂)ₘ and (CH₂)ₙ are optionally substituted by one or more (preferably 1 to 5, more preferably 1 to 3) substituents selected from, for example, halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH- or -C≡C-.
In these the formulas, R⁶ and R⁷ are the same or different and each is a hydrogen atom or C₁₋₄ alkyl, or R⁶ and R⁷ form a ring together with a nitrogen atom. In these the formulas, moreover, R⁸ is a hydrogen atom or C₁₋₄ alkyl and R⁹ is C₁₋₄ alkyl. When R⁶ and R⁷ form a ring together with a nitrogen atom, as the nitrogen-containing heterocyclic group, for example, a 3- to 8-membered (preferably 5- or 6-membered) saturated or unsaturated (preferably saturated) aliphatic heterocyclic group such as azetidinyl, pyrrolidinyl, piperidinyl, homopiperidinyl, heptamethyleneimino, morpholinyl, thiomorpholinyl, piperazinyl, homopiperazinyl and the like, and the like can be mentioned.

As X², a single bond is preferable.
As R⁴, a hydrogen atom or a C₁₋₈ alkyl group, a C₂₋₈ alkenyl group, a C₆₋₁₈ aryl group or heterocyclic group, each of which is optionally substituted is preferable. As the "C₆₋₁₈ aryl group" for R⁴, phenyl is preferable. As the "heterocyclic group" for R⁴, the aforementioned "5- or 6-membered aromatic monocyclic heterocyclic group" is preferable, and furyl is more preferable.
As the "optionally substituted group bonded via a carbon atom or a sulfur atom" for R², a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted, can be mentioned.
The "C₁₋₈ alkyl group", "C₂₋₈ alkenyl group", "C₂₋₈ alkynyl group", "C₁₋₈ alkyl-carbonyl group", "C₁₋₈ alkyl-sulfonyl group", "C₃₋₈ cycloalkyl group", "C₆₋₁₈ aryl group", "C₆₋₁₈ aryl-C₁₋₄ alkyl group", "C₆₋₁₈ aryl-carbonyl group", "C₆₋₁₈ aryl-C₁₋₄ alkyl-carbonyl group", "C₆₋₁₈ aryl-sulfonyl group", "heterocyclic group", "heterocyclyl-C₁₋₄ alkyl group", "heterocyclyl-carbonyl group" and "heterocyclyl-C₁₋₄ alkyl-carbonyl group" are optionally substituted by, for example, one or more (preferably 1 to 5, more preferably 1 to 3) substituents selected from the above-mentioned substituent group T.

As R², a hydrogen atom or a C₁₋₈ alkyl group, a C₆₋₁₈ aryl group, a C₆₋₁₈ aryl-C₁₋₄ alkyl group, a C₆₋₁₈ aryl-carbonyl group, a C₆₋₁₈ aryl-sulfonyl group or heterocyclyl-C₁₋₄ alkyl group, each of which is optionally substituted, is preferable. Particularly, optionally substituted C₁₋₈ alkyl group and the like are preferable and, for example, ethyl substituted at the 2-position and the like can be mentioned. As the substituent of the optionally substituted C₁₋₈ alkyl group, (g) -(CH₂)ₘ-Z²-(CH₂)ₙ-Q in the above-mentioned substituent group T and the like are preferable, particularly that wherein m is 0, Z² is - NR⁸-CO- or -O- and Q is hydroxy, namely, -O-(CH₂)ₙ-OH or -NR⁸-CO-(CH₂)ₙ-OH ((CH₂)ₙ is optionally substituted by C₁₋₄ alkyl (e.g., methyl)) and the like are preferable. Particularly, - NR⁸-CO-(CH₂)ₙ-OH((CH₂)ₙ is optionally substituted by C₁₋₄ alkyl (e.g., methyl)) is preferable. In these the formulas, R⁸ is preferably a hydrogen atom and n is preferably 2. As the (CH₂)ₙ moiety, -CH₂-C(CH₃)₂-, -CH₂-CH₂- and the like can be mentioned and -CH₂-C(CH₃)₂- and the like are preferable. As the substituent of the optionally substituted C₁₋₈ alkyl group for R², (h) -(CH₂)ₘ-Z²-(CH₂)ₙ-Z¹- (optionally halogenated C₁₋₄ alkyl) of the above-mentioned substituent group T and the like are preferable, particularly that wherein m is 0, Z² is -NR⁸-CO- or -O-, and Z¹ is -SO₂-, namely, -O-(CH₂)ₙ-SO₂-(optionally halogenated C₁₋₄ alkyl) or -NR⁸-CO-(CH₂)ₙ-SO₂-(optionally halogenated C₁₋₄ alkyl) ((CH₂)ₙ is optionally substituted by C₁₋₄ alkyl (e.g., methyl)) and the like are preferable. In these the formulas, R⁸ is preferably a hydrogen atom, and n is preferably 1 or 2. As the (CH₂)ₙ moiety, -CH₂-, -CH₂-CH₂-, -C(CH₃)₂- and the like can be mentioned. As the optionally halogenated C₁₋₄ alkyl moiety, for example, methyl, tert-butyl and the like can be mentioned.
As the substituent of the optionally substituted C₁₋₈ alkyl group for R², for example, -NH-CO-CH₂-C(CH₃)₂-OH, -O-CH₂-CH₂-OH, -NH-CO-CH₂-SO₂-CH₃, -NH-CO-C(CH₃)₂-SO₂-CH₃, -O-CH₂-CH₂-SO₂-C(CH₃)3 and the like can be mentioned, and -NH-CO-CH₂-C(CH₃)₂-OH and the like are preferable.
As the "C₆₋₁₈ aryl group" for R², phenyl is preferable. As the "C₆₋₁₈ aryl-C₁₋₄ alkyl group" for R², benzyl is preferable. As the "C₆₋₁₈ aryl-carbonyl group" for R², benzoyl is preferable. As the "C₆₋₁₈ aryl-sulfonyl group" for R², phenylsulfonyl is preferable. As the "heterocyclic group" or "heterocyclyl-" of "heterocyclyl-C₁₋₄ alkyl group", "heterocyclyl-carbonyl group" and "heterocyclyl-C₁₋₄ alkyl-carbonyl group" for R², the aforementioned "5- or 6-membered aromatic monocyclic heterocyclic group" or the aforementioned "aliphatic heterocyclic group" is preferable, and furyl or tetrahydrofuryl is more preferable.
In the substituents that a group represented by R² may have, when R⁶ and R⁷ form a ring together with a nitrogen atom, the "ring" optionally further has 1 to 5 (preferably 1 to 3) the same or different substituents. As such substituents, substituents similar to those exemplified for "aryl group" or "heterocyclic group" for B can be mentioned.
The aforementioned "carbamoyl group" and "ureido group" optionally have 1 or 2 optionally substituted C₁₋₈ alkyl group (s). Alternatively, the "carbamoyl group" and "ureido group" may have two substituents and they may form an optionally substituted ring, together with the adjacent nitrogen atom. As the "ring" of the "optionally substituted ring", rings similar to those formed by R⁶ and R⁷ together with a nitrogen atom as exemplified above can be mentioned. As the "substituent" of the "optionally substituted C₁₋₈ alkyl group" and as the "substituent" of the "optionally substituted ring", groups similar to the substituents of the above-mentioned substituent group T can be mentioned.

As the "optionally substituted carbamoyl group", carbamoyl, C₁₋₈ alkylcarbamoyl, di (C₁₋₈ alkyl) carbamoyl, C₆₋₁₈ aryl-C₁₋₄ alkylcarbamoyl, azetidin-1-ylcarbonyl, pyrrolidin-1-ylcarbonyl, piperidin-1-ylcarbonyl, piperazin-1-ylcarbonyl, morpholin-4-ylcarbonyl, thiomorpholin-4-ylcarbonyl, (C₁₋₄ alkyl)piperidin-1-ylcarbonyl, (C₆₋₁₈ aryl-C₁₋₄ alkyl)piperidin-1-ylcarbonyl and the like can be mentioned.
As the "optionally substituted ureido group", ureido, 3-(C₁₋₈ alkyl)ureido, 3,3-di(C₁₋₈ alkyl)ureido, 3-(C₆₋₁₈ aryl-C₁₋₄ alkyl)ureido, azetidine-1-ylcarbonylamino, pyrrolidin-1-ylcarbonylamino, piperidin-1-ylcarbonylamino, piperazin-1-ylcarbonylamino, morpholin-4-ylcarbonylamino, thiomorpholin-4-ylcarbonylamino, (C₁₋₄ alkyl)piperidin-1-ylcarbonylamino, (C₆₋₁₈ aryl-C₁₋₄ alkyl)piperidin-1-ylcarbonylamino and the like can be mentioned.

As the "ring structure" of the optionally substituted ring structure formed by R³ bonded to a carbon atom or a hetero atom on the aryl group or the heteroaryl group for A, a saturated or unsaturated (preferably saturated) 4- to 8-membered (preferably 5- or 6-membered) nitrogen-containing heterocycle can be mentioned. Specifically,

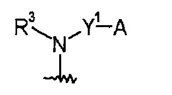

is

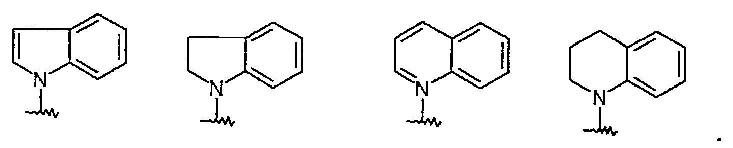

The "ring structure" may have 1 to 5 (preferably 1 to 3, more preferably 1 or 2) the same or different substituents at any substitutable position(s). As such substituents, substituents similar to those exemplified for "aryl group" or "heterocyclic group" for B can be mentioned.

As the "ring structure" of the optionally substituted ring structure formed by R¹ and R² bonded to each other, a saturated or unsaturated (preferably saturated) 4- to 8-membered (preferably 5- or 6-membered) heterocycle can be mentioned. When R¹ and R² are bonded to each other to form an optionally substituted ring structure, for example,

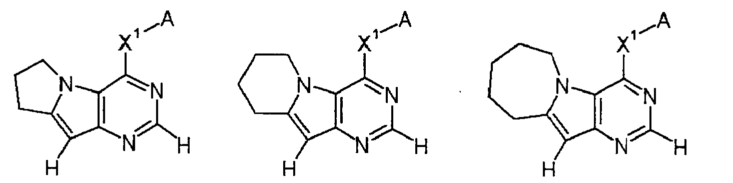

wherein each symbol is as defined above, and the like can be mentioned.
As the "ring structure" of the optionally substituted ring structure formed by R² and R³ bonded to each other, a saturated or unsaturated (preferably saturated) 4- to 8-membered (preferably 5- to 7-membered) heterocycle can be mentioned. When R² and R³ are bonded to each other to form an optionally substituted ring structure, for example,

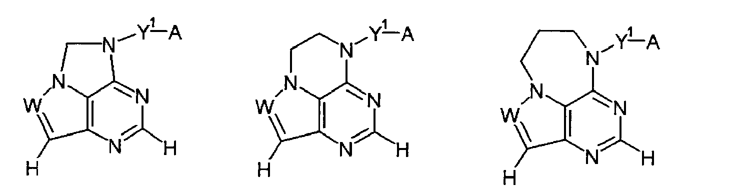

wherein each symbol is as defined above, and the like can be mentioned. The "ring structure" formed by R¹ and R², or R² and R³ bonded to each other may have 1 to 5 (preferably 1 to 3, more preferably 1 or 2) the same or different substituents selected from the above-mentioned substituent group T at any substitutable position(s).

When W is C(R¹), compound (I) is represented by the following the formula (IA):

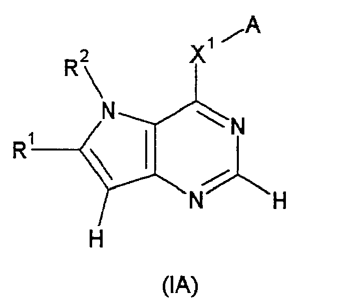

wherein each symbol is as defined above.
When W is N, compound (I) is represented by the following the formula (IB) or (IC):

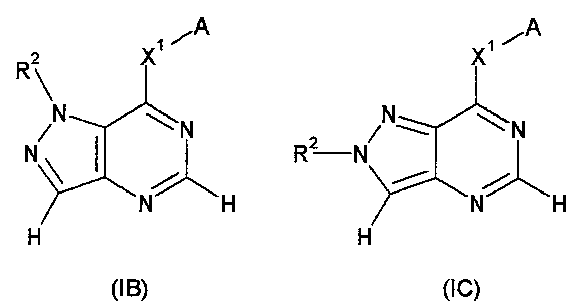

wherein each symbol is as defined above.
Specifically, as compound (I) or a salt thereof or a prodrug thereof, the following compounds (Ia) - (Ik) or a salt thereof or a prodrug thereof and the like are preferably used.

### [Compound (Ia)]

A compound represented by the formula:

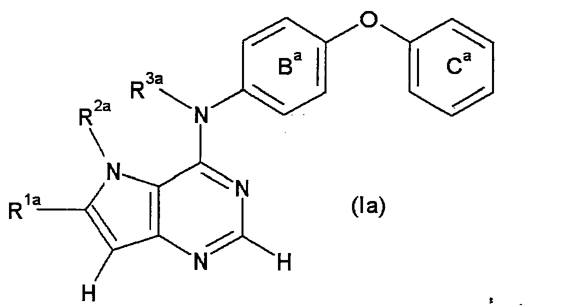

wherein R^{1a} is a hydrogen atom or an optionally substituted group bonded via a carbon atom, a nitrogen atom or an oxygen atom,
R^{2a} is an optionally substituted group bonded via a carbon atom or a sulfur atom, or
R^{1a} and R^{2a}, or R^{2a} and R^{3a} are optionally bonded to each other to form an optionally substituted ring structure,
R^{3a} is a hydrogen atom or an optionally substituted aliphatic hydrocarbon group, or
R^{3a} is optionally bonded to a carbon atom of the adjacent phenyl group to form an optionally substituted ring structure,
B^{a} is an optionally substituted benzene ring, and
C^{a} is an optionally substituted C₆₋₁₈ aryl group.

As the "optionally substituted group bonded via a carbon atom, a nitrogen atom or an oxygen atom" for R^{1a}, those similar to the "optionally substituted group bonded via a carbon atom, a nitrogen atom or an oxygen atom" for R¹ can be used.
As R^{1a}, a hydrogen atom is preferable.
As the "optionally substituted group bonded via a carbon atom or a sulfur atom" for R^{2a}, those similar to the "optionally substituted group bonded via a carbon atom or a sulfur atom" for R² can be used.
As the "optionally substituted ring structure" constructed by R^{1a} and R^{2a}, or R^{2a} and R^{3a} bonded to each other, those similar to the "optionally substituted ring structure" constructed by R¹ and R², or R² and R³ bonded to each other can be used.
As the "optionally substituted aliphatic hydrocarbon group" for R^{3a}, those similar to the "optionally substituted aliphatic hydrocarbon group" for R³ can be used.
As the "optionally substituted ring structure" constructed by R^{3a} bonded to a carbon atom of the adjacent phenyl group, "optionally substituted ring structure" constructed by R³ bonded to a carbon atom of the adjacent phenyl group can be used.
As R^{3a}, a hydrogen atom is preferable.
As the substituent of the "optionally substituted benzene ring" for B^{a}, for example, 1 to 5, the same or different substituents selected from halogen, optionally halogenated C₁₋₄ alkyl, hydroxy, optionally halogenated C₁₋₄ alkoxy, C₁₋₄ alkoxymethyl, hydroxy-C₁₋₄ alkyl, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino are used.
As the substituent of the "optionally substituted benzene ring" for B^{a}, halogen, optionally halogenated C₁₋₄ alkyl and the like are preferable and, for example, chlorine, methyl and the like can be mentioned. Of these, halogen (e.g., chlorine) is preferable. As B^{a}, a benzene ring wherein the 1-position of the ring is the carbon atom bonded to N and the 3-position is substituted by chlorine or methyl (preferably chlorine) and the like can be mentioned.
As the "C₆₋₁₈ aryl group" of the "optionally substituted C₆₋₁₈ aryl group" for C^{a}, for example, phenyl, biphenylyl, naphthyl, anthryl, phenanthryl, acenaphthylenyl and the like are used. Of these, phenyl is preferable.

As the "substituent" of the "optionally substituted C₆₋₁₈ aryl group" for C^{a}, those similar to the "optionally substituted benzene ring" for B^{a} can be used. As the "substituent" of the "optionally substituted C₆₋₁₈ aryl group" for C^{a}, halogen, optionally halogenated C₁₋₄ alkyl, optionally halogenated C₁₋₄ alkoxy and the like are preferable and, for example, chlorine, trifluoromethyl, trifluoromethoxy and the like can be mentioned. Of these, optionally halogenated C₁₋₄ alkyl (e.g., trifluoromethyl) and the like are preferable. As C^{a}, for example, 3-(trifluoromethyl)phenyl, 3-(trifluoromethoxy)phenyl, 3-chlorophenyl and the like can be mentioned. Of these, 3-(trifluoromethyl)phenyl and the like are preferable. As R^{2a}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each optionally substituted by 1 to 5 substituents selected from the group consisting of
(a) halogen,
(b) oxo,
(c) optionally halogenated C₁₋₄ alkyl,
(d) -(CH₂)ₘ-Q.
(e) -(CH₂)ₘ-Z¹-(optionally halogenated C₁₋₄ alkyl),
(f) -(CH₂)ₘ-Z¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group)
   (preferably, 5- to 8-membered heterocyclic group having 1 to 3 hetero atoms selected from a nitrogen atom, an oxygen atom and an optionally oxidized sulfur atom),
(k) -(CH₂)ₘ-Z²-C₁₋₄ alkoxy, and
(l) -(CH₂)ₘ-Z₂-(CH₂)ₙ-Z¹-(CH₂)ₙ-Z¹-C₁₋₄ alkyl
   wherein m is an integer of 0 to 4, n is an integer of 1 to 4, Q is hydroxy, carboxy, cyano, nitro, -NR⁶R⁷, CONR⁶R⁷, -OCONH₂ or -SO₂NR⁶R⁷,
   Z¹ is -O-, -CO-, -C(OH)R⁸-, -C(=N-OR⁸)-, -S-, -SO-, -SO₂-,-N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-SO₂- or -NR⁸-C(=NH)-NH-,
   Z² is -O-, -CO-, -C(OH)R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, - NR⁸-CO-, -NR⁸-CO₂-, -NR⁸-CO-NH-, -NR⁸-C(=NH)-NH-, -NR⁸-SO₂- or - SO₂-NR⁸-,
   (CH₂)ₘ and (CH₂)ₙ is optionally substituted by 1 to 5 substituents selected from halogen, optionally halogenated C₁₋₄ alkyl and hydroxy, when m or n is not less than 2, a subset - CH₂CH₂- of (CH₂)ₘ and (CH₂)ₙ may be replaced by -CH=CH- or -C≡C-, R⁶ and R⁷ are the same or different and each is a hydrogen atom or a C₁₋₄ alkyl group, or R⁶ and R⁷ are bond to form, together with a nitrogen atom, a 3- to 8-membered saturated or unsaturated aliphatic heterocyclic group,
   R⁸ is a hydrogen atom or C1₋₄ alkyl and R⁹ is C₁₋₄ alkyl, is preferable.
As R^{2a}, optionally substituted C₁₋₈ alkyl group and the like are preferable and, for example, ethyl substituted at the 2-position and the like can be mentioned. As the substituent of the optionally substituted C₁₋₈ alkyl group, (g)-(CH₂)ₘ-Z²-(CH₂)ₙ-Q in the above-mentioned substituent group and the like are preferable, particularly that wherein m is 0, Z² is -NR⁸-CO- or -O- and Q is hydroxy, namely, -O-(CH₂)ₙ-OH or -NR⁸-CO-(CH₂)ₙ-OH ((C₂)ₙ is optionally substituted by C₁₋₄ alkyl (e.g., methyl)) and the like are preferable. Particularly, -NR⁸-CO-(CH₂)ₙ-OH ((CH₂)ₙ is optionally substituted by C₁₋₄ alkyl (e.g., methyl)) is preferable. In these the formulas, R⁸ is preferably a hydrogen atom and n is preferably 2. As the (CH₂) moiety, -CH₂-C(CH₃)₂-, -CH₂-CH₂- and the like can be mentioned and -CH₂-C(CH₃)₂- and the like are preferable.
As the substituent of the optionally substituted C₁₋₈ alkyl group for R^{2a}, (h) -(CH₂)ₘ-Z²-(CH₂)ₙ-Z¹-(optionally halogenated C₁₋₄ alkyl) of the above-mentioned substituent group and the like are preferable, particularly that wherein m is 0, Z² is -NR⁸-CO- or -O-, and Z¹ is -SO₂-, namely, -O-(CH₂)ₙ-SO₂-(optionally halogenated C₁₋₄ alkyl) or -NR⁸-CO-(CH₂)ₙ-SO₂-(optionally halogenated C₁₋₄ alkyl) ((-CH₂)ₙ is optionally substituted by C₁₋₄ alkyl (e.g., methyl)) and the like are preferable. In these the formulas, R⁸ is preferably a hydrogen atom, and n is preferably 1 or 2. As the (CH₂)ₙ moiety, -CH₂-, -CH₂-CH₂-, -C(CH₃)₂- and the like can be mentioned. As the optionally halogenated C₁₋₄ alkyl moiety, for example, methyl, tert-butyl and the like can be mentioned.
As the substituent of the optionally substituted C₁₋₈ alkyl group for R^{2a}, for example, -NH-CO-CH₂-C(CH₃)₂-OH, -O-CH₂-CH₂-OH, -NH-CO-CH₂-SO₂-CH₃, -NH-CO-C(CH₃)₂-SO₂-CH₃, -O-CH₂-CH₂-SO₂-C(CH₃)₃ and the like can be mentioned, and -NH-CO-CH₂-C(CH₃)₂-OH and the like are preferable.

As compound (Ia), a compound wherein
B^{a} is a benzene ring optionally substituted by 1 to 4 substituents selected from halogen, C₁₋₄ alkyl, hydroxy-C₁₋₄ alkyl and C₁₋₄ alkoxy;
C^{a} is a phenyl group optionally substituted by 1 to 5 substituents selected from (i) halogen, (ii) optionally halogenated C₁₋₄ alkyl, (iii) hydroxy-C₁₋₄ alkyl, (iv) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like), (v) optionally halogenated C₁₋₄ alkoxy, (vi) C₁₋₄ alkyl-carbonyl, (vii) cyano, (viii) carbamoyl optionally substituted by C₁₋₈ alkyl and (ix) C₁₋₄ alkoxy-carbonyl;
R^{1a} is
(i) a hydrogen atom,
(ii) a cyano group, or
(iii) a C₁₋₄ alkyl group or a C₂₋₄ alkenyl group, each of which is optionally substituted by -NR⁸-CO-(CH₂)ₙ-NR⁶R⁷ wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and when n is not less than 2, a subset -CH₂CH₂- of (CH₂)ₙ is optionally replaced by - CH=CH-;
   R^{2a} is a C₁₋₈ alkyl group, a C₂₋₈ alkenyl group or a C₂₋₈ alkynyl group, each of which is optionally substituted by substituent(s) selected from
   (a) hydroxy,
   (b) carboxy,
   (c) cyano,
   (d) optionally halogenated C₁₋₄ alkoxy,
   (e) -O-(CH₂)ₙ-OH,
   (f) -O-(CH₂)ₙ-O-CO-NH₂,
   (g) -O-(CH₂)ₙ-O-(optionally halogenated C₁₋₄ alkyl),
   (h) -O-(CH₂)ₙ-SO₂-(optionally halogenated C₁₋₄ alkyl),
   (i) -O-(CH₂)ₙ-SO₂-C₆₋₁₈ aryl,
   (j) -O-(CH₂)ₙ-SO₂-(CH₂)ₙ-OH,
   (k) -O-(CH₂)ₙ-NR⁸-CO-C₁₋₄ alkyl,
   (l) -O-(CH₂)ₙ-NR⁸-CO-(CH₂)ₙ-SO₂-C₁₋₄ alkyl,
   (m) -O-(CH₂)ₙ-NR⁸-SO₂-(optionally halogenated C₁₋₄ alkyl),
   (n) -CO-NR⁸-(CH₂)ₙ-OH,
   (o) -CO-NR⁸-(CH₂)ₙ-SO₂-(optionally halogenated C₁₋₄ alkyl),
   (p) -CO-NR⁸-O-C₁₋₄ alkyl,
   (q) -NR⁶R⁷,
   (r) -NR⁸-(CH₂)ₙ-OH,
   (s) -NR⁸-(CH₂)ₙ-SO₂-C₁₋₄ alkyl,
   (t) -NR⁸-CO-(optionally halogenated C₁₋₄ alkyl),
   (u) -NR⁸-CO-(CH₂)ₙ-OH,
   (v) -NR⁸-CO-(CH₂)ₙ-CN,
   (w) -NR⁸-CO-(CH₂)ₙ-NR⁶R⁷,
   (x) -NR⁸-CO-(CH₂)ₙ-O-C₁₋₄ alkyl,
   (y) -NR⁸-CO-(CH₂)ₙ-SO-(optionally halogenated C₁₋₄ alkyl),
   (z) -NR⁸-CO-(CH₂)ₙ-SO₂- (optionally halogenated C₁₋₄ alkyl),
   (aa) -NR⁸-CO-(CH₂)ₙ-SO₂-C₃₋₈ cycloalkyl,
   (bb) -NR⁸-CO-(CH₂)ₙ-NR⁸-SO₂-C₁₋₄ alkyl,
   (cc) -NR⁸-CO₂-(CH₂)ₙ-SO₂-C₁₋₄ alkyl,
   (dd) -NR⁸-CO-NH-(CH₂)ₙ-SO₂-C₁₋₄ alkyl,
   (ee) -NR⁸-CO-NH-O-C₁₋₄ alkyl,
   (ff) -NR⁸-CO-NH- (CH₂)ₙ-O-C₁₋₄ al kyl,
   (gg) -NR⁸-C (=NH)-NH-C₁₋₄ alkyl,
   (hh) -NR⁸-SO₂-(CH₂)ₙ-SO₂-C₁₋₄ alkyl,
   (ii) -S-(CH₂)ₙ-OH,
   (jj) -SO-(CH₂)ₙ-OH,
   (kk) -SO₂-(CH₂)ₙ-OH, and
   (ll) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
      wherein n is an integer of 1 to 4, R⁶ and R⁷ are the same or different and each is a hydrogen atom or a C₁₋₄ alkyl group, R⁸ is a hydrogen atom or a C₁₋₄ alkyl group, (CH₂)ₙ is optionally substituted by optionally halogenated C₁₋₄ alkyl or hydroxy, and when n is not less than 2, a subset -CH₂CH₂- of (CH₂)ₙ is optionally replaced by -CH=CH-; and
      R^{3a} is a hydrogen atom or a C₁₋₆ alkyl group; or R^{1a} and R^{2a} are optionally bonded to form

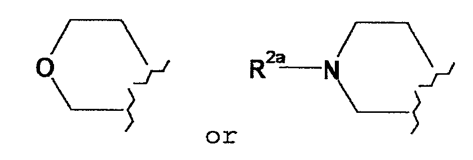

R2^{a} and R^{3a} are optionally bonded to form C₂₋₄ alkylene optionally substituted by an imino group is preferable.

As R⁸, a hydrogen atom, methyl, ethyl and the like are preferable, and a hydrogen atom is particularly preferable. As R^{2a}, a C₁₋₈ alkyl group, a C₂₋₈ alkenyl group or a C₂₋₈ alkynyl group, each of which is optionally substituted by substituent(s) selected from
(a) hydroxy,
(b) carboxy,
(c) cyano,
(d) optionally halogenated C₁₋₄ alkoxy,
(e) -O-(CH₂)ₙ-OH (wherein (CH₂)ₙ is optionally substituted by hydroxy),
(f) -O-(CH₂)ₙ-O-CO-NH₂,
(g) -O-(CH₂)ₙ-O-(optionally halogenated C₁₋₄ alkyl),
(h) -O-(CH₂)ₙ-SO₂-(optionally halogenated C₁₋₄ alkyl),
(i) -O-(CH₂)ₙ-SO₂-C₆₋₁₈ aryl,
(j) -O-(CH₂)ₙ-SO₂-(CH₂)ₙ-OH,
(k) -O-(CH₂)ₙ-NR⁸-CO-C₁₋₄ alkyl,
(l) -O-(CH₂)ₙ-NR⁸-CO-(CH₂)ₙ-SO₂-C₁₋₄ alkyl,
(m) -O-(CH₂)ₙ-NR⁸-SO₂-(optionally halogenated C₁₋₄ alkyl),
(n) -CO-NR⁸-(CH₂)ₙ-OH,
(o) -CO-NR⁸- (CH₂)ₙ-SO₂- (optionally halogenated C₁₋₄ alkyl),
(p) -CO-NR⁸-O-C₁₋₄ alkyl,
(q) -NR⁶R⁷,
(r) -NR⁸- (CH₂)ₙ-OH,
(s) -NR⁸-(CH₂)ₙ-SO₂-C₁₋₄ alkyl,
(t) -NR⁸-CO-(optionally halogenated C₁₋₄ alkyl),
(u) -NR⁸-CO-(CH₂)ₙ-OH (wherein (CH₂)ₙ is optionally substituted by optionally halogenated C₁₋₄ alkyl or hydroxy),
(v) -NR⁸-CO-(CH₂)ₙ-CN,
(w) -NR⁸-CO-(CH₂)ₙ-NR⁶R⁷ (when n is not less than 2, a subset -CH₂CH₂- of (CH₂)ₙ is optionally replaced by -CH=CH-),
(x) -NR⁸-CO-(CH₂)ₙ-O-C₁₋₄ alkyl,
(y) -NR⁸-CO-(CH₂)ₙ-SO-(optionally halogenated C₁₋₄ alkyl),
(z) -NR⁸-CO-(CH₂)ₙ-SO₂-(optionally halogenated C₁₋₄ alkyl) (wherein (CH₂)ₙ is optionally substituted by C₁₋₄ alkyl),
(aa) -NR⁸-CO-(CH₂)ₙ-SO₂-C₃₋₈ cycloalkyl,
(bb) -NR⁸-CO-(CH₂)ₙ-NR⁸-SO₂-C₁₋₄ alkyl,
(cc) -NR⁸-CO₂-(CH₂)ₙ-SO₂-C₁₋₄ alkyl,
(dd) -NR⁸-CO-NH-(CH₂)ₙ-SO₂-C₁₋₄ alkyl,
(ee) -NR⁸-CO-NH-O-C₁₋₄ alkyl,
(ff) -NR⁸-CO-NH- (CH₂)ₙ-O-C₁₋₄ alkyl,
(gg) -NR⁸-C (=NH)-NH-C₁₋₄ alkyl,
(hh) -NR⁸-SO₂-(CH₂)ₙ-SO₂-C₁₋₄ alkyl,
(ii) -S-(CH₂)ₙ-OH,
(jj) -SO-(CH₂)ₙ-OH,
(kk) -SO₂-(CH₂)ₙ-OH, and
(ll) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, and R⁸ is a hydrogen atom or a C₁₋₄ alkyl group, is preferable.
As R⁸, a hydrogen atom, methyl, ethyl and the like are preferable, and a hydrogen atom is particularly preferable.

As compound (Ia), moreover, a compound wherein
B^{a} is a benzene ring optionally substituted by 1 to 4 substituents selected from halogen and optionally halogenated C₁₋₄ alkyl;
C^{a} is a phenyl group substituted by 1 to 5 substituents selected from (i) halogen, (ii) optionally halogenated C₁₋₄ alkyl, (iii) hydroxy-C₁₋₄ alkyl, (iv) heterocyclyl-C₁₋₄ alkyl (preferably, 5- to 8-membered heterocyclyl-C₁₋₄ alkyl, said 5-to 8-membered heterocycle has 1 to 3 hetero atoms selected from a nitrogen atom, an oxygen atom and an optionally oxidized sulfur atom, such as imidazolyl and the like), (v) optionally halogenated C₁₋₄ alkoxy, (vi) cyano, and (vii) carbamoyl optionally substituted by C₁₋₈ alkyl;
R^{1a} is a hydrogen atom;
R^{2a} is a C₁₋₈ alkyl group, a C₂₋₈ alkenyl group or a C₂₋₈ alkynyl group, each of which is substituted by substituent(s) selected from
(a) hydroxy,
(b) optionally halogenated C₁₋₄ alkoxy,
(c) -O-(CH₂)ₙ-OH,
(d) -O-(CH₂)ₙ-O-CO-NH₂,
(e) -O-(CH₂)ₙ-O-C₁₋₄ alkyl,
(f) -O-(CH₂)ₙ-SO₂-(optionally halogenated C₁₋₄ alkyl),
(g) -O-(CH₂)ₙ-SO₂-C₆₋₁₈ aryl,
(h) -O-(CH₂)ₙ-SO₂-(CH₂)ₙ-OH,
(i) -O-(CH₂)ₙ-NR⁸-SO₂-(optionally halogenated C₁₋₄ alkyl),
(j) -CO-NR⁸-(CH₂)ₙ-OH,
(k) -CO-NR⁸-(CH₂)ₙ-SO₂-(optionally halogenated C₁₋₄ alkyl),
(l) -NR⁶R⁷,
(m) -NR⁸-(CH₂)ₙ-OH,
(n) -NR⁸-(CH₂)ₙ-SO₂-C₁₋₄ alkyl,
(o) -NR⁸-CO-(CH₂)ₙ-OH,
(p) -NR⁸-CO-(CH₂)ₙ-O-C₁₋₄ alkyl,
(q) -NR⁸-CO-(CH₂)ₙ-SO-(optionally halogenated C₁₋₄ alkyl),
(r) -NR⁸-CO-(CH₂)ₙ-SO₂-(optionally halogenated C₁₋₄ alkyl),
(s) -NR⁸-CO-(CH₂)ₙ-SO₂-C₃₋₈ cycloalkyl,
(t) -NR⁸-CO₂-(CH₂)ₙ-SO₂-C₁₋₄ alkyl,
(u) -NR⁸-CO-NH-(CH₂)ₙ-SO₂-C₁₋₄ alkyl,
(v) -NR⁸-SO₂-(CH₂)ₙ-SO₂-C₁₋₄ alkyl,
(w) -S- (CH₂)ₙ-OH,
(x) -SO- (CH₂)ₙ-OH,
(y) -SO₂-(CH₂)ₙ-OH, and
(z) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and (CH₂)ₙ is optionally substituted by C₁₋₄ alkyl or hydroxy;
   R^{3a} is a hydrogen atom or a C₁₋₆ alkyl group; or
   R^{1a} and R^{2a} are optionally bonded to form

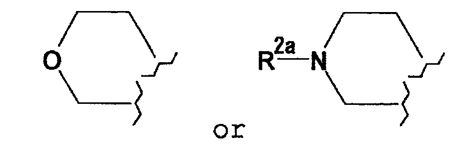

; and R^{2a} and R^{3a} are optionally bonded to form C₂₋₄ alkylene, is preferable.

Of these, as R^{2a}, a C₁₋₈ alkyl group, a C₂₋₈ alkenyl group or a C₂₋₈ alkynyl group (particularly, a C₁₋₈ alkyl group), each of which is substituted by substituent(s) selected from
(a) hydroxy,
(b) optionally halogenated C₁₋₄ alkoxy,
(c) -O-(CH₂)ₙ-OH (wherein (CH₂)ₙ is optionally substituted by hydroxy),
(d) -O-(CH₂)ₙ-O-CO-NH₂,
(e) -O-(CH₂)ₙ-O-C₁₋₄ alkyl,
(f) -O-(CH₂)ₙ-SO₂-(optionally halogenated C₁₋₄ alkyl),
(g) -O-(CH₂)ₙ-SO₂-C₆₋₁₈ aryl,
(h) -O-(CH₂)ₙ-SO₂-(CH₂)ₙ-OH,
(i) -O-(CH₂)ₙ-NR⁸-SO₂-(optionally halogenated C₁₋₄ alkyl),
(j) -CO-NR⁸-(CH₂)ₙ-OH,
(k) -CO-NR⁸-(CH₂)ₙ-SO₂-(optionally halogenated C₁₋₄ alkyl),
(l) -NR⁶R⁷,
(m) -NR⁸-(CH₂)ₙ-OH,
(n) -NR⁸-(CH₂)ₙ-SO₂-C₁₋₄ alkyl,
(o) -NR⁸-CO-(CH₂)ₙ-OH (wherein (CH₂)ₙ is optionally substituted by C₁₋₄ alkyl),
(p) -NR⁸-CO-(CH₂)ₙ-O-C₁₋₄ alkyl,
(q) -NR⁸-CO-(CH₂)ₙ-SO-(optionally halogenated C₁₋₄ alkyl),
(r) -NR⁸-CO-(CH₂)ₙ-SO₂-(optionally halogenated C₁₋₄ alkyl) (wherein (CH₂)ₙ is optionally substituted by C₁₋₄ alkyl),
(s) -NR⁸-CO-(CH₂)ₙ-SO₂-C₃₋₈ cycloalkyl,
(t) -NR⁸-CO₂-(CH₂)ₙ-SO₂-C₁₋₄ alkyl,
(u) -NR⁸-CO-NH-(CH₂)ₙ-SO₂-C₁₋₄ alkyl,
(v) -NR⁸-SO₂-(CH₂)ₙ-SO₂-C₁₋₄ alkyl,
(w) -S-(CH₂)ₙ-OH,
(x) -SO-(CH₂)ₙ-OH,
(y) -SO₂-(CH₂)ₙ-OH, and
(z) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, R⁸ is a hydrogen atom or a C₁₋₄ alkyl group, is preferable.

As R^{2a}, (i) a C₅₋₈ alkyl group substituted by hydroxy, (ii) a C₁₋₈ alkyl group substituted by substituent(s) selected from
(a) halogenated C₁₋₄ alkoxy,
(b) -O-(CH₂)ₙ-OH,
(c) -O-(CH₂)ₙ-O-CO-NH₂,
(d) -O-(CH₂)ₙ-O-(optionally halogenated C₁₋₄ alkyl),
(e) -O-(CH₂)ₙ-SO₂-(optionally halogenated C₁₋₄ alkyl) ,
(f) -O-(CH₂)ₙ-SO₂-C₆₋₁₈ aryl,
(g) -O-(CH₂)ₙ-NR⁸-SO₂-(optionally halogenated C₁₋₄ alkyl),
(h) -CO-NR⁸-(CH₂)ₙ-OH,
(i) -CO-NR⁸-(CH₂)ₙ-SO₂-(optionally halogenated C₁₋₄ alkyl),
(j) -NR⁸-(CH₂)ₙ-SO₂-C₁₋₄ alkyl,
(k) -NR⁸-CO-(CH₂)ₙ-OH,
(l) -NR⁸-CO-(CH₂)ₙ-O-C₁₋₄ alkyl,
(m) -NR⁸-CO-(CH₂)ₙ-SO-(optionally halogenated C₁₋₄ alkyl),
(n) -NR⁸-CO-(CH₂)ₙ-SO₂-(optionally halogenated C₁₋₄ alkyl),
(o) -NR⁸-CO-(CH₂)ₙ-SO₂-C₃₋₈ cycloalkyl,
(p) -NR⁸-CO₂-(CH₂)ₙ-SO₂-C₁₋₄ alkyl,
(q) -NR⁸-CO-NH-(CH₂)ₙ-SO₂-C₁₋₄ alkyl,
(r) -NR⁸-SO₂-(CH₂)ₙ-SO₂-C₁₋₄ alkyl,
(s) -S-(CH₂)ₙ-OH,
(t) -SO-(CH₂)ₙ-OH,
(u) -SO₂-(CH₂)ₙ-OH, and
(v) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁸ is a hydrogen atom or a C₁₋₄ alkyl group, and (CH₂)ₙ is optionally substituted by C₁₋₄ alkyl or hydroxy,
   (iii) a C₂₋₈ alkenyl group optionally substituted by hydroxy, or
   (iv) a C₂₋₈ alkynyl group optionally substituted by hydroxy is preferable, and particularly, as R^{2a}, (i) a C₅₋₈ alkyl group substituted by hydroxy,
   (ii) a C₁₋₈ alkyl group substituted by substituent(s) selected from
   (a) halogenated C₁₋₄ alkoxy,
   (b) -O-(CH₂)ₙ-OH (wherein (CH₂)ₙ is optionally substituted by hydroxy),
   (c) -O-(CH₂)ₙ-O-CO-NH₂,
   (d) -O-(CH₂)ₙ-O-(optionally halogenated C₁₋₄ alkyl),
   (e) -O-(CH₂)ₙ-SO₂-(optionally halogenated C₁₋₄ alkyl),
   (f) -O-(CH₂)ₙ-SO₂-C₆₋₁₈ aryl,
   (g) -O-(CH₂)ₙ-NR⁸-SO₂-(optionally halogenated C₁₋₄ alkyl),
   (h) -CO-NR⁸-(CH₂)ₙ-OH,
   (i) -CO-NR⁸-(CH₂)ₙ-SO₂-(optionally halogenated C₁₋₄ alkyl),
   (j) -NR⁸-(CH₂)ₙ-SO₂-C₁₋₄ alkyl,
   (k) -NR⁸-CO-(CH₂)ₙ-OH (wherein (CH₂)ₙ is optionally substituted by C₁₋₄ alkyl),
   (l) -NR⁸-CO-(CH₂)ₙ-O-C₁₋₄ alkyl,
   (m) -NR⁸-CO-(CH₂)ₙ-SO-(optionally halogenated C₁₋₄ alkyl),
   (n) -NR⁸-CO-(CH₂)ₙ-SO₂-(optionally halogenated C₁₋₄ alkyl) (wherein (CH₂)ₙ is optionally substituted by C₁₋₄ alkyl),
   (o) -NR⁸-CO-(CH₂)ₙ-SO₂-C₃₋₈ cycloalkyl,
   (p) -NR⁸-CO₂-(CH₂)ₙ-SO₂-C₁₋₄ alkyl,
   (q) -NR⁸-CO-NH-(CH₂)ₙ-SO₂-C₁₋₄ alkyl,
   (r) -NR⁸-SO₂-(CH₂)ₙ-SO₂-C₁₋₄ alkyl,
   (s) -S-(CH₂)ₙ-OH,
   (t) -SO- (CH₂)ₙ-OH,
   (u) -SO₂-(CH₂)ₙ-OH, and
   (v) -NR⁸-CO-(optionally substituted heterocyclic group)
      (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
      wherein n is an integer of 1 to 4, and R⁸ is a hydrogen atom or a C₁₋₄ alkyl group,
      (iii) a C₂₋₈ alkenyl group optionally substituted by hydroxy, or (iv) a C₂₋₈ alkynyl group optionally substituted by hydroxy is preferable, and as R⁸, a hydrogen atom, methyl, ethyl and the like are preferable, and a hydrogen atom is particularly preferable.

### [compound (Ib)]

A compound represented by the formula:

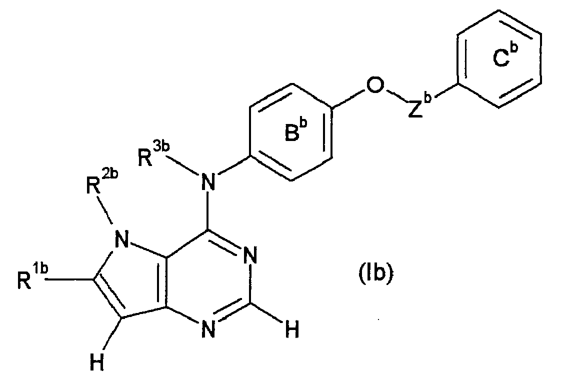

wherein R^{1b} is a hydrogen atom or an optionally substituted group bonded via a carbon atom, a nitrogen atom or an oxygen atom,
R^{2b} is an optionally substituted group bonded via a carbon atom or a sulfur atom, or
R^{1b} and R^{2b}, or R^{2b} and R^{3b} are optionally bonded to each other to form an optionally substituted ring structure,
R^{3b} is a hydrogen atom or an optionally substituted aliphatic hydrocarbon group, or R^{3b} is optionally bonded to a carbon atom of the adjacent phenyl group to form an optionally substituted ring structure,
B^{b} is an optionally substituted benzene ring, C^{b} is an optionally substituted C₆₋₁₈ aryl group, and
Z^{b} is an optionally substituted C₁₋₃ alkylene group.

As the "optionally substituted group bonded via a carbon atom, a nitrogen atom or an oxygen atom" for R^{1b}, those similar to the "optionally substituted group bonded via a carbon atom, a nitrogen atom or an oxygen atom" for R¹ can be used.
As R^{1b}, a hydrogen atom is preferable.
As the "optionally substituted group bonded via a carbon atom or a sulfur atom" for R^{2b}, those similar to the "optionally substituted group bonded via a carbon atom or a sulfur atom" for R² can be used.
As the "optionally substituted ring structure" formed by R^{1b} and R^{2b}, or R^{2b} and R^{3b} bonded to each other, those similar to the "optionally substituted ring structure" formed by R¹ and R², or R² and R³ bonded to each other can be used.
As the "optionally substituted aliphatic hydrocarbon group" for R^{3b}, those similar to the "optionally substituted aliphatic hydrocarbon group" for R³ can be used.

As the "optionally substituted ring structure" formed by R^{3b} and a carbon atom of the adjacent phenyl group, those similar to the "optionally substituted ring structure" formed by R³ and a carbon atom of the adjacent phenyl group can be used.
As R^{3b}, a hydrogen atom is preferable.
As the "optionally substituted benzene ring" for B^{b}, those similar to the "optionally substituted benzene ring" for B^{a} can be used.
As the substituent of the "optionally substituted benzene ring" for B^{b}, halogen and the like are preferable and, for example, chlorine and the like can be mentioned, and halogen (e.g., chlorine) is preferable. As B^{b}, a benzene ring wherein the 1-position of the ring is the carbon atom bonded to N and the 3-position is substituted by chlorine and the like can be mentioned.
As the "optionally substituted C₆₋₁₈ aryl group" for C^{b}, those similar to the "optionally substituted C₆₋₁₈ aryl group" for C^{a} can be used. As the "C₆₋₁₈ aryl group" of the "optionally substituted C₆₋₁₈ aryl group" for C^{b}, phenyl is preferable.
As the substituent of the "optionally substituted C₆₋₁₈ aryl group" for C^{b}, halogen and the like are preferable and, for example, fluorine and the like can be mentioned, and halogen (e.g., fluorine) is preferable.
As C^{b}, for example, 3-fluorophenyl and the like can be mentioned.
As the "C₁₋₃ alkylene group" of the "optionally substituted C₁₋₃ alkylene group" for Z^{b}, methylene, ethylene, trimethylene and propylene can be used. Of these, methylene is preferable.
As the "substituent" of the "optionally substituted C₁₋₃ alkylene group" for Z^{b}, 1 to 3 substituents selected from halogen, hydroxy, C₁₋₄ alkoxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino can be used.

As R^{2b}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from
(a) halogen,
(b) oxo,
(c) optionally halogenated C₁₋₄ alkyl,
(d) -(CH₂)ₘ-Q,
(e) -(CH₂)ₘ-Z¹-(optionally halogenated C₁₋₄ alkyl),
(f) -(CH₂)ₘ-Z¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂)ₘ-Z²-C₁₋₄ alkoxy, and
(l) -(CH₂)ₘ-Z²-(CH₂)ₙ-Z¹-(CH₂)ₙ-Z¹-C₁₋₄ alkyl
   wherein m is an integer of 0 to 4, n is an integer of 1 to 4, Q is hydroxy, carboxy, cyano, nitro, -NR⁶R⁷, -CONR⁶R⁷, -OCONH₂ or -SO₂NR⁶R⁷,
   Z¹ is -O-, -CO-, -C(OH)R⁸-, -C(=N-OR⁸)-, -S-, -SO-, -SO₂-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-SO₂-, or -NR⁸-C(=NH)-NH-, Z² is -O-, -CO-, -C(OH)R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-C(=NH)-NH-, -NR⁸-SO₂-, or -SO₂-NR⁸-,
   (CH₂)ₘ and (CH₂)ₙ are optionally substituted by 1 to 5 substituents selected from halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH- or -C≡C-,
   R⁶ and R⁷ are the same or different and each is a hydrogen atom or a C₁₋₄ alkyl group, or R⁶ and R⁷ are bonded to form, together with a nitrogen atom, a 3- to 8-membered saturated or unsaturated aliphatic heterocyclic group,
   R⁸ is a hydrogen atom or C₁₋₄ alkyl, and R⁹ is C₁₋₄ alkyl, is preferable.
As R^{2b}, optionally substituted C₁₋₈ alkyl group and the like are preferable and, for example, ethyl wherein the 2-position is substituted and the like can be mentioned. As the substituent of the optionally substituted C₁₋₈ alkyl group, (g) -(CH₂)ₘ-Z²-(CH₂)ₙ-Q in the above-mentioned substituent group and the like are preferable, and particularly, that wherein m is 0, Z² is -O- and Q is hydroxy, namely, -O-(CH₂)ₙ-OH and the like are preferable. In the formula, n is preferably 2.
As the substituent of the optionally substituted C₁₋₈ alkyl group for R^{2b}, for example, -O-CH₂-CH₂-OH and the like can be mentioned.

As compound (Ib), a compound wherein
B^{b} is a benzene ring optionally substituted by halogen;
C^{b} is a phenyl group optionally substituted by 1 to 5 substituents selected from halogen, optionally halogenated C₁₋₄ alkyl and cyano;
R^{1b} is (i) a hydrogen atom, or
(ii) a C₂₋₄ alkenyl group optionally substituted by hydroxy;
R^{2b} is
(i) a C₁₋₈ alkyl group optionally substituted by substituent(s) selected from
   (a) halogen,
   (b) hydroxy,
   (c) C₁₋₄ alkoxy,
   (d) -O-(CH₂)ₙ-OH,
   (e) -O-(CH₂)ₙ-O-C₁₋₄ alkyl,
   (f) -CO-NR⁸-(CH₂)ₙ-OH,
   (g) -NR⁶R⁷, and
   (h) -NR⁸-(CH₂)ₙ-OH,
   wherein n is an integer of 1 to 4, R⁶ and R⁷ are the same or different and each is a hydrogen atom or a C₁₋₄ alkyl group, and R⁸ is a hydrogen atom or a C₁₋₄ alkyl group,
(ii) a C₆₋₁₈ aryl-C₁₋₄ alkyl group optionally substituted by substituent(s) selected from
   (a) C₁₋₄ alkyl optionally having hydroxy,
   (b) carboxy,
   (c) C₁₋₄ alkoxy-carbonyl,
   (d) 5- to 8-membered heterocyclyl-carbonyl having 1 to 3 hetero atoms selected from a nitrogen atom, an oxygen atom and a sulfur atom, which optionally has substituent(s) selected from hydroxy and C₁₋₄ alkyl, and
   (e) C₁₋₄ alkyl-carbamoyl optionally having substituent(s) selected from hydroxy and carbamoyl,
(iii) a C₆₋₁₈ aryl-carbonyl group optionally substituted by C₁₋₄ alkoxy,
(iv) a C₆₋₁₈ aryl-sulfonyl group optionally substituted by C₁₋₄ alkoxy, or
(v) a 5- to 8-membered heterocyclyl-C₁₋₄ alkyl group having 1 to 3 hetero atoms selected from a nitrogen atom, an oxygen atom and a sulfur atom, which is optionally substituted by substituent(s) selected from
   (a) carboxy, and
   (b) C₁₋₄ alkoxy-carbonyl;
      R^{3b} is a hydrogen atom or a C₁₋₆ alkyl group; or
      R^{2b} and R^{3b} are optionally bonded to form C₂₋₄ alkylene; and
      Z^{b} is a C₁₋₃ alkylene group, is preferable.

Moreover, as compound (Ib), a compound wherein B^{b} is a benzene ring optionally substituted by halogen;
C^{b} is a phenyl group optionally substituted by 1 to 5 substituents selected from halogen and optionally halogenated C₁₋₄ alkyl;
R^{1b} is a hydrogen atom;
R^{2b} is a C₁₋₈ alkyl group optionally substituted by substituent(s) selected from
(a) hydroxy,
(b) -O-(CH₂)ₙ-OH,
(c) -O-(CH₂)ₙ-O-C₁₋₄ alkyl,
(d) -CO-NR⁸-(CH₂)ₙ-OH,
(e) -NR⁶R⁷, and
(f) -NR⁸-(CH₂)ₙ-OH,
   wherein n is an integer of 1 to 4, R⁶ and R⁷ are the same or different and each is a hydrogen atom or a C₁₋₄ alkyl group, and R⁸ is a hydrogen atom or a C₁₋₄ alkyl group;
   R^{3b} is a hydrogen atom or a C₁₋₆ alkyl group; and
   Z^{b} is a C₁₋₃ alkylene group is preferable.

Particularly, as compound (Ib), a compound wherein B^{b} is a benzene ring optionally substituted by halogen;
C^{b} is a phenyl group optionally substituted by 1 to 5 substituents selected from halogen and optionally halogenated C₁₋₄ alkyl;
R^{1b} is a hydrogen atom;
R^{2b} is a C₁₋₈ alkyl group substituted by substituent(s) selected from
(a) -O-(CH₂)ₙ-OH,
(b) -O-(CH₂)ₙ-O-C₁₋₄ alkyl, and
(c) -CO-NR⁸-(CH₂)ₙ-OH,
   wherein n is an integer of 1 to 4, and R⁸ is a hydrogen atom or a C₁₋₄ alkyl group;
   R^{3b} is a hydrogen atom or a C₁₋₆ alkyl group; and
   Z^{b} is a methylene group is preferable.
As R⁸, a hydrogen atom, methyl, ethyl and the like are preferable, and a hydrogen atom is particularly preferable.

### [Compound (Ic)]

A compound represented by the formula:

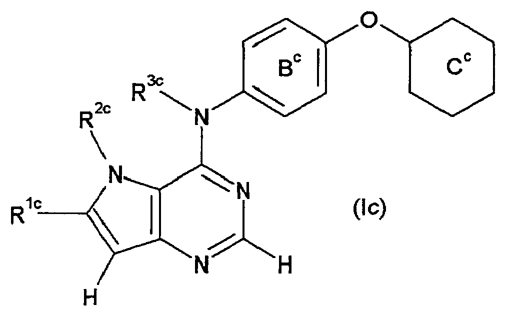

wherein R^{1c} is a hydrogen atom or an optionally substituted group bonded via a carbon atom, a nitrogen atom or an oxygen atom,
R^{2c} is an optionally substituted group bonded via a carbon atom or a sulfur atom, or
R^{1c} and R^{2c}, or R^{2c} and R^{3c} are optionally bonded to each other to form an optionally substituted ring structure,
R^{3c} is a hydrogen atom or an optionally substituted aliphatic hydrocarbon group, or R^{3c} is optionally bonded to a carbon atom of the adjacent phenyl group to form an optionally substituted ring structure,
B^{c} is an optionally substituted benzene ring, and C^{c} is an optionally substituted heterocyclic group.

As the "optionally substituted group bonded via a carbon atom, a nitrogen atom or an oxygen atom" for R^{1c}, those similar to the "optionally substituted group bonded via a carbon atom, a nitrogen atom or an oxygen atom" for R¹ can be used.
As the "optionally substituted group bonded via a carbon atom or a sulfur atom" for R^{2c}, those similar to the "optionally substituted group bonded via a carbon atom or a sulfur atom" for R² can be used.
As the "optionally substituted ring structure" formed by R^{1c} and R^{2c}, or R^{2c} and R^{3c} bonded to each other, those similar to the "optionally substituted ring structure" formed by R¹ and R², or R² and R³ bonded to each other can be used.
As the "optionally substituted aliphatic hydrocarbon group" for R^{3c}, those similar to the "optionally substituted aliphatic hydrocarbon group" for R³ can be used.

As the "optionally substituted ring structure" formed by R^{3c} and a carbon atom of the adjacent phenyl group, those similar to the "optionally substituted ring structure" formed by R³ and a carbon atom of the adjacent phenyl group can be used.
As the "optionally substituted benzene ring" for B^{c}, those similar to the "optionally substituted benzene ring" for B^{a} can be used.
As the "heterocyclic group" of the "optionally substituted heterocyclic group" for C^{c}, the aforementioned "heterocyclic group" can be used, and a 5- to 8-membered heterocyclic group having 1 to 3 hetero atoms selected from a nitrogen atom, an oxygen atom and an optionally oxidized sulfur atom can be particularly preferably used. Specifically, 5- or 6-membered aromatic monocyclic heterocyclic groups such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like, 3- to 8-membered (preferably 5- or 6-membered) saturated or unsaturated (preferably saturated) aliphatic heterocyclic groups such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, dihydro-1,2,4-oxadiazolyl and the like can be used, and particularly, pyridyl, pyrimidinyl, piperidyl (particularly, 4-piperidyl) and the like are preferable.
As the "substituent" of the "optionally substituted heterocyclic group" for C^{c}, those similar to the "substituent" of the "optionally substituted C₆₋₁₈ aryl group" for C^{a} can be used.

As R^{2c}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from the group consisting of
(a) halogen,
(b) oxo,
(c) optionally halogenated C₁₋₄ alkyl,
(d) -(CH₂)ₘ-Q,
(e) -(CH₂)ₘ-Z¹-(optionally halogenated C₁₋₄ alkyl),
(f) -(CH₂)ₘ-Z¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group)
   (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂)ₘ-Z²-C₁₋₄ alkoxy, and
(l) -(CH₂)ₘ-Z²-(CH₂)ₙ-Z¹-(CH₂)ₙ-Z¹-C₁₋₄ alkyl
   wherein m is an integer of 0 to 4, n is an integer of 1 to 4, Q is hydroxy, carboxy, cyano, nitro, -NR⁶R⁷, -CONR⁶R⁷ or -SO₂NR⁶R⁷,
   Z¹ is -O-, -CO-, -C(OH)R⁸-, -C(=N-OR⁸)-, -S-, -SO-, -SO₂-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-SO₂-, or -NR⁸-C(=NH)-NH-, Z² is -O-, -CO-, -C(OH)R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-C(=NH)-NH-, -NR⁸-SO₂-, or -SO₂-NR⁸-,
   (CH₂)ₘ and (CH₂)ₙ are optionally substituted by 1 to 5 substituents selected from halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH-,
   R⁶ and R⁷ are the same or different and each is a hydrogen atom or a C₁₋₄ alkyl group, or R⁶ and R⁷ are bonded to form, together with a nitrogen atom, a 3- to 8-membered saturated or unsaturated aliphatic heterocyclic group,
   R⁸ is a hydrogen atom or C₁₋₄ alkyl, and R⁹ is C₁₋₄ alkyl, is preferable.

As compound (Ic), a compound wherein
B^{c} is a benzene ring optionally substituted by 1 to 4 substituents selected from halogen and optionally halogenated C₁₋₄ alkyl;
C^{c} is a 5- to 8-membered heterocyclic group having 1 to 3 hetero atoms selected from a nitrogen atom, an oxygen atom and a sulfur atom (e.g., pyridyl, pyrimidyl, 4-piperidyl), which is optionally substituted by 1 to 5 substituents selected from
(i) halogen,
(ii) C₁₋₄ alkyl,
(iii) C₁₋₄ alkyl-carbonyl,
(iv) optionally halogenated C₁₋₄ alkoxy-carbonyl,
(v) C₃₋₈ cycloalkyl-carbonyl, and
(vi) a carbamoyl group optionally substituted by substituent(s) selected from
   (a) optionally halogenated C₁₋₈ alkyl,
   (b) C₃₋₈ cycloalkyl, and
   (c) C₆₋₁₈ aryl optionally substituted by substituent(s) selected from halogen, C₁₋₄ alkyl and C₁₋₄ alkoxy;
      R^{1c} is (i) a hydrogen atom,
      (ii) a C₂₋₄ alkenyl group optionally substituted by hydroxy, or
      (iii) a 5- to 8-membered heterocyclic group having 1 to 3 hetero atoms selected from a nitrogen atom, an oxygen atom and a sulfur atom;
      R^{2c} is
      (i) a C₁₋₄ alkyl group optionally substituted by substituent(s) selected from
      (a) halogen,
      (b) hydroxy,
      (c) C₁₋₄ alkoxy,
      (d) carboxy,
      (e) C₁₋₄ alkoxy-carbonyl,
      (f) -O-(CH₂)ₙ-OH,
      (g) -O-(CH₂)ₙ-O-C₁₋₄ alkyl,
      (h) -CO-NR⁸-(CH₂)ₙ-OH, and
         (i) -NR⁸-CO-(CH₂)ₙ-SO₂-C₁₋₄ alkyl
            wherein n is an integer of 1 to 4, and R⁸ is a hydrogen atom or a C₁₋₄ alkyl group, or
         (ii) a C₆₋₁₈ aryl-C₁₋₄ alkyl group optionally substituted by C₁₋₄ alkyl optionally having hydroxy; and
            R^{3c} is a hydrogen atom or a C₁₋₆ alkyl group; or
            R^{2c} and R^{3c} are optionally bonded to form C₂₋₄ alkylene, is preferable.

Moreover, as compound (Ic), a compound wherein
B^{c} is a benzene ring optionally substituted by 1 to 4 substituents selected from halogen and C₁₋₄ alkyl;
C^{c} is a 5- to 8-membered heterocyclic group having 1 to 3 hetero atoms selected from a nitrogen atom, an oxygen atom and a sulfur atom, which is optionally substituted by 1 to 5 substituents selected from
(i) C₁₋₄ alkyl,
(ii) C₁₋₄ alkyl-carbonyl,
(iii) optionally halogenated C₁₋₄ alkoxy-carbonyl,
(iv) C₃₋₈ cycloalkyl-carbonyl, and
(v) a carbamoyl group optionally substituted by substituent(s) selected from
   (a) optionally halogenated C₁₋₈ alkyl,
   (b) C₃₋₈ cycloalkyl, and
   (c) C₆₋₁₈ aryl optionally substituted by halogen;
      R^{1c} is a hydrogen atom;
      R^{2c} is a C₁₋₄ alkyl group optionally substituted by substituent(s) selected from
   (a) hydroxy,
   (b) C₁₋₄ alkoxy,
   (c) -O-(CH₂)ₙ-OH,
   (d) -O-(CH₂)ₙ-O-C₁₋₄ alkyl, and
   (e) -NR⁸-CO-(CH₂)ₙ-SO₂-C₁₋₄ alkyl
      wherein n is an integer of 1 to 4, and R⁸ is a hydrogen atom or a C₁₋₄ alkyl group; and
      R^{3c} is a hydrogen atom or a C₁₋₆ alkyl group, is preferable, particularly, a compound wherein R^{2c} is a C₁₋₄ alkyl group optionally substituted by substituent(s) selected from
   (a) -O-(CH₂)ₙ-OH, and
   (b) -O-(CH₂)ₙ-O-C₁₋₄ alkyl,
      wherein n is an integer of 1 to 4, is preferable.

### [Compound (Id)]

A compound represented by the formula

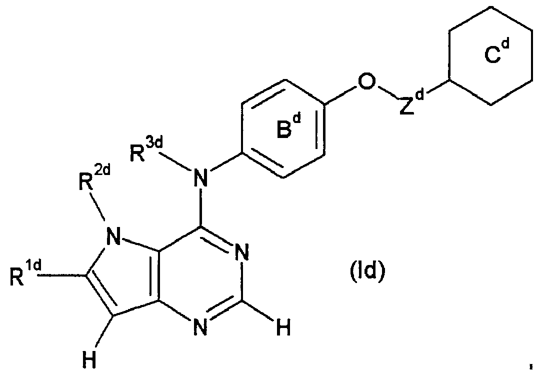

wherein R^{1d} is a hydrogen atom or an optionally substituted group bonded via a carbon atom, a nitrogen atom or an oxygen atom,
R^{2d} is an optionally substituted group bonded via a carbon atom or a sulfur atom, or
R^{1d} and R^{2d}, or R^{2d} and R^{3d} are optionally bonded to each other to form an optionally substituted ring structure,
R^{3d} is a hydrogen atom or an optionally substituted aliphatic hydrocarbon group, or R^{3d} is optionally bonded to a carbon atom of the adjacent phenyl group to form an optionally substituted ring structure,
B^{d} is an optionally substituted benzene ring, C^{d} is an optionally substituted heterocyclic group, and Z^{d} is an optionally substituted C₁₋₃ alkylene group.

As the "optionally substituted group bonded via a carbon atom, a nitrogen atom or an oxygen atom" for R^{1d}, those similar to the "optionally substituted group bonded via a carbon atom, a nitrogen atom or an oxygen atom" for R¹ can be used.
As the "optionally substituted group bonded via a carbon atom or a sulfur atom" for R^{2d}, those similar to the "optionally substituted group bonded via a carbon atom or a sulfur atom" for R² can be used.
As the "optionally substituted ring structure" formed by R^{1d} and R^{2d}, or R^{2d} and R^{3d} bonded to each other, those similar to the "optionally substituted ring structure" formed by R¹ and R², or R² and R³ bonded to each other can be used.
As the "optionally substituted aliphatic hydrocarbon group" for R^{3d}, those similar to the "optionally substituted aliphatic hydrocarbon group" for R³ can be used.
As the "optionally substituted ring structure" formed by R^{3d} and a carbon atom of the adjacent phenyl group, those similar to the "optionally substituted ring structure" formed by R³ and a carbon atom of the adjacent phenyl group can be used.
As the "optionally substituted benzene ring" for B^{d}, those similar to the "optionally substituted benzene ring" for B^{a} can be used.
As the "optionally substituted heterocyclic group" for C^{d}, those similar to the "optionally substituted heterocyclic group" for C^{c} can be used.
As the "optionally substituted C₁₋₃ alkylene ring" for Z^{d}, those similar to the "optionally substituted C₁₋₃ alkylene ring" for Z^{b} can be used.

As R^{2d}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from
(a) halogen,
(b) oxo,
(c) optionally halogenated C₁₋₄ alkyl,
(d) -(CH₂)ₘ-Q,
(e) -(CH₂)ₘ-Z¹-(optionally halogenated C₁₋₄ alkyl),
(f) -(CH₂)ₘ-Z¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂)ₘ-Z²-C₁₋₄ alkoxy, and
(l) -(CH₂)ₘ-Z²-(CH₂)ₙ-Z¹-(CH₂)ₙ-Z¹-C₁₋₄ alkyl
   wherein m is an integer of 0 to 4, n is an integer of 1 to 4, Q is hydroxy, carboxy, cyano, nitro, -NR⁶R⁷, -CONR⁶R⁷ or -SO₂NR⁶R⁷,
   Z¹ is -O-, -CO-, -C(OH)R⁸-, -C(=N-OR⁸)-, -S-, -SO-, -SO₂-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-SO₂-, or -NR⁸-C(=NH)-NH-, Z² is -O-, -CO-, -C(OH)R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-C(=NH)-NH-, -NR⁸-SO₂-, or -SO₂-NR⁸-,
   (CH₂)ₘ and (CH₂)ₙ are optionally substituted by 1 to 5 substituents selected from halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH-,
   R⁶ and R⁷ are the same or different and each is a hydrogen atom, or a C₁₋₄ alkyl group, or R⁶ and R⁷ are bonded to form, together with a nitrogen atom, a 3- to 8-membered saturated or unsaturated aliphatic heterocyclic group,
   R⁸ is a hydrogen atom or C₁₋₄ alkyl, and R⁹ is C₁₋₄ alkyl, is preferable.

As compound (Id), a compound wherein
B^{d} is a benzene ring optionally substituted by halogen;
C^{d} is a 5- to 8-membered heterocyclic group having 1 to 3 hetero atoms selected from a nitrogen atom, an oxygen atom and a sulfur atom;
R^{1d} is a hydrogen atom;
R^{2d} is
(i) C₁₋₄ alkyl optionally substituted by substituent(s) selected from
   (a) C₁₋₄ alkoxy
   (b) -O-(CH₂)ₙ-OH, and
   (c) -NR⁸-CO-(CH₂)ₙ-SO₂-C₁₋₄ alkyl
      wherein n is an integer of 1 to 4, and R⁸ is a hydrogen atom or a C₁₋₄ alkyl group, or
(ii) a 5- to 8-membered heterocyclyl-C₁₋₄ alkyl group having 1 to 3 hetero atoms selected from a nitrogen atom, an oxygen atom and a sulfur atom, which is optionally substituted by substituent(s) selected from
   (a) carboxy, and
   (b) C₁₋₄ alkoxy-carbonyl;
      R^{3d} is a hydrogen atom or a C₁₋₆ alkyl group; and
      Z^{d} is a C₁₋₃ alkylene group, is preferable.

Moreover, as compound (Id), a compound wherein
B^{d} is a benzene ring optionally substituted by halogen;
C^{d} is a 5- to 8-membered heterocyclic group having 1 to 3 hetero atoms selected from a nitrogen atom, an oxygen atom and a sulfur atom;
R^{1d} is a hydrogen atom,
R^{2d} is a C₁₋₄ alkyl group optionally substituted by C₁₋₄ alkoxy, R^{3d} is a hydrogen atom or a C₁₋₆ alkyl group; and
Z^{d} is a methylene group, is preferable.

### [Compound (Ie)]

A compound represented by the formula:

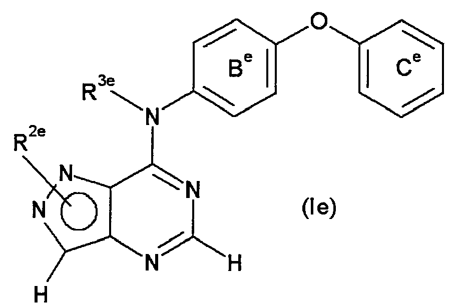

wherein R^{2e} is an optionally substituted group bonded via a carbon atom or a sulfur atom, or
R^{2e} and R^{3e} are optionally bonded to each other to form an optionally substituted ring structure,
R^{3e} is a hydrogen atom or an optionally substituted aliphatic hydrocarbon group, or R^{3e} is optionally bonded to a carbon atom of the adjacent phenyl group to form an optionally substituted ring structure,
B^{e} is an optionally substituted benzene ring, and C^{e} is an optionally substituted C₆₋₁₈ aryl group.

As the "optionally substituted group bonded via a carbon atom or a sulfur atom" for R^{2e}, those similar to the "optionally substituted group bonded via a carbon atom or a sulfur atom" for R² can be used.
As the "optionally substituted ring structure" formed by R^{2e} and R^{3e} bonded to each other, those similar to the "optionally substituted ring structure" formed by R² and R³ bonded to each other can be used.
As the "optionally substituted aliphatic hydrocarbon group" for R^{3e}, those similar to the "optionally substituted aliphatic hydrocarbon group" for R³ can be used.
As the "optionally substituted ring structure" formed by R^{3e} and a carbon atom of the adjacent phenyl group, those similar to the "optionally substituted ring structure" formed by R³ and a carbon atom of the adjacent phenyl group can be used.
As the "optionally substituted benzene ring" for B^{e}, those similar to the "optionally substituted benzene ring" for B^{a} can be used.
As the "optionally substituted C₆₋₁₈ aryl group" for C^{e}, those similar to the "optionally substituted C₆₋₁₈ aryl group" for C^{a} can be used.

As R^{2e}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from
(a) halogen,
(b) oxo,
(c) optionally halogenated C₁₋₄ alkyl,
(d) -(CH₂)ₘ-Q,
(e) -(CH₂)ₘ-Z¹-(optionally halogenated C₁₋₄ alkyl),
(f) -(CH₂)ₘ-Z¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂)ₘ-Z²-C₁₋₄ alkoxy, and
(l) -(CH₂)ₘZ²-(CH₂)ₙ-Z¹-(CH₂)ₙ-Z¹-C₁₋₄ alkyl
   wherein m is an integer of 0 to 4, n is an integer of 1 to 4,
   Q is hydroxy, carboxy, cyano, nitro, -NR⁶R⁷, -CONR⁶R⁷ or -SO₂NR⁶R⁷,
   Z¹ is -O-, -CO-, -C(OH)R⁸-, -C(=N-OR⁸)-, -S-, -SO-, -SO₂-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-SO₂-, or -NR⁸-C(=NH)-NH-, Z² is -O-, -CO-, -C(OH)R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-C(=NH)-NH-, -NR⁸-SO₂-, or -SO₂-NR⁸-,
   (CH₂)ₘ and (CH₂)ₙ are optionally substituted by 1 to 5 substituents selected from halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH-,
   R⁶ and R⁷ are the same or different and each is a hydrogen atom or a C₁₋₄ alkyl group, or R⁶ and R⁷ are bonded to form, together with a nitrogen atom, a 3- to 8-membered saturated or unsaturated aliphatic heterocyclic group,
   R⁸ is a hydrogen atom or C₁₋₄ alkyl, and R⁹ is C₁₋₄ alkyl, is preferable.

As compound (Ie), a compound wherein
Be is a benzene ring optionally substituted by halogen;
C^{e} is a phenyl group optionally substituted by optionally halogenated C₁₋₄ alkyl; and
R^{2e} is a C₁₋₄ alkyl group optionally substituted by -O-(CH₂)ₙ-OH wherein n is an integer of 1 to 4, is preferable.
Moreover, as compound (Ie), a compound wherein
Be is a benzene ring optionally substituted by halogen;
C^{e} is a phenyl group optionally substituted by optionally halogenated C₁₋₄ alkyl; and
R^{2e} is a C₁₋₄ alkyl group substituted by -O-(CH₂)ₙ-OH wherein n is an integer of 1 to 4, is preferable.

### [Compound (If)]

A compound represented by the formula:

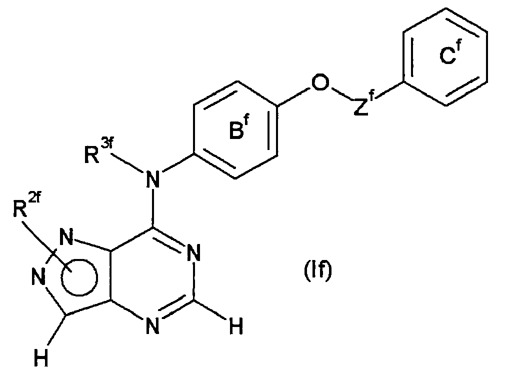

wherein R^{2f} is an optionally substituted group bonded via a carbon atom or a sulfur atom, or
R^{2f} and R^{3f} are optionally bonded to each other to form an optionally substituted ring structure,
R^{3f} is a hydrogen atom or an optionally substituted aliphatic hydrocarbon group, or R^{3f} is optionally bonded to a carbon atom of the adjacent phenyl group to form an optionally substituted ring structure,
B^{f} is an optionally substituted benzene ring, C^{f} is an optionally substituted C₆₋₁₈ aryl group, and
Z^{f} is an optionally substituted C₁₋₃ alkylene group.

As the "optionally substituted group bonded via a carbon atom or a sulfur atom" for R^{2f}, those similar to the "optionally substituted group bonded via a carbon atom or a sulfur atom" for R² can be used.
As the "optionally substituted ring structure" formed by R^{2f} and R^{3f} bonded to each other, those similar to the "optionally substituted ring structure" formed by R² and R³ bonded to each other can be used.
As the "optionally substituted aliphatic hydrocarbon group" for R^{3f}, those similar to the "optionally substituted aliphatic hydrocarbon group" for R³ can be used.
As the "optionally substituted ring structure" formed by R^{3f} and a carbon atom of the adjacent phenyl group, those similar to the "optionally substituted ring structure" formed by R³ and a carbon atom of the adjacent phenyl group can be used.
As the "optionally substituted benzene ring" for B^{f}, those similar to the "optionally substituted benzene ring" for B^{a} can be used.
As the "optionally substituted C₆₋₁₈ aryl group" for C^{f}, those similar to the "optionally substituted C₆₋₁₈ aryl group" for C^{a} can be used.

As the "optionally substituted C₁₋₃ alkylene group" for Z^{f}, those similar to the "optionally substituted C₁₋₃ alkylene group" for Z^{b} can be used. As R^{2f}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from
(a) halogen,
(b) oxo,
(c) optionally halogenated C₁₋₄ alkyl,
(d) -(CH₂)ₘ-Q,
(e) -(CH₂)ₘ Z¹-(optionally halogenated C₁₋₄ alkyl),
(f) -(CH₂)ₘZ¹-C₃₋₈ cycloalkyl,
(g) -(CH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group)
   (preferably, said heterocyclic group is a 5- to 8-membered heterocyclic group having 1 to 3 hetero atoms selected from a nitrogen atom, an oxygen atom and an optionally oxidized sulfur atom),
(k) -(CH2)ₘ-Z²-C₁₋₄ alkoxy, and
(l) -(CH₂)ₘ-Z²-(CH₂)ₙ-Z¹-(CH₂)ₙ-Z¹-C₁₋₄ alkyl
   wherein m is an integer of 0 to 4, n is an integer of 1 to 4, Q is hydroxy, carboxy, cyano, nitro, -NR⁶R⁷, -CONR⁶R⁷ or -SO₂NR⁶R⁷,
   Z¹ is -O-, -CO-, -C(OH)R⁸-, -C(=N-OR⁸)-, -S-, -SO-, -SO₂-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-SO₂-, or -NR⁸-C(=NH)-NH-, Z² is -O-, -CO-, -C(OH)R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-C(=NH)-NH-, -NR⁸-SO₂-, or -SO₂-NR⁸-,
   (CH₂)ₘ and (CH₂)ₙ are optionally substituted by 1 to 5 substituents selected from halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH-,
   R⁶ and R⁷ are the same or different and each is a hydrogen atom or a C₁₋₄ alkyl group, or R⁶ and R⁷ are bonded to form, together with a nitrogen atom, a 3- to 8-membered saturated or unsaturated aliphatic heterocyclic group,
   R⁸ is a hydrogen atom or C₁₋₄ alkyl, and R⁹ is C₁₋₄ alkyl, is preferable.

As compound (If), a compound wherein
B^{f} is a benzene ring optionally substituted by halogen;
C^{f} is a phenyl group optionally substituted by halogen;
R^{2f} is
(i) a C₁₋₄ alkyl group optionally substituted by 1 to 5 substituents selected from the group consisting of
   (a) hydroxy,
   (b) -O-(CH₂)ₙ-OH,
   (c) -NR⁸-(CH₂)ₙ-O-C₁₋₄ alkyl,
   (d) -NR⁸-(CH₂)ₙ-heterocyclic group (preferably, said heterocyclic group is a 5- to 8-membered heterocyclic group having 1 to 3 hetero atoms selected from a nitrogen atom, an oxygen atom and a sulfur atom), and
   (e) -NR⁸-(CH₂)ₙ-SO₂-C₁₋₄ alkyl,
      wherein n is an integer of 1 to 4, and R⁸ is a hydrogen atom or a C₁₋₄ alkyl group,
(ii) a C₆₋₁₈ aryl group optionally substituted by 1 to 5 substituents selected from the group consisting of
   (a) C₁₋₄ alkyl optionally substituted by substituent(s) selected from hydroxy, -NR⁸-(CH₂)ₙ-OH, -NR⁸-(CH₂)ₙ-O-C₁₋₄ alkyl,
      -NR⁸-(CH₂)ₙ-heterocyclic group (preferably, said heterocyclic group is a 5- to 8-membered heterocyclic group having 1 to 3 hetero atoms selected from a nitrogen atom, an oxygen atom and a sulfur atom) and -NR⁸-(CH₂)ₙ-SO₂-C₁₋₄ alkyl, and
   (b) -CO-NR⁸-(CH₂)ₙ-O-C₁₋₄ alkyl,
      wherein n is an integer of 1 to 4, and R⁸ is a hydrogen atom or a C₁₋₄ alkyl group, or
(iii) a C₆₋₁₈ aryl-C₁₋₄ alkyl group optionally substituted by 1 to 5 substituents selected from the group consisting of
   (a) carboxy,
   (b) C₁₋₄ alkoxy-carbonyl, and
   (c) -CO-NR⁸-(CH₂)ₙ-O-C₁₋₄ alkyl,
      wherein n is an integer of 1 to 4, and R⁸ is a hydrogen atom or a C₁₋₄ alkyl group,
      R^{3f} is a hydrogen atom or a C₁₋₆ alkyl group; and
      Z^{f} is a C₁₋₃ alkylene group; or
      R^{2f} and R^{3f} are optionally bonded to form C₂₋₄ alkylene, is preferable.
As R⁸, a hydrogen atom, methyl, ethyl and the like are preferably, and a hydrogen atom is particularly preferable.

Moreover, as compound (If), a compound wherein
B^{f} is a benzene ring optionally substituted by halogen;
C^{f} is a phenyl group optionally substituted by halogen;
R^{2f} is a C₁₋₄ alkyl group optionally substituted by 1 to 5 substituents selected from the group consisting of
(a) hydroxy, and
(b) -O-(CH₂)ₙ-OH wherein n is an integer of 1 to 4;
   R^{3f} is a hydrogen atom or a C₁₋₆ alkyl group;
   Z^{f} is methylene, is preferable, and particularly, a compound wherein R^{2f} is a C₁₋₄ alkyl group substituted by -O-(CH₂)ₙ-OH wherein n is an integer of 1 to 4, is preferable.

### [Compound (Ig)]

A compound represented by the formula:

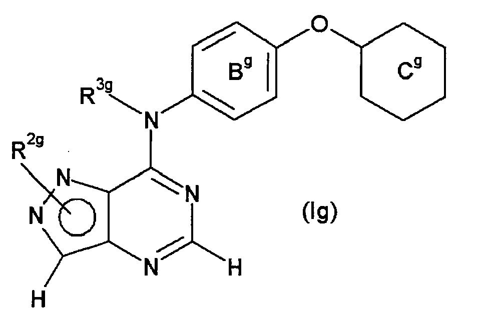

wherein R^{2g} is an optionally substituted group bonded via a carbon atom or a sulfur atom, or
R^{2g} and R^{3g} are optionally bonded to each other to form an optionally substituted ring structure,
R^{3g} is a hydrogen atom or an optionally substituted aliphatic hydrocarbon group, or R^{3g} is optionally bonded to a carbon atom of the adjacent phenyl group to form an optionally substituted ring structure,
B^{g} is an optionally substituted benzene ring, and C^{g} is an optionally substituted heterocyclic group.

As the "optionally substituted group bonded via a carbon atom or a sulfur atom" for R^{2g}, those similar to the "optionally substituted group bonded via a carbon atom or a sulfur atom" for R² can be used.
As the "optionally substituted ring structure" formed by R^{2g} and R^{3g} bonded to each other, those similar to the "optionally substituted ring structure" formed by R² and R³ bonded to each other can be used.
As the "optionally substituted aliphatic hydrocarbon group" for R^{3g}, those similar to the "optionally substituted aliphatic hydrocarbon group" for R³ can be used.
As the "optionally substituted ring structure" formed by R^{3g} and a carbon atom of the adjacent phenyl group, those similar to the "optionally substituted ring structure" formed by R³ and a carbon atom of the adjacent phenyl group can be used.
As the "optionally substituted benzene ring" for B^{g}, those similar to the "optionally substituted benzene ring" for B^{a} can be used.
As the "optionally substituted heterocyclic group" for C^{g}, those similar to the "optionally substituted heterocyclic group" for C^{c} can be used.

As R^{2g}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from the group consisting of
(a) halogen,
(b) oxo,
(c) optionally halogenated C₁₋₄ alkyl,
(d) -(CH₂)ₘ-Q,
(e) -(CH₂)ₘ-Z¹-(optionally halogenated C₁₋₄ alkyl),
(f) -(CH₂)ₘ-Z¹-C₃₋₈ cycloalkyl,
(g) -(OH₂)ₘ-Z²-(CH₂)ₙ-Q,
(h) -(CH₂)ₘ-Z²-(CH₂)ₙ-Z¹-(optionally halogenated C₁₋₄ alkyl),
(i) -(CH₂)ₘ-Z²-(CH₂)ₙ-Z¹-C₃₋₈ cycloalkyl,
(j) -(CH₂)ₘ-Z¹-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂)M-Z²-C₁₋₄ alkoxy, and
(l) -(CH₂)ₘ-Z²-(CH₂)ₙ-Z¹-(CH₂)ₙ-Z¹-C₁₋₄ alkyl
   wherein m is an integer of 0 to 4, n is an integer of 1 to 4,
   Q is hydroxy, carboxy, cyano, nitro, -NR⁶R⁷, -CONR⁶R⁷ or -SO₂NR⁶R⁷,
   Z¹ is -O-, -CO-, -C(OH)R⁸-, -C(=N-OR⁸)-, -S-, -SO-, -SO₂-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-SO₂-, or -NR⁸-C(=NH)-NH-, Z² is -O-, -CO-, -C(OH)R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-C(=NH)-NH-, -NR⁸-SO₂-, or -SO₂-NR⁸-,
   (CH₂)ₘ and (CH₂)ₙ are optionally substituted by 1 to 5 substituents selected from halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH-.
   R⁶ and R⁷ are the same or different and each is a hydrogen atom or a C₁₋₄ alkyl group, or R⁶ and R⁷ are bonded to form, together with a nitrogen atom, a 3- to 8-membered saturated or unsaturated aliphatic heterocyclic group,
   R⁸ is a hydrogen atom or C₁₋₄ alkyl, and R⁹ is C₁₋₄ alkyl, is preferable.

As compound (Ig), a compound wherein
B^{g} is a benzene ring optionally substituted by C₁₋₄ alkyl;
C^{g} is a 5- to 8-membered heterocyclic group having 1 to 3 hetero atoms selected from a nitrogen atom, an oxygen atom and a sulfur atom, which is optionally substituted by C₁₋₄ alkyl; R^{2g} is
(i) a C₁₋₄ alkyl group optionally substituted by hydroxy,
(ii) a C₆₋₁₈ aryl group optionally substituted by substituent(s) selected from
   (a) nitro,
   (b) amino,
   (c) -CO-NR⁸-(CH₂)ₙ-O-C₁₋₄ alkyl,
   (d) -NR⁸-CO-(CH₂)ₙ-O-C₁₋₄ alkyl,
   (e) -NR⁸-CO-(CH₂)ₙ-NR⁶R⁷,
   (f) -NR⁸-CO-(CH₂)ₙ-COOH,
   (g) -NR⁸-CO-(CH₂)ₙ-CO₂-C₁₋₄ alkyl, and
   (h) -NR⁸-CO-(CH₂)ₘO-(CH₂)ₙ-O-C₁₋₄ alkyl,
      wherein m is an integer of 0 to 4, n is an integer of 1 to 4, R⁶ and R⁷ are the same or different and each is a hydrogen atom or a C₁₋₄ alkyl group, and R⁸ is a hydrogen atom or a C₁₋₄ alkyl group, or
(iii) a C₆₋₁₈ aryl-C₁₋₄ alkyl group optionally substituted by substituent(s) selected from
   (a) carboxy,
   (b) C₁₋₄ alkoxy-carbonyl, and
   (c) -CO-NR⁸-(CH₂)ₙ-O-C₁₋₄ alkyl,
      wherein n is an integer of 1 to 4, and R⁸ is a hydrogen atom or a C₁₋₄ alkyl group;
      R^{3g} is a hydrogen atom or a C₁₋₆ alkyl group; or
      R^{2g} and R^{3g} are optionally bonded to form C₂₋₄ alkylene, is preferable.

As compound (Ig), a compound wherein
R^{2g} is
(i) a C₆₋₁₈ aryl group optionally substituted by substituent(s) selected from
   (a) nitro,
   (b) amino,
   (c) -CO-NR⁸-(CH₂)ₙ-O-C₁₋₄ alkyl,
   (d) -NR⁸-CO-(CH₂)ₙ-O-C₁₋₄ alkyl,
   (e) -NR⁸-CO-(-CH₂)ₙ-NR⁶R⁷,
   (f) -NR⁸-CO-(CH₂)ₙ-COOH,
   (g) -NR⁸-CO-(CH₂)ₙ-CO₂-C₁₋₄ alkyl, and
   (h) -NR⁸-CO-(CH₂)ₘO-(CH₂)ₙ-O-C₁₋₄ alkyl,
      wherein m is an integer of 0 to 4, n is an integer of 1 to 4, R⁶ and R⁷ are the same or different and each is a hydrogen atom or a C₁₋₄ alkyl group, and R⁸ is a hydrogen atom or a C₁₋₄ alkyl group, or
(ii) a C₆₋₁₈ aryl-C₁₋₄ alkyl group substituted by substituent(s) selected from
   (a) carboxy,
   (b) C₁₋₄ alkoxy-carbonyl, and
   (c) -CO-NR⁸-(CH₂)ₙ-O-C₁₋₄ alkyl,
      wherein n is an integer of 1 to 4, and R⁸ is a hydrogen atom or a C₁₋₄ alkyl group, is preferable.
   As R⁸, a hydrogen atom, methyl, ethyl and the like are preferable, and a hydrogen atom is particularly preferable.

### [Compound (Ih)]

A compound (I) selected from the following (A) to (H). (A) A compound (I) wherein W is C(R¹);
A is a phenoxy-C₆₋₁₈ aryl group wherein the phenyloxy moiety is optionally substituted by 1 to 5 substituents selected from
(i) halogen,
(ii) optionally halogenated C₁₋₄ alkyl,
(iii) hydroxy-C₁₋₄ alkyl,
(iv) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
(v) optionally halogenated C₁₋₄ alkoxy,
(vi) C₁₋₄ alkyl-carbonyl,
(vii) cyano,
(viii) carbamoyl optionally substituted by C₁₋₈ alkyl, and
(ix) C₁₋₄ alkoxy-carbonyl, and the C₆₋₁₈ aryl moiety is optionally further substituted by 1 to 4 substituents selected from halogen, C₁₋₄ alkyl, hydroxy-C₁₋₄ alkyl, C₁₋₄ alkoxy, carboxy and C₁₋₄ alkoxy-carbonyl;
   X¹ is -NR^{3'}- wherein R^{3'} is a hydrogen atom or a C₁₋₆ alkyl group; R¹ is
   (i) a hydrogen atom,
   (ii) a cyano group, or
   (iii) a C₁₋₄ alkyl group or a C₂₋₄ alkenyl group, each of which is optionally substituted by -NR⁸-CO-(CH₂)ₙ-NR⁶R⁷
      wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and when n is not less than 2, a subset -CH₂CH₂- of (CH₂)ₙ is optionally replaced by - CH=CH-;
      R² is (i) a hydrogen atom or
   (ii) a C₁₋₈ alkyl group, a C₂₋₈ alkenyl group or a C₂₋₈ alkynyl group, each of which is optionally substituted by substituent(s) selected from
      (a) hydroxy,
      (b) carboxy,
      (c) cyano,
      (d) optionally halogenated C₁₋₄ alkoxy,
      (e) -O-(CH₂)ₙ-OH,
      (f) -O-(CH₂)ₙ-O-CO-NH₂,
      (g) -O-(CH₂)ₙ-O-(optionally halogenated C₁₋₄ alkyl),
      (h) -O-(CH₂)ₙ-SO₂- (optionally halogenated C₁₋₄ alkyl),
      (i) -O-(CH₂)ₙ-SO₂-C₆₋₁₈ aryl,
      (j) -O-(CH₂)ₙ-SO₂-(CH₂)ₙ-OH,
      (k) -O-(CH₂)ₙ-NR⁸-CO-C₁₋₄ alkyl,
      (l) -O-(CH₂)ₙ-NR⁸-CO-(CH₂)ₙ-SO₂-C₁₋₄ alkyl,
      (m) -O-(CH₂)ₙ-NR⁸-SO₂- (optionally halogenated C₁₋₄ alkyl),
      (n) -CO-NR⁸-(CH₂)ₙ-OH,
      (o) -CO-NR⁸-(CH₂)ₙ-SO₂-(optionally halogenated C₁₋₄ alkyl),
      (p) -CO-NR⁸-O-C₁₋₄ alkyl,
      (q) -NR⁶R⁷,
      (r) -NR⁸-(CH₂)ₙ-OH,
      (s) -NR₈-(CH₂)ₙ-SO₂-C₁₋₄ alkyl,
      (t) -NR⁸-CO-(optionally halogenated C₁₋₄ alkyl),
      (u) -NR⁸-CO- (CH₂)ₙ-OH,
      (v) -NR⁸-CO-(CH₂)ₙ-CN,
      (w) -NR⁸-CO-(CH₂)ₙ-NR⁶R⁷,
      (x) -NR⁸-CO-(CH₂)ₙ-O-C₁₋₄ alkyl,
      (y) -NR⁸-CO-(CH₂)ₙ-SO-(optionally halogenated C₁₋₄ alkyl),
      (z) -NR⁸-CO-(CH₂)ₙ-SO₂- (optionally halogenated C₁₋₄ alkyl),
      (aa) -NR⁸-CO-(CH₂)ₙ-SO₂-C₃₋₈ cycloalkyl,
      (bb) -NR⁸-CO-(CH₂)ₙ-NR⁸-SO₂-C₁₋₄ alkyl,
      (cc) -NR⁸-CO₂-(CH₂)ₙ-SO₂-C₁₋₄ alkyl,
      (dd) -NR⁸-CO-NH- (CH₂)ₙ-SO₂-C₁₋₄ alkyl,
      (ee) -NR⁸-CO-NH-O-C₁₋₄ alkyl,
      (ff) -NR⁸-CO-NH-(CH₂)ₙ-O-C₁₋₄ alkyl,
      (gg) -NR⁸-C(=NH)-NH-C₁₋₄ alkyl,
      (hh) -NR⁸-SO₂-(CH₂)-SO₂-C₁₋₄ alkyl,
      (ii) -S-(CH₂)ₙ-OH,
      (jj) -SO-(CH₂)ₙ-OH,
      (kk) -SO₂-(CH₂)ₙ-OH, and
      (ll) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
         wherein n is an integer of 1 to 4, R⁶ and R⁷ are the same or different and each is a hydrogen atom or a C₁₋₄ alkyl group, R⁸ is a hydrogen atom or a C₁₋₄ alkyl group, (CH₂)ₙ is optionally substituted by optionally halogenated C₁₋₄ alkyl or hydroxy, and when n is not less than 2, and a subset -CH₂CH₂- of (CH₂)ₙ is optionally replaced by -CH=CH-; or
         R¹ and R² are optionally bonded to form

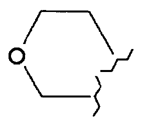

R² and R^{3'} are optionally bonded to form C₂₋₄ alkylene optionally substituted by an imino group,
particularly preferably, R^{2a} is a C₁₋₈ alkyl group, a C₂₋₈ alkenyl group or a C₂₋₈ alkynyl group (particularly, a C₁₋₈ alkyl group), each of which is optionally substituted by substituent(s) selected from
(a) hydroxy,
(b) carboxy,
(c) cyano,
(d) optionally halogenated C₁₋₄ alkoxy,
(e) -O-(CH₂)ₙ-OH (wherein (CH₂)ₙ is optionally substituted by hydroxy),
(f) -O-(CH₂)ₙ-O-CO-NH₂,
(g) -O-(CH₂)ₙ-O-(optionally halogenated C₁₋₄ alkyl),
(h) -O-(CH₂)ₙ-SO₂-(optionally halogenated C₁₋₄ alkyl),
(i) -O-(CH₂)ₙ-SO₂-C₆₋₁₈ aryl,
(j) -O-(CH₂)n-SO₂-(CH₂)ₙ-OH,
(k) -O-(CH₂)ₙ-NR⁸-CO-C₁₋₄ alkyl,
(l) -O-(CH₂)ₙ-NR⁸-CO-(CH₂)ₙ-SO₂-C₁₋₄ alkyl,
(m) -O-(CH₂)ₙ-NR⁸-SO₂- (optionally halogenated C₁₋₄ alkyl),
(n) -CO-NR⁸-(CH₂)ₙ-OH,
(o) -CO-NR⁸-(CH₂)ₙ-SO₂-(optionally halogenated C₁₋₄ alkyl),
(p) -CO-NR⁸-O-C₁₋₄ alkyl,
(q) -NR⁶R⁷,
(r) -NR⁸-(CH₂)ₙ-OH,
(s) -NR⁸-(CH₂)ₙ-SO₂-C₁₋₄ alkyl,
(t) -NR⁸-CO-(optionally halogenated C₁₋₄ alkyl),
(u) -NR⁸-CO-(CH₂)ₙ-OH (wherein (CH₂)ₙ is optionally substituted by optionally halogenated C₁₋₄ alkyl or hydroxy),
(v) -NR⁸-CO-(CH₂)ₙ-CN,
(w) -NR⁸-CO-(CH₂)ₙ-NR⁶R⁷ (when n is not less than 2, a subset -CH₂CH₂- of (CH₂)ₙ is optionally replaced by -CH=CH-),
(x) -NR⁸-CO-(CH₂)ₙ-O-C₁₋₄ alkyl,
(y) -NR⁸-CO-(CH₂)ₙ-SO-(optionally halogenated C₁₋₄ alkyl),
(z) -NR⁸-CO-(CH₂)ₙ-SO₂-(optionally halogenated C₁₋₄ alkyl)
   (wherein (CH₂)ₙ is optionally substituted by C₁₋₄ alkyl),
(aa) -NR⁸-CO-(CH₂)ₙ-SO₂-C₃₋₈ cycloalkyl,
(bb) -NR⁸-CO-(CH₂)ₙ-NR⁸-SO₂-C₁₋₄ alkyl,
(cc) -NR⁸-CO₂-(CH₂)ₙ-SO₂-C₁₋₄ alkyl,
(dd) -NR⁸-CO-NH-(CH2)ₙ-SO₂-C₁₋₄ alkyl,
(ee) -NR⁸-CO-NH-O-C₁₋₄ alkyl,
(ff) -NR⁸-CO-NH-(CH₂)ₙ-O-C₁₋₄ alkyl,
(gg) -NR⁸-C(=NH)-NH-C₁₋₄ alkyl,
(hh) -NR⁸-SO₂-(CH₂)ₙ-SO₂-C₁₋₄ alkyl,
(ii) -S-(CH₂₎ₙ-OH,
(jj) -SO-(CH₂)ₙ-OH,
(kk) -SO₂-(CH₂)ₙ-OH, and
(ll) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
wherein n is an integer of 1 to 4, R⁶ and R⁷ are the same or different and each is a hydrogen atom or a C₁₋₄ alkyl group, and R⁸ is a hydrogen atom or a C₁₋₄ alkyl group.

(B) A compound (I) wherein W is C(R¹);
   A is a phenyl-C₁₋₃ alkoxy-C₆₋₁₈ aryl group wherein the phenyl moiety is optionally substituted by 1 to 5 substituents selected from halogen, optionally halogenated C₁₋₄ alkyl and cyano, and
   the C₆₋₁₈ aryl moiety is optionally further substituted by 1 to 4 substituents selected from halogen, C₁₋₄ alkyl optionally having hydroxy and C₁₋₄ alkoxy;
   X¹ is -NR^{3'}- wherein R^{3'} is a hydrogen atom or a C₁₋₆ alkyl group;
   R¹ is (i) a hydrogen atom, or
   (ii) a C₁₋₄ alkyl group or a C₂₋₄ alkenyl group, each of which is optionally substituted by substituent(s) selected from
   (a) hydroxy,
   (b) amino, and
   (c) -NR⁸-CO-(CH₂)ₙ-NR⁶R⁷,
      wherein n is an integer of 1 to 4, R⁶ and R⁷ are the same or different and each is a hydrogen atom or a C₁₋₄ alkyl group, and R⁸ is a hydrogen atom or a C₁₋₄ alkyl group,
   (iii) a C₆₋₁₈ aryl group optionally substituted by substituent(s) selected from
   (a) amino,
   (b) carboxy,
   (c) -NR⁸-CO-(CH₂)ₙ-O-C₁₋₄ alkyl, and
   (d) -NR⁸-CO-(CH₂)ₙ-O-C₁₋₄ alkyl,
      wherein n is an integer of 1 to 4, and R⁸ is a hydrogen atom or a C₁₋₄ alkyl group, and when n is not less than 2, a subset -CH₂-CH₂- of (CH₂)ₙ is optionally replaced by -CH=CH-, or
   (iv) a 5- to 8-membered heterocyclic group having 1 to 3 hetero atoms selected from a nitrogen atom, an oxygen atom and a sulfur atom; R² is (i) a hydrogen atom,
   (ii) a C₁₋₈ alkyl group optionally substituted by substituent(s) selected from
   (a) halogen,
   (b) hydroxy,
   (c) C₁₋₄ alkoxy,
   (d) -O-(CH₂)ₙ-OH,
   (e) -O-(CH₂)ₙ-O-C₁₋₄ alkyl,
   (f) -CO-NR⁸-(CH₂)ₙ-OH,
   (g) -NR⁶R⁷, and
   (h) -NR⁸-(CH₂)ₙ-OH,
      wherein n is an integer of 1 to 4, R⁶ and R⁷ are the same or different and each is a hydrogen atom or a C₁₋₄ alkyl group, and R⁸ is a hydrogen atom or a C₁₋₄ alkyl group,
      (iii) a C₆₋₁₈ aryl-C₁₋₄ alkyl group optionally substituted by substituent(s) selected from
      (a) C₁₋₄ alkyl optionally having hydroxy,
      (b) carboxy,
      (c) C₁₋₄ alkoxy-carbonyl,
      (d) 5- to 8-membered heterocyclyl-carbonyl having 1 to 3 hetero atoms selected from a nitrogen atom, an oxygen atom and a sulfur atom, which optionally has substituent(s) selected from hydroxy and C₁₋₄ alkyl, and
      (e) C₁₋₄ alkyl-carbamoyl optionally having substituent(s) selected from hydroxy and carbamoyl,
         (iv) a C₆₋₁₈ aryl-carbonyl group optionally substituted by C₁₋₄ alkoxy,
         (v) a C₆₋₁₈ aryl-sulfonyl group optionally substituted by C₁₋₄ alkoxy, or
         (vi) a 5- to 8-membered heterocyclyl-C₁₋₄ alkyl group having 1 to 3 hetero atoms selected from a nitrogen atom, an oxygen atom and a sulfur atom, which is optionally substituted by substituent(s) selected from
         (a) carboxy, and
         (b) C₁₋₄ alkoxy-carbonyl; or
         R² and R^{3'} are optionally bonded to form C₂₋₄ alkylene.

(C) A compound (I) wherein W is C(R¹);
   A is a 5- to 8-membered heterocycleoxy-C₆₋₁₈ aryl group containing 1 to 3 hetero atoms selected from a nitrogen atom, an oxygen atom and a sulfur atom, wherein the heterocycleoxy moiety is optionally substituted by 1 to 5 substituents selected from
   (i) halogen,
   (ii) C₁₋₄ alkyl,
   (iii) C₁₋₄ alkyl-carbonyl,
   (iv) optionally halogenated C₁₋₄ alkoxy-carbonyl,
   (v) C₃₋₈ cycloalkyl-carbonyl, and
   (vi) a carbamoyl group optionally substituted by substituent(s) selected from
      (a) optionally halogenated C₁₋₈ alkyl,
      (b) C₃₋₈ cycloalkyl, and
      (c) C₆₋₁₈ aryl optionally substituted by substituent(s) selected from halogen, C₁₋₄ alkyl and C₁₋₄ alkoxy, and
         the C₆₋₁₈ aryl moiety is optionally further substituted by 1 to 4 substituents selected from halogen and optionally halogenated C₁₋₄ alkyl;
         X¹ is -NR^{3'}- wherein R^{3'} is a hydrogen atom or a C₁₋₆ alkyl group; R¹ is (i) a hydrogen atom,
         (ii) a C₁₋₄ alkyl group or a C₂₋₄ alkenyl group, each of which is optionally substituted by substituent(s) selected from
            (a) hydroxy,
            (b) amino,
            (c) -NR⁸-CO-(CH₂)ₙ-NR⁶R⁷, and
            (d) -NR⁸-CO-(CH₂)ₙ-O-C₁₋₄ alkyl,
               wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and when n is not less than 2, a subset -CH₂CH₂- of (CH₂)ₙ is optionally replaced by -CH=CH-,
         (iii) a C₆₋₁₈ aryl group optionally substituted by substituent(s) selected from
            (a) C₁₋₄ alkyl optionally substituted by substituent(s) selected from hydroxy, -NR⁸-(CH₂)n-SO₂-C₁₋₄ alkyl and -NR⁸-CO-(CH₂)ₙ-O-C₁₋₄ alkyl,
            (b) amino,
            (c) C₁₋₄ alkoxy,
            (d) carboxy, and
            (e) -NR⁸-CO-(CH₂)ₙ-O-C₁₋₄ alkyl,
               wherein n is an integer of 1 to 4, and R⁸ is a hydrogen atom or a C₁₋₄ alkyl group, or
         (iv) a 5- to 8-membered heterocyclic group having 1 to 3 hetero atoms selected from a nitrogen atom, an oxygen atom and a sulfur atom;
            R² is (i) a hydrogen atom,
         (ii) a C₁₋₄ alkyl group optionally substituted by substituent(s) selected from
            (a) halogen,
            (b) hydroxy,
            (c) C₁₋₄ alkoxy,
            (d) carboxy,
            (e) C₁₋₄ alkoxy-carbonyl,
            (f) -O-(CH₂)ₙ-OH,
            (g) -O-(CH₂)ₙ-O-C₁₋₄ alkyl,
            (h) -CO-NR⁸-(CH₂)ₙ-OH, and
            (i) -NR⁸-CO-(CH₂)ₙ-SO₂-C₁₋₄ alkyl
               wherein n is an integer of 1 to 4, and R⁸ is a hydrogen atom or a C₁₋₄ alkyl group, or
            (iii) a C₆₋₁₈ aryl-C₁₋₄ alkyl group optionally substituted by C₁₋₄ alkyl optionally having hydroxy; or
               R² and R^{3'} are optionally bonded to form C₂₋₄ alkylene.

(D) A compound (I) wherein W is C(R¹);
   A is 5- to 8-membered heterocyclyl-C₁₋₃ alkoxy-C₆₋₁₈ aryl group containing 1 to 3 hetero atoms selected from a nitrogen atom, an oxygen atom and a sulfur atom;
   wherein the C₆₋₁₈ aryl moiety is optionally further substituted by halogen;
   X¹ is -NR^{3'}- wherein R^{3'} is a hydrogen atom or a C₁₋₆ alkyl group;
   R¹ is (i) a hydrogen atom or
   (ii) a 5- to 8-membered heterocyclic group having 1 to 3 hetero atoms selected from a nitrogen atom, an oxygen atom and a sulfur atom;
      R² is (i) a hydrogen atom,
   (ii) C₁₋₄ alkyl optionally substituted by substituent(s) selected from
      (a) C₁₋₄ alkoxy,
      (b) -O-(CH₂)ₙ-OH, and
      (c) -NR⁸-CO-(CH₂)ₙ-SO₂-C₁₋₄ alkyl,
         wherein n is an integer of 1 to 4, and R⁸ is a hydrogen atom or a C₁₋₄ alkyl group, or
   (iii) a 5- to 8-membered heterocyclyl-C₁₋₄ alkyl group having 1 to 3 hetero atoms selected from a nitrogen atom, an oxygen atom and a sulfur atom, which is optionally substituted by substituent(s) selected from
      (a) carboxy, and
      (b) C₁₋₄ alkoxy-carbonyl.

(E) A compound (I) wherein W is N;
   A is a phenoxy-C₆₋₁₈ aryl group wherein the phenyloxy moiety is optionally substituted by 1 to 5 substituents selected from optionally halogenated C₁₋₄ alkyl and cyano, and
   the C₆₋₁₈ aryl moiety is optionally further substituted by 1 to 4 substituents selected from halogen and C₁₋₄ alkyl;
   X¹ is -NR^{3'}- wherein R^{3'} is a hydrogen atom or a C₁₋₆ alkyl group;
   R² is (i) a hydrogen atom or
   (ii) a C₁₋₄ alkyl group optionally substituted by -O-(CH₂)ₙ-OH wherein n is an integer of 1 to 4.

(F) A compound (I) wherein W is N;
   A is a phenyl-C₁₋₃ alkoxy-C₆₋₁₈ aryl group wherein the phenyl moiety is optionally substituted by 1 to 5 substituents selected from halogen and cyano, and
   the C₆₋₁₈ aryl moiety is optionally further substituted by 1 to 5 substituents selected from halogen and C₁₋₄ alkyl;
   X¹ is -NR^{3'}- wherein R^{3'} is a hydrogen atom or a C₁₋₆ alkyl group;
   R² is (i) a hydrogen atom,
   (ii) a C₁₋₄ alkyl group optionally substituted by 1 to 5 substituents selected from the group consisting of
      (a) hydroxy,
      (b) -O-(CH₂)ₙ-OH,
      (c) -NR⁸-(CH₂)ₙ-O-C₁₋₄ alkyl,
      (d) -NR⁸-(CH₂)ₙ-heterocyclic group (preferably, said heterocyclic group is a 5- to 8-membered heterocyclic group having 1 to 3 hetero atoms selected from a nitrogen atom, an oxygen atom and a sulfur atom), and
      (e) -NR⁸-(CH₂)ₙ-SO₂-C₁₋₄ alkyl,
         wherein n is an integer of 1 to 4, and R⁸ is a hydrogen atom or a C₁₋₄ alkyl group,
   (iii) a C₆₋₁₈ aryl group optionally substituted by C₁₋₄ alkyl optionally substituted by substituent(s) selected from hydroxy, -NR⁸-(CH₂)ₙ-OH, -NR⁸-(CH₂)ₙ-heterocyclic group (preferably, said heterocyclic group is a 5- to 8-membered heterocyclic group having 1 to 3 hetero atoms selected from a nitrogen atom, an oxygen atom and a sulfur atom) and -NR⁸-(CH₂)ₙ-SO₂-C₁₋₄ alkyl, wherein n is an integer of 1 to 4 and R⁸ is a hydrogen atom or a C₁₋₄ alkyl group, or
   (iv) a C₆₋₁₈ aryl-C₁₋₄ alkyl group optionally substituted by 1 to 5 substituents selected from the group consisting of
      (a) carboxy,
      (b) C₁₋₄ alkoxy-carbonyl, and
      (c) -CO-NR⁸-(CH₂)ₙ-O-C₁₋₄ alkyl,
         wherein n is an integer of 1 to 4, and R⁸ is a hydrogen atom or a C₁₋₄ alkyl group; or
         R² and R^{3'} are optionally bonded to form C₂₋₄ alkylene.

(G) A compound (I) wherein W is N;
   A is a 5- to 8-membered heterocycleoxy-C₆₋₁₈ aryl group containing 1 to 3 hetero atoms selected from a nitrogen atom, an oxygen atom and a sulfur atom, wherein the heterocycleoxy moiety is optionally substituted by C₁₋₄ alkyl, and
   the C₆₋₁₈ aryl moiety is optionally further substituted by C₁₋₄ alkyl;
   X¹ is -NR^{3'}- wherein R^{3'} is a hydrogen atom or a C₁₋₆ alkyl group;
   R² is (i) a hydrogen atom,
   (ii) a C₁₋₄ alkyl group optionally substituted by hydroxy,
   (iii) a C₆₋₁₈ aryl group optionally substituted by substituent(s) selected from
      (a) nitro,
      (b) amino,
      (c) -CO-NR⁸-(CH₂)ₙ-O-C₁₋₄ alkyl,
      (d) -NR⁸-CO-(CH₂)ₙ-O-C₁₋₄ alkyl,
      (e) -NR⁸-CO-(CH₂)ₙ-N⁶R⁷,
      (f) -NR⁸-CO-(CH₂)ₙ-COOH,
      (g) -NR⁸-CO-(CH₂)ₙ-CO₂-C₁₋₄ alkyl, and
      (h) -NR⁸-CO-(CH₂)ₘ-O-(CH₂)ₙ-O-C₁₋₄ alkyl,
         wherein m is an integer of 0 to 4, n is an integer of 1 to 4, R⁶ and R⁷ are the same or different and each is a hydrogen atom or a C₁₋₄ alkyl group, and R⁸ is a hydrogen atom or a C₁₋₄ alkyl group, or
   (iv) a C₆₋₁₈ aryl-C₁₋₄ alkyl group optionally substituted by substituent(s) selected from
      (a) carboxy,
      (b) C₁₋₄ alkoxy-carbonyl, and
      (c) -CO-NR⁸-(CH₂)ₙ-O-C₁₋₄ alkyl,
         wherein n is an integer of 1 to 4, and R⁸ is a hydrogen atom or a C₁₋₄ alkyl group; and
         R² and R^{3'} are optionally bonded to form C₂₋₄ alkylene.

(H) A compound (I) wherein W is CH; A is a C₆₋₁₈ aryl group optionally substituted by substituent(s) selected from
   (a) carboxy,
   (b) C₁₋₄ alkoxy-carbonyl,
   (c) a 5- to 8-membered heterocyclyl-carbonyl group containing 1 to 3 hetero atoms selected from a nitrogen atom, an oxygen atom and a sulfur atom (preferably, a 5- to 8-membered cyclic amino-carbonyl group optionally having 1 or 2 hetero atoms selected from a nitrogen atom, an oxygen atom and a sulfur atom), which is optionally substituted by C₆₋₁₈ aryl-C₁₋₄ alkyl,
   (d) a carbamoyl group optionally substituted by C₆₋₁₈ aryl-C₁₋₄ alkyl, and
   (e) a ureido group optionally substituted by C₆₋₁₈ aryl-C₁₋₄ alkyl;
      X¹ is -NR^{3'}- wherein R^{3'} is a hydrogen atom or a C₁₋₆ alkyl group; and
      R² is a hydrogen atom.

### [Compound (Ii)]

A compound (I) wherein A is a C₆₋₁₈ aryl group substituted by substituent(s) selected from
(i) a phenoxy group substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (e) optionally halogenated C₁₋₄ alkoxy,
   (f) C₁₋₄ alkyl-carbonyl,
   (g) cyano,
   (h) carbamoyl optionally substituted by C₁₋₈ alkyl, and
   (i) C₁₋₄ alkoxy-carbonyl,
(ii) a phenyl-C₁₋₃ alkoxy group substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (e) optionally halogenated C₁₋₄ alkoxy,
   (f) C₁₋₄ alkyl-carbonyl,
   (g) cyano,
   (h) carbamoyl optionally substituted by C₁₋₈ alkyl, and
   (i) C₁₋₄ alkoxy-carbonyl,
(iii) a 5- to 8-membered heterocycleoxy group containing 1 to 3 hetero atoms selected from a nitrogen atom, an oxygen atom and a sulfur atom, which is substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (e) optionally halogenated C₁₋₄ alkoxy,
   (f) C₁₋₄ alkyl-carbonyl,
   (g) cyano,
   (h) carbamoyl optionally substituted by C₁₋₈ alkyl, and
   (i) C₁₋₄ alkoxy-carbonyl, and
(iv) 5- to 8-membered heterocyclyl-C₁₋₃ alkoxy containing 1 to 3 hetero atoms selected from a nitrogen atom, an oxygen atom and a sulfur atom, which is substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (e) optionally halogenated C₁₋₄ alkoxy,
   (f) C₁₋₄ alkyl-carbonyl,
   (g) cyano,
   (h) carbamoyl optionally substituted by C₁₋₈ alkyl, and
   (i) C₁₋₄ alkoxy-carbonyl;
   wherein the C₆₋₁₈ aryl group is optionally further substituted by 1 to 4 substituents selected from halogen and optionally halogenated C₁₋₄ alkyl;
   R¹ is a hydrogen atom;
   R² is a C₁₋₈ alkyl group, a C₂₋₈ alkenyl group or a C₂₋₈ alkynyl group, each of which is substituted by substituent(s) selected from
   (a) hydroxy,
   (b) carboxy,
   (c) cyano,
   (d) optionally halogenated C₁₋₄ alkoxy,
   (e) -O-(CH₂)ₙ-OH,
   (f) -O-(CH₂)ₙ-O-CO-NH₂,
   (g) -O-(CH₂)ₙ-O-(optionally halogenated C₁₋₄ alkyl),
   (h) -O-(CH₂)ₙ-SO₂- (optionally halogenated C₁₋₄ alkyl),
   (i) -O-(CH₂)ₙ-SO₂-C₆₋₁₈ aryl,
   (j) -O-(CH₂)ₙ-SO₂-(CH₂)ₙ-OH,
   (k) -O-(CH₂)ₙ-NR⁸-CO-C₁₋₄ alkyl,
   (l) -O-(CH₂)ₙ-NR⁸-CO-(CH₂)ₙ-SO₂-C₁₋₄ alkyl,
   (m) -O-(CH₂)ₙ-NR⁸-SO₂- (optionally halogenated C₁₋₄ alkyl),
   (n) -CO-NR⁸-(CH₂)ₙ-OH,
   (o) -CO-NR⁸-(CH₂)ₙ-SO₂- (optionally halogenated C₁₋₄ alkyl),
   (p) -CO-NR⁸-O-C₁₋₄ alkyl,
   (q) -NR⁶R⁷,
   (r) -NR⁸-(CH₂)ₙ-OH,
   (s) -NR⁸-(CH₂)ₙ-SO₂-C₁₋₄ alkyl,
   (t) -NR⁸-CO-(optionally halogenated C₁₋₄ alkyl),
   (u) -NR⁸-CO- (CH₂)ₙ-OH,
   (v) -NR⁸-CO-(CH₂)ₙ-CN,
   (w) -NR⁸-CO-(CH₂)ₙ-NR⁶R⁷,
   (x) -NR⁸-CO-(CH₂)ₙ-O-C₁₋₄ alkyl,
   (y) -NR⁸-CO-(CH₂)ₙ-SO-(optionally halogenated C₁₋₄ alkyl),
   (z) -NR⁸-CO-(CH₂)ₙ-SO₂-(optionally halogenated C₁₋₄ alkyl),
   (aa) -NR⁸-CO-(CH₂)ₙ-SO₂-C₃₋₈ cycloalkyl,
   (bb) -NR⁸-CO-(CH₂)ₙ-NR⁸-SO₂-C₁₋₄ alkyl,
   (cc) -NR⁸-CO₂-(CH₂)ₙ-SO₂-C₁₋₄ alkyl,
   (dd) -NR⁸-CO-NH-(CH₂)ₙ-SO₂-C₁₋₄ alkyl,
   (ee) -NR⁸-CO-NH-O-C₁₋₄ alkyl,
   (ff) -NR⁸-CO-NH-(CH₂)ₙ-O-C₁₋₄ alkyl,
   (gg) -NR⁸-C(=NH) -NH-C₁₋₄ alkyl,
   (hh) -NR⁸-SO₂-(CH₂)ₙ-SO₂-C₁₋₄ alkyl,
   (ii) -S-(CH₂)ₙ-OH,
   (jj) -SO-(CH₂)ₙ-OH,
   (kk) -SO₂-(CH₂)ₙ-OH, and
   (ll) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
      wherein n is an integer of 1 to 4, R⁶ and R⁷ are the same or different and each is a hydrogen atom or a C₁₋₄ alkyl group, R⁸ is a hydrogen atom or a C₁₋₄ alkyl group, (CH₂)ₙ is optionally substituted by optionally halogenated C₁₋₄ alkyl or hydroxy, and when n is not less than 2, a subset -CH₂CH₂- of (CH₂)ₙ is optionally replaced by -CH=CH-;
      R³ is a hydrogen atom or a C₁₋₆ alkyl group; or
      R¹ and R² are optionally bonded to form

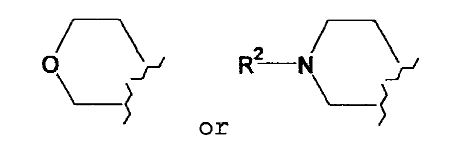

R² and R³ are optionally bonded to form C₂₋₄ alkylene optionally substituted by an imino group.

Particularly preferably, R² is a C₁₋₈ alkyl group, a C₂₋₈ alkenyl group or a C₂₋₈ alkynyl group (particularly, a C₁₋₈ alkyl group), each of which is optionally substituted by substituent(s) selected from
(a) hydroxy,
(b) carboxy,
(c) cyano,
(d) optionally halogenated C₁₋₄ alkoxy,
(e) -O-(CH₂)ₙ-OH (wherein (CH₂)ₙ is optionally substituted by hydroxy),
(f) -O-(CH₂)ₙ-O-CO-NH₂,
(g) -O-(CH₂)ₙ-O-(optionally halogenated C₁₋₄ alkyl),
(h) -O-(CH₂)ₙ-SO₂-(optionally halogenated C₁₋₄ alkyl),
(i) -O-(CH₂)ₙ-SO₂-C₆₋₁₈ aryl,
(j) -O-(CH₂)ₙ-SO₂-(CH₂)ₙ-OH,
(k) -O-(CH₂)ₙ-NR⁸-CO-C₁₋₄ alkyl,
(l) -O-(CH₂)ₙ-NR⁸-CO-(CH₂)ₙ-SO₂-C₁₋₄ alkyl,
(m) -O-(CH₂)ₙ-NR⁸-SO₂-(optionally halogenated C₁₋₄ alkyl),
(n) -CO-NR⁸-(CH₂)ₙ-OH,
(o) -CO-NR⁸-(CH₂)n-SO₂- (optionally halogenated C₁₋₄ alkyl),
(p) -CO-NR⁸-O-C₁₋₄ alkyl,
(q) -NR⁶R⁷,
(r) -NR⁸-(CH₂)ₙ-OH,
(s) -NR⁸-(CH₂)ₙ-SO₂-C₁₋₄ alkyl,
(t) -NR⁸-CO-(optionally halogenated C₁₋₄ alkyl),
(u) -NR⁸-CO-(CH₂)ₙ-OH (wherein (CH₂)ₙ is optionally substituted by optionally halogenated C₁₋₄ alkyl or hydroxy),
(v) -NR⁸-CO-(CH₂)ₙ-CN,
(w) -NR⁸-CO-(CH₂)ₙ-NR⁶R⁷ (when n is not less than 2, a subset -CH₂CH₂- of (CH₂)ₙ is optionally replaced by -CH=CH-),
(x) -NR⁸-CO-(CH₂)ₙ-O-C₁₋₄ alkyl,
(y) -NR⁸-CO-(CH₂)ₙ-SO- (optionally halogenated C₁₋₄ alkyl),
(z) -NR⁸-CO-(CH₂)ₙ-SO₂-(optionally halogenated C₁₋₄ alkyl)
   (wherein (CH₂)ₙ is optionally substituted by C₁₋₄ alkyl),
(aa) -NR⁸-CO-(CH₂)ₙ-SO₂-C₃₋₈ cycloalkyl,
(bb) -NR⁸-CO-(CH₂)ₙ-NR⁸-SO₂-C₁₋₄ alkyl,
(cc) -NR⁸-CO₂-(CH₂)ₙ-SO₂-C₁₋₄ alkyl,
(dd) -NR⁸-CO-NH-(CH₂)ₙ-SO₂-C₁₋₄ alkyl,
(ee) -NR⁸-CO-NH-O-C₁₋₄ alkyl,
(ff) -NR⁸-CO-NH-(CH₂)ₙ-O-C₁₋₄ alkyl,
(gg) -NR⁸-C(=NH)-NH-C₁₋₄ alkyl,
(hh) -NR⁸-SO₂-(CH₂)ₙ-SO₂-C₁₋₄ alkyl,
(ii) -S- (CH₂)ₙ-OH,
(jj) -SO-(CH₂)ₙ-OH,
(kk) -SO₂-(CH₂)ₙ-OH, and
(ll) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, and R⁸ is a hydrogen atom or a C₁₋₄ alkyl group.

### [Compound (Ij)]

A compound (I) wherein
A is a C₆₋₁₈ aryl group substituted by substituent(s) selected from
(i) a phenoxy group substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl and the like),
   (e) optionally halogenated C₁₋₄ alkoxy,
   (f) cyano,
   (g) carbamoyl optionally substituted by C₁₋₈ alkyl, and
   (h) C₁₋₄ alkoxy-carbonyl,
(ii) a phenyl-C₁₋₃ alkoxy group substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl and the like),
   (e) optionally halogenated C₁₋₄ alkoxy,
   (f) cyano,
   (g) carbamoyl optionally substituted by C₁₋₈ alkyl, and
   (h) C₁₋₄ alkoxy-carbonyl,
(iii) a 5- to 8-membered heterocycleoxy group containing 1 to 3 hetero atoms selected from a nitrogen atom, an oxygen atom and a sulfur atom, which is substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl and the like),
   (e) optionally halogenated C₁₋₄ alkoxy,
   (f) cyano,
   (g) carbamoyl optionally substituted by C₁₋₈ alkyl, and
   (h) C₁₋₄ alkoxy-carbonyl, and
(iv) 5- to 8-membered heterocyclyl-C₁₋₃ alkoxy containing 1 to 3 hetero atoms selected from a nitrogen atom, an oxygen atom and a sulfur atom, which is substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl having 1 to 3 hetero atoms selected from a nitrogen atom, an oxygen atom and an optionally oxidized sulfur atom, such as imidazolyl and the like),
   (e) optionally halogenated C₁₋₄ alkoxy,
   (f) cyano,
   (g) carbamoyl optionally substituted by C₁₋₈ alkyl, and
   (h) C₁₋₄ alkoxy-carbonyl;
      wherein the C₆₋₁₈ aryl group is optionally further substituted by 1 to 4 substituents selected from halogen and optionally halogenated C₁₋₄ alkyl;
      R¹ is a hydrogen atom;
      R² is a C₁₋₈ alkyl group, a C₂₋₈ alkenyl group or a C₂₋₈ alkynyl group, each of which is substituted by substituent(s) selected from
   (a) hydroxy,
   (b) optionally halogenated C₁₋₄ alkoxy,
   (c) -O-(CH₂)ₙ-OH,
   (d) -O-(CH₂)ₙ-O-CO-NH₂,
   (e) -O-(CH₂)ₙ-O-C₁₋₄ alkyl,
   (f) -O-(CH₂)ₙ-SO₂-(optionally halogenated C₁₋₄ alkyl),
   (g) -O-(CH₂)ₙ-SO₂-C₆₋₁₈ aryl,
   (h) -O-(CH₂)ₙ-SO₂-(CH₂)ₙ-OH,
   (i) -O-(CH₂)ₙ-NR⁸-SO₂-(optionally halogenated C₁₋₄ alkyl),
   (j) -CO-NR⁸-(CH₂)ₙ-OH,
   (k) -CO-NR⁸-(CH₂)ₙ-SO₂-(optionally halogenated C₁₋₄ alkyl),
   (l) -NR⁶R⁷,
   (m) -NR⁸-(CH₂)ₙ-OH,
   (n) -NR⁸-(CH₂)ₙ-SO₂-C₁₋₄ alkyl,
   (o) -NR⁸-CO-(CH₂)ₙ-OH,
   (p) -NR⁸-CO-(CH₂)ₙ-O-C₁₋₄ alkyl,
   (q) -NR⁸-CO-(CH₂)ₙ-SO-(optionally halogenated C₁₋₄ alkyl),
   (r) -NR⁸-CO-(CH₂)ₙ-SO₂-(optionally halogenated C₁₋₄ alkyl),
   (s) -NR⁸-CO-(CH₂)ₙ-SO₂-C₃₋₈ cycloalkyl,
   (t) -NR⁸-CO₂-(CH₂)ₙ-SO₂-C₁₋₄ alkyl,
   (u) -NR⁸-CO-NH-(CH₂)ₙ-SO₂-C₁₋₄ alkyl,
   (v) -NR⁸-SO₂-(CH₂)ₙ-SO₂-C₁₋₄ alkyl,
   (w) -S-(CH₂)ₙ-OH,
   (x) -SO-(CH₂)ₙ-OH,
   (y) -SO₂-(CH₂)ₙ-OH, and
   (z) -NR⁸-CO-(optionally substituted heterocyclic group)
      (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
      wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and (CH₂)ₙ is optionally substituted by C₁₋₄ alkyl or hydroxy;
      R³ is a hydrogen atom or a C₁₋₆ alkyl group; or
      R¹ and R² are optionally bonded to form

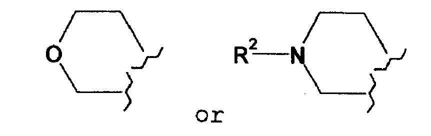

R² and R³ are optionally bonded to form C₂₋₄ alkylene.

Particularly preferably, R² is a C₁₋₈ alkyl group, a C₂₋₈ alkenyl group or a C₂₋₈ alkynyl group (particularly, a C₁₋₈ alkyl group), each of which is substituted by substituent(s) selected from
(a) hydroxy,
(b) optionally halogenated C₁₋₄ alkoxy,
(c) -O-(CH₂)ₙ-OH (wherein (CH₂)ₙ is optionally substituted by hydroxy),
(d) -O-(CH₂)ₙ-O-CO-NH₂,
(e) -O-(CH₂)ₙ-O-C₁₋₄ alkyl,
(f) -O-(CH₂)ₙ-SO₂-(optionally halogenated C₁₋₄ alkyl),
(g) -O-(CH₂)ₙ-SO₂-C₆₋₁₈ aryl,
(h) -O-(CH₂)ₙ-SO₂-(CH₂)ₙ-OH,
(i) -O-(CH₂)ₙ-NR⁸-SO₂-(optionally halogenated C₁₋₄ alkyl),
(j) -CO-NR⁸-(CH₂)ₙ-OH,
(k) -CO-NR⁸-(CH₂)ₙ-SO₂-(optionally halogenated C₁₋₄ alkyl),
(l) -NR⁶R⁷,
(m) -NR⁸-(CH₂)ₙ-OH,
(n) -NR⁸-(CH₂)ₙ-SO₂-C₁₋₄ alkyl,
(o) -NR⁸-CO-(CH₂)ₙ-OH (wherein (CH₂)ₙ is optionally substituted by C₁₋₄ alkyl),
(p) -NR⁸-CO-(CH₂)ₙ-O-C₁₋₄ alkyl,
(q) -NR⁸-CO-(CH₂)ₙ-SO-(optionally halogenated C₁₋₄ alkyl),
(r) -NR⁸-CO-(CH₂)ₙ-SO₂-(optionally halogenated C₁₋₄ alkyl) (wherein (CH₂)ₙ is optionally substituted by C₁₋₄ alkyl),
(s) -NR⁸-CO-(CH₂)ₙ-SO₂-C₃₋₈ cycloalkyl,
(t) -NR⁸-CO₂-(CH₂)ₙ-SO₂-C₁₋₄ alkyl,
(u) -NR⁸-CO-NH-(CH₂)ₙ-SO₂-C₁₋₄ alkyl,
(v) -NR⁸-SO₂-(CH₂)ₙ-SO₂-C₁₋₄ alkyl,
(w) -S-(CH₂)ₙ-OH,
(x) -SO-(CH₂)ₙ-OH,
(y) -SO₂-(CH₂)ₙ-OH, and
(z) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, and R⁸ is a hydrogen atom or a C₁₋₄ alkyl group, and the like is preferable.

### [Compound (Ik)]

A compound (I) wherein
R² is (i) a C₅₋₈ alkyl group substituted by hydroxy,
(ii) a C₁₋₈ alkyl group substituted by substituent(s) selected from
   (a) halogenated C₁₋₄ alkoxy,
   (b) -O-(CH₂)ₙ-OH,
   (c) -O-(CH₂)ₙ-O-CO-NH₂,
   (d) -O-(CH₂)ₙ-O-(optionally halogenated C₁₋₄ alkyl),
   (e) -O-(CH₂)ₙ-SO₂-(optionally halogenated C₁₋₄ alkyl),
   (f) -O-(CH₂)ₙ-SO₂-C₆₋₁₈ aryl,
   (g) -O-(CH₂)ₙ-NR⁸-SO₂-(optionally halogenated C₁₋₄ alkyl),
   (h) -CO-NR⁸-(CH₂)ₙ-OH,
   (i) -CO-NR⁸-(CH₂)ₙ-SO₂-(optionally halogenated C₁₋₄ alkyl),
   (j) -NR⁸-(CH₂)ₙ-SO₂-C₁₋₄ alkyl,
   (k) -NR⁸-CO-(CH₂)ₙ-OH,
   (l) -NR⁸-CO-(CH₂)ₙ-O-C₁₋₄ alkyl,
   (m) -NR⁸-CO-(CH₂)ₙ-SO-(optionally halogenated C₁₋₄ alkyl),
   (n) -NR⁸-CO-(CH₂)ₙ-SO₂-(optionally halogenated C₁₋₄ alkyl),
   (o) -NR⁸-CO-(CH₂)ₙ-SO₂-C₃₋₈ cycloalkyl,
   (p) -NR⁸-CO₂-(CH₂)ₙ-SO₂-C₁₋₄ alkyl,
   (q) -NR⁸-CO-NH-(CH₂)ₙ-SO₂-C₁₋₄ alkyl,
   (r) -NR⁸-SO₂-(CH₂)ₙ-SO₂-C₁₋₄ alkyl,
   (s) -S-(CH₂)ₙ-OH,
   (t) -SO-(CH₂)ₙ-OH,
   (u) -SO₂-(CH₂)ₙ-OH, and
   (v) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
      wherein n is an integer of 1 to 4, R⁸ is a hydrogen atom or a C₁₋₄ alkyl group, and (CH₂)ₙ is optionally substituted by C₁₋₄ alkyl,
(iii) a C₂₋₈ alkenyl group optionally substituted by hydroxy, or
(iv) a C₂₋₈ alkynyl group optionally substituted by hydroxy.
Particularly preferably, R² is (i) a C₅₋₈ alkyl group substituted by hydroxy,
(ii) a C₁₋₈ alkyl group substituted by substituent(s) selected from
   (a) halogenated C₁₋₄ alkoxy,
   (b) -O-(CH₂)ₙ-OH (wherein (CH₂)ₙ is optionally substituted by hydroxy),
   (c) -O-(CH₂)ₙ-O-CO-NH₂,
   (d) -O-(CH₂)ₙ-O-(optionally halogenated C₁₋₄ alkyl),
   (e) -O-(CH₂)ₙ-SO₂-(optionally halogenated C₁₋₄ alkyl),
   (f) -O-(CH₂)ₙ-SO₂-C₆₋₁₈ aryl,
   (g) -O-(CH₂)ₙ-NR⁸-SO₂-(optionally halogenated C₁₋₄ alkyl),
   (h) -CO-NR⁸-(CH₂)ₙ-OH,
   (i) -CO-NR⁸-(CH₂)ₙ-SO₂-(optionally halogenated C₁₋₄ alkyl),
   (j) -NR⁸-(CH₂)ₙ-SO₂-C₁₋₄ alkyl,
   (k) -NR⁸-CO-(CH₂)ₙ-OH (wherein (CH₂)ₙ is optionally substituted by C₁₋₄ alkyl),
   (l) -NR⁸-CO-(CH₂)ₙ-O-C₁₋₄ alkyl,
   (m) -NR⁸-CO-(CH₂)ₙ-SO-(optionally halogenated C₁₋₄ alkyl),
   (n) -NR⁸-CO-(CH₂)ₙ-SO₂-(optionally halogenated C₁₋₄ alkyl) (wherein (CH₂)ₙ is optionally substituted by C₁₋₄ alkyl),
   (o) -NR⁸-CO-(CH₂)ₙ-SO₂-C₃₋₈ cycloalkyl,
   (p) -NR⁸-CO₂-(CH₂)ₙ-SO₂-C₁₋₄ alkyl,
   (q) -NR⁸-CO-NH-(CH₂)ₙ-SO₂-C₁₋₄ alkyl,
   (r) -NR⁸-SO₂-(CH₂)ₙ-SO₂-C₁₋₄ alkyl,
   (s) -S-(CH₂)ₙ-OH,
   (t) -SO-(CH₂)ₙ-OH,
   (u) -SO₂-(CH₂)ₙ-OH, and
   (v) -NR⁸-CO-(optionally substituted heterocyclic group)
      (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
      wherein n is an integer of 1 to 4, and R⁸ is a hydrogen atom or a C₁₋₄ alkyl group,
(iii) a C₂₋₈ alkenyl group optionally substituted by hydroxy, or
(iv) a C₂₋₈ alkynyl group optionally substituted by hydroxy.

As the salts of the compound (I), for example, metal salt, ammonium salt, salts with organic base, salts with inorganic acid, salts with organic acid, salts with basic or acidic amino acid and the like can be mentioned. As preferable examples of the metal salt, for example, alkali metal salts such as sodium salt, potassium salt and the like; alkaline earth metal salts such as calcium salt, magnesium salt, barium salt and the like; aluminum salt and the like can be mentioned. As preferable examples of the salts with organic base, for example, salts with trimethylamine, triethylamine, pyridine, picoline, 2,6-lutidine, ethanolamine, diethanolamine, triethanolamine, tromethamine [tris(hydroxymethyl)methylamine], t-butylamine, cyclohexylamine, dicyclohexylamine, N,N'-dibenzylethylenediamine and the like can be mentioned. As preferable examples of salts with inorganic acid, for example, salts with hydrochloric acid, hydrobromic acid, nitric acid, sulfuric acid, phosphoric acid and the like can be mentioned. As preferable examples of the salts with organic acid, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like can be mentioned. As preferable examples of the salts with basic amino acid, for example, salts with arginine, lysine, ornithine and the like can be mentioned, and as preferable examples of the salts with acidic amino acid, for example, salts with aspartic acid, glutamic acid and the like can be mentioned.
Of these, pharmaceutically acceptable salts are preferable. For example, when a compound contains an acidic functional group, inorganic salts such as alkali metal salts (e.g., sodium salt, potassium salt etc.), alkaline earth metal salts (e.g., calcium salt, magnesium salt, barium salt etc.) and the like, ammonium salt and the like, and when a compound contains a basic functional group, for example, salts with inorganic acid such as hydrochloric acid, hydrobromic acid, nitric acid, sulfuric acid, phosphoric acid and the like, or salts with organic acid such as acetic acid, phthalic acid, fumaric acid, oxalic acid, tartaric acid, maleic acid, citric acid, succinic acid, methanesulfonic acid, p-toluenesulfonic acid and the like can be mentioned. As compound (I), preferred is a compound wherein A is an aryl group substituted by a group of the formula -Y²-B and optionally further substituted, wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted.
As a preferable embodiment of compound (I), a compound wherein W is C(R¹);
A is an aryl group substituted by a group of the formula -Y²-B, and optionally further substituted, wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted; R¹ is a hydrogen atom or a group of the formula -X²-R⁴ wherein X² is a single bond, -NH- or -O-, and R⁴ is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted;
R² is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted; and
X¹ is -NR³- wherein R³ is a hydrogen atom or an optionally substituted aliphatic hydrocarbon group, can be mentioned.

As another preferably embodiment of compound (I), a compound wherein
W is N;
X¹ is -NR³- wherein R³ is a hydrogen atom or an optionally substituted aliphatic hydrocarbon group;
A is an aryl group substituted by a group of the formula -Y²-B, and optionally further substituted, wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted; R² is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted, can be mentioned.

As another preferable embodiment of compound (I), a compound wherein W is N;
X¹ is -NR³-;
A is an aryl group substituted by a group of the formula -Y²-B, and optionally further substituted, wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted; and R² and R³ are bonded to each other to form an optionally substituted ring structure, can be mentioned.

As compound (I),
(i) 2-{2-[4-({3-chloro-4-[(3-fluorobenzyl)oxy]phenyl}amino)-5H-pyrrolo[3,2-d]pyrimidin-5-yl]ethoxy}ethanol,
(ii) 2-{2-[4-({3-chloro-4-[3-(trifluoromethyl)phenoxy]phenyl}amino)-5H-pyrrolo[3,2-d]pyrimidin-5-yl]ethoxy}ethanol,
(iii) N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide,
(iv) N-{2-[4-({3-chloro-4-[3-(trifluoromethyl)phenoxy]phenyl}amino)-5H-pyrrolo[3,2-d]pyrimidin-5-yl]ethyl}-2-(methylsulfonyl)acetamide,
(v) N-{2-[4-({3-chloro-4-[3-(trifluoromethyl)phenoxy]phenyl}amino)-5H-pyrrolo[3,2-d]pyrimidin-5-yl]ethyl}-2-methyl-2-(methylsulfonyl)propanamide,
(vi) 5-{2-[2-(tert-butylsulfonyl)ethoxy]ethyl}-N-{3-chloro-4-[3-(trifluoromethyl)phenoxy]phenyl}-5H-pyrrolo[3,2-d]pyrimidine-4-amine,
(vii) 2-(methylsulfonyl)-N-{2-[4-({3-methyl-4-[3-(trifluoromethoxy)phenoxy]phenyl}amino)-5H-pyrrolo[3,2-d]pyrimidin-5-yl]ethyl}acetamide,
(viii) N-[2-(4-{[3-chloro-4-(3-chlorophenoxy)phenyl]amino}-5H-pyrrolo[3,2-d]pyrimidin-5-yl)ethyl]-2-(methylsulfonyl)acetamide, and
(ix) N-{2-[4-({3-chloro-4-[3-(trifluoromethoxy)phenoxy]phenyl}amino)-5H-pyrrolo[3,2-d]pyrimidin-5-yl]ethyl}-2-(methylsulfonyl)acetamide and the like are preferable. Particularly, N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide is preferable.

Compound (I) and a salt thereof can be produced according to the method described in W02005/118588.

When compound (I) has isomers such as optical isomer, stereoisomer, positional isomer, rotational isomer and the like, and any isomers and mixtures are encompassed in the compound (I). For example, when compound (I) has an optical isomer, an optical isomer separated from a racemate is also encompassed in the compound (I). These isomers can be obtained as independent products by a synthesis means or a separation means (concentration, solvent extraction, column chromatography, recrystallization and the like) known *per se.* The compound (I) may be a crystal, and both a single crystal and crystal mixtures are encompassed in the compound (I). Crystals can be produced by crystallization according to crystallization methods known *per se*. The compound (I) may be a solvate (e.g., hydrate etc.) or a non-solvate, both of which are encompassed in the compound (I).
A compound labeled with an isotope (e.g., ²H, ³H, ¹⁴C, ³⁵S, ¹²⁵I and the like) is also encompassed in the compound (I).

As the salts of the compound (I), for example, metal salt, ammonium salt, salts with organic base, salts with inorganic acid, salts with organic acid, salts with basic or acidic amino acid and the like can be mentioned. As preferable examples of the metal salt, for example, alkali metal salts such as sodium salt, potassium salt and the like; alkaline earth metal salts such as calcium salt, magnesium salt, barium salt and the like; aluminum salt and the like can be mentioned. As preferable examples of the salts with organic base, for example, salts with trimethylamine, triethylamine, pyridine, picoline, 2,6-lutidine, ethanolamine, diethanolamine, triethanolamine, tromethamine [tris(hydroxymethyl)methylamine], t-butylamine, cyclohexylamine, dicyclohexylamine, N,N'-dibenzylethylenediamine and the like can be mentioned. As preferable examples of salts with inorganic acid, for example, salts with hydrochloric acid, hydrobromic acid, nitric acid, sulfuric acid, phosphoric acid and the like can be mentioned. As preferable examples of the salts with organic acid, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like can be mentioned. As preferable examples of the salts with basic amino acid, for example, salts with arginine, lysine, ornithine and the like can be mentioned, and as preferable examples of the salts with acidic amino acid, for example, salts with aspartic acid, glutamic acid and the like can be mentioned.

Of these, pharmaceutically acceptable salts are preferable. For example, when a compound contains an acidic functional group, inorganic salts such as alkali metal salts (e.g., sodium salt, potassium salt etc.), alkaline earth metal salts (e.g., calcium salt, magnesium salt, barium salt etc.) and the like, ammonium salt and the like, and when a compound contains a basic functional group, for example, salts with inorganic acid such as hydrochloric acid, hydrobromic acid, nitric acid, sulfuric acid, phosphoric acid and the like, or salts with organic acid such as acetic acid, phthalic acid, fumaric acid, oxalic acid, tartaric acid, maleic acid, citric acid, succinic acid, methanesulfonic acid, p-toluenesulfonic acid and the like can be mentioned.

A prodrug of the compound (I) or a salt thereof (hereinafter referred to as compound (I)) means a compound which is converted to the compound (I) with a reaction due to an enzyme, an gastric acid, etc. under the physiological condition in the living body, that is, a compound which is converted to the compound (I) with oxidation, reduction, hydrolysis, etc. according to an enzyme; a compound which is converted to the compound (I) by hydrolysis etc. due to gastric acid, etc. A prodrug for compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) to an acylation, alkylation, phosphorylation or boration (e.g., a compound obtained by subjecting an hydroxy group in compound (I) to an acetylation, palmitoylation, propanoylation, pivaloylation, succinylation, fumarylation, alanylation, dimethylaminomethylcarbonylation, etc.); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.) and the like. Any of these compounds can be produced from compound (I) by a method known *per se*.
A prodrug for compound (I) may also be one which is converted into compound (I) under a physiological condition, such as those described in IYAKUHIN no KAIHATSU (Development of Pharmaceuticals), Vol. 7, Design of Molecules, p.163-198, Published by HIROKAWA SHOTEN (1990).

In the present specification, the mTOR inhibitor is not particularly limited as long as it has an action to inhibit mTOR (mammalian Target of Rapamycin) and, for example, temsirolimus, rapamycin, AZD8055, RAD001, CCI-779, Ap23573/MK8669, BEZ235, XL-765 and the like can be mentioned. Among these, rapamycin is preferable.

In the present specification, the PI3 kinase inhibitor is not particularly limited as long as it has an action to inhibit PI3 (phosphatidylinositol-3-kinase) and, for example, PI-103:

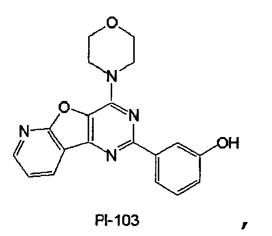

the compound described in W02005/011686, SF-1126, XL-147, BGT226, GDC-0941, BEZ235, XL-765 and the like can be mentioned. Among these, PI-103 is preferable.

In the present specification, the cMet inhibitor is not particularly limited as long as it has an action to inhibit cMet, which is a receptor type tyrosine kinase, and, for example, PF-2341066:

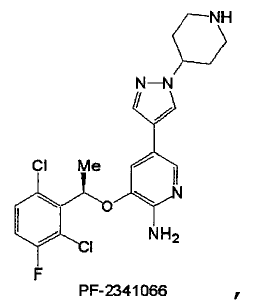

the compounds described in US-A-2004/0198750 and US-A-2007/0197537, ARQ-197, JNJ-38877605, SGX-523, XL-880, XL-187, MGCD-265 and the like can be mentioned. Among these, PF-2341066 is preferable.

The combination drug of the present invention may further contain a hormonal therapeutic agent or an anti-cancer agent. That is, 3 or more agents may be used in combination.

In the present specification, examples of the "hormonal therapeutic agents" include fosfestrol, diethylstylbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, Tadenan, mepartricin, raloxifene, ormeloxifene, levormeloxifene, anti-estrogens (e.g., tamoxifen citrate, toremifene citrate, and the like), ER down-regulator (e.g., fulvestrant (Faslodex (trademark)) and the like), human menopausal gonadotrophin, follicle stimulating hormone, pill preparations, mepitiostane, testrolactone, aminoglutethimide, LH-RH agonists (e.g., goserelin acetate, buserelin, leuprorelin, and the like), droloxifene, epitiostanol, ethinylestradiol sulfonate, aromatase inhibitors (e.g., fadrozole hydrochloride, anastrozole, retrozole, exemestane, vorozole, formestane, and the like), anti-androgens (e.g., flutamide, bicartamide, nilutamide, and the like), 5α-reductase inhibitors (e.g., finasteride, dutasteride, epristeride, and the like), adrenocorticohormone drugs (e.g., dexamethasone, prednisolone, betamethasone, triamcinolone, and the like), androgen synthesis inhibitors (e.g., abiraterone, and the like), retinoid and drugs that retard retinoid metabolism (e.g., liarozole, and the like), etc. and LH-RH agonists (e.g., goserelin acetate, buserelin, leuprorelin) and ER down-regulator (e.g., fulvestrant (Faslodex (trademark)) and the like) are preferable.

In the present specification, as the "anti-cancer agent", for example, chemotherapeutic agent, immunotherapeutic agent, a pharmaceutical agent that inhibits the action of cell growth factor and a receptor thereof and the like can be mentioned.

As examples of said "chemotherapeutic agents", there may be mentioned alkylating agents, antimetabolites, anticancer antibiotics, plant-derived anticancer agents, and the like.
Examples of the "alkylating agents" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, sodium estramustine phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin, and the like.
Examples of the "antimetabolites" include mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur, and the like), aminopterine, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine, pemetrexed disodium (Alimta (trademark)) and the like.
Examples of the "anticancer antibiotics" include actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride (Adriacin (trademark)), aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, and the like.
Examples of the "plant-derived anticancer agents" include etoposide, etoposide phosphate, vinblastine sulfate, vincristine sulfate, vindesine sulfate, teniposide, paclitaxel (Taxol (trademark), docetaxel, vinorelbine, and the like.

Examples of said "immunotherapeutic agents (BRM)" include picibanil, krestin, sizofiran, lentinan, ubenimex, interferons, interleukins, macrophage colony-stimulating factor, granulocyte colony-stimulating factor, erythropoietin, lymphotoxin, BCG vaccine, *Corynebacterium parvum,* levamisole, polysaccharide K, procodazole, and the like.

As the "growth factor" in said "pharmaceutical agents inhibiting the action of cell growth factors or cell growth factor receptors", there may be mentioned any substances that promote cell proliferation, which are normally peptides having a molecular weight of not more than 20,000 that are capable of exhibiting their activity at low concentrations by binding to a receptor, including (1) EGF (epidermal growth factor) or substances possessing substantially the same activity as it [e.g., EGF, heregulin, and the like], (2) insulin or substances possessing substantially the same activity as it [e.g., insulin, IGF (insulin-like growth factor)-1, IGF-2, and the like], (3) FGF (fibroblast growth factor) or substances possessing substantially the same activity as it [e.g., acidic FGF, basic FGF, KGF (keratinocyte growth factor), FGF-10, and the like], (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), and the like], and the like.
Examples of said "growth factor receptors" include any receptors capable of binding to the aforementioned growth factors, including EGF receptor, heregulin receptor (HER2), insulin receptor, IGF receptor, FGF receptor-1 or FGF receptor-2, and the like.
Examples of said "pharmaceutical agent that inhibits the action of cell growth factor" include HER2 antibody (trastuzumab (Herceptin (trademark)) etc.), imatinib mesylate, ZD1839 or EGFR antibody (cetuximab (Erbitux) (trademark)) etc.), antibody to VEGF (e.g., bevacizumab (Avastin) (trademark)), VEGFR antibody, VEGFR inhibitor, EGFR inhibitor (erlotinib (Tarceva)(trademark)) and gefitinib (Iressa (trademark)) etc.).

In addition to the aforementioned drugs, Akt inhibitors, L-asparaginase, aceglatone, procarbazine hydrochloride, protoporphyrin-cobalt complex salt, mercuric hematoporphyrin-sodium, topoisomerase I inhibitors (e.g., irinotecan hydrochloride (Topotecin (trademark), Campto (trademark), topotecan, and the like), topoisomerase II inhibitors (e.g., sobuzoxane, and the like), differentiation inducers (e.g., retinoid, vitamin D, and the like), angiogenesis inhibitors (e.g., thalidomide, SU11248, and the like), α-blockers (e.g., tamsulosin hydrochloride, naftopidil, urapidil, alfuzosin, terazosin, prazosin, silodosin, and the like) serine/threonine kinase inhibitor, endothelin receptor antagonist (e.g., atrasentan, and the like), proteasome inhibitor (e.g., bortezomib, and the like), Hsp 90 inhibitor (e.g., 17-AAG, and the like), spironolactone, minoxidil, 11α-hydroxyprogesterone, bone resorption inhibiting/metastasis suppressing agent (e.g., zoledronic acid, alendronic acid, pamidronic acid, etidronic acid, ibandronic acid, clodronic acid) and the like can be used.

Of those mentioned above, a hormonal therapeutic agent or anti-cancer agent, ER down-regulator (e.g., fulvestrant (Faslodex (trademark)) etc.), HER2 antibody (trastuzumab (Herceptin (trademark)) etc.), EGFR antibody (cetuximab (Erbitux (trademark) etc.), EGFR inhibitor (erlotinib (Tarceva (trademark), gefitinib (Iressa (trademark)) etc.), VEGFR inhibitor or a chemotherapeutic agent (paclitaxel (Taxol (trademark) etc.) is preferable.
Particularly, fulvestrant (Faslodex (trademark)), trastuzumab (Herceptin (trademark)), cetuximab (Erbitux (trademark)), erlotinib (Tarceva (trademark)), gefitinib (Iressa (trademark)), paclitaxel (Taxol (trademark)) and the like are preferable.
In addition, doxorubicin hydrochloride (Adriacin (trademark)), irinotecan hydrochloride (Topotecin (trademark), Campto (trademark)), 5FU, docetaxel and methotrexate are among the preferable examples.

When (1) an HER2 inhibitor having a pyrrolopyrimidine skeleton or pyrazolopyrimidine skeleton (hereinafter abbreviated as HER2 inhibitor) and (2) one or more pharmaceutical agents selected from an mTOR inhibitor, a PI3 kinase inhibitor and a cMet inhibitor (hereinafter to be abbreviated as a concomitant drug) are used in combination, the administration time of the HER2 inhibitor and the concomitant drug is not limited, and the HER2 inhibitor and the concomitant drug can be simultaneously administered to an administration subject, or may be administered in a staggered manner. The dosage of the concomitant drug may be determined according to the dose clinically used, and can be appropriately selected depending on an administration subject, administration route, disease, combination and the like. The administration mode of the HER2 inhibitor and the concomitant drug is not particularly restricted, and it is sufficient that the HER2 inhibitor and the concomitant drug are combined in administration. Examples of such administration mode include the following methods:
(1) The HER2 inhibitor and the concomitant drug are simultaneously produced to give a single preparation which is administered. (2) The HER2 inhibitor and the concomitant drug are separately produced to give two kinds of preparations which are administered simultaneously by the same administration route. (3) The HER2 inhibitor and the concomitant drug are separately produced to give two kinds of preparations which are administered by the same administration route only at the different times. (4) The HER2 inhibitor and the concomitant drug are separately produced to give two kinds of preparations which are administered simultaneously by different administration routes. (5) The HER2 inhibitor and the concomitant drug are separately produced to give two kinds of preparations which are administered by different administration routes at different times (e.g., the HER2 inhibitor and the concomitant drug are administered in this order, or in the reverse order).

The combination drug of the present invention is useful as a therapeutic agent suppressing growth of cancer expressing HER2 and/or EGFR kinase, and as an agent preventing hormone dependent cancer and hormone dependent cancer from transferring to hormone independent cancer. In addition, the combination drug is useful as a pharmaceutical agent since it shows low toxicity (e.g., acute toxicity, chronic toxicity, genetic toxicity, reproductive toxicity, cardiotoxicity, drug interaction, carcinogenicity and the like), high water solubility, and superior stability, in vivo kinetics (absorbability, distribution, metabolism, excretion and the like) and efficacy expression.
That is, the combination drug of the present invention can be used as a safe agent for the prophylaxis or treatment of diseases due to abnormal cell proliferation such as various cancers (particularly, breast cancer (e.g., invasive ductal carcinoma, ductal cancer in situ, inflammatory breast cancer etc.), prostate cancer (e.g., hormone-dependent prostate cancer, non-hormone dependent prostate cancer etc.), pancreatic cancer (e.g., pancreatic duct cancer etc.), gastric cancer (e.g., papillary adenocarcinoma, mucinous adenocarcinoma, adenosquamous carcinoma etc.), lung cancer (e.g., non-small cell lung cancer, small cell lung cancer, malignant mesothelioma etc.), colon cancer (e.g., gastrointestinal stromal tumor etc.), rectal cancer (e.g., gastrointestinal stromal tumor etc.), colorectal cancer (e.g., familial colorectal cancer, hereditary nonpolyposis colorectal cancer, gastrointestinal stromal tumor etc.), small intestinal cancer (e.g., non-Hodgkin lymphoma, gastrointestinal stromal tumor, etc.), esophagus cancer, duodenal cancer, cancer of the tongue, cancer of pharynx (e.g., nasopharyngeal carcinoma, oropharyngeal cancer, hypopharyngeal cancer etc.), salivary gland cancer, brain tumor (e.g., pineal astrocytoma, pilocytic astrocytoma, diffuse astrocytoma, anaplastic astrocytoma etc.), schwannoma, liver cancer (e.g., primary liver cancer, Extrahepatic Bile Duct Cancer etc.), kidney cancer (e.g., renal cell carcinoma, transitional cell cancers of renal pelvis and ureter etc.), cancer of the bile duct, endometrial carcinoma, cancer of the uterine cervix, ovarian cancer (e.g., ovarian epithelial cancer, extragonadal germ cell tumor, ovarian germ cell tumor, ovarian low malignant potential tumor etc.), urinary bladder cancer, urethral cancer, skin cancer (e.g., ocular melanoma, Merkel cell carcinoma etc.), hemangioma, malignant lymphoma, malignant melanoma, thyroid cancer (e.g., medullary thyroid carcinoma etc.), parathyroid cancer, nasal cavity cancer, paranasal sinus cancer, bone tumors (e.g., osteosarcoma, Ewing's tumor, uterus sarcoma, soft tissue sarcoma etc.), vascular fibroma, retinoblastoma, penile cancer, testis tumor, solid cancer in childhood (e.g., Wilms' tumor, childhood kidney tumor, etc.), Kaposi's sarcoma, Kaposi's sarcoma derived from AIDS, maxillary tumor, fibrous histiocytoma, leiomyosarcoma, rhabdomyosarcoma, leukemia (e.g., acute myeloid leukemia, acute lymphoblastic leukemia etc.) etc.), atherosclerosis, angiogenesis (e.g., angiogenesis associated with growth of solid cancer and sarcoma, angiogenesis associated with tumor metastasis, angiogenesis associated with diabetic retinopathy, etc.), and viral diseases (HIV infection etc.) and the like.
The tyrosine kinase dependent disease further includes cardiovascular diseases related to abnormal tyrosine kinase enzyme activity. Accordingly, the combination drug of the present invention can also be used as an agent for the prophylaxis or treatment of cardiovascular disease such as restenosis.
The combination drug of the present invention is useful as an anti-cancer agent for the prophylaxis or treatment of cancer, particularly breast cancer, ovarian cancer, prostate cancer, lung cancer, pancreatic cancer, kidney cancer, colorectal cancer, small intestinal cancer, esophagus cancer and gastric cancer and the like.
The combination drug of the present invention shows low toxicity and can be directly used as it is as a pharmaceutical agent or as a pharmaceutical composition containing a pharmaceutically acceptable carrier known *per se* etc. for a mammal (e.g., human, horse, bovine, dog, cat, rat, mouse, rabbit, swine, monkey etc.).
The combination drug of the present invention can be safely administered orally or parenterally (e.g., topical, rectal, intravenous administration etc.), for example, as a pharmaceutical composition obtained by mixing an HER2 inhibitor and/or the above-mentioned concomitant drug with a pharmacologically acceptable carrier according to a method known per *se,* such as tablet (including sugar-coated tablet, film-coated tablet), powder, granule, capsule (including soft capsule), liquid, injection, suppository, sustained-release preparation and the like. Injection can be administered intravenously, intramuscularly, subcutaneously, or by intraorgan administration or direct administration to the lesion.
As the pharmacologically acceptable carrier that may be used for the production of the combination drug of the present invention, various organic or inorganic carrier substances conventionally used as a preparation material can be mentioned. For example, excipient, lubricant, binder and disintegrant for solid preparations, solvent, solubilizing agents, suspending agent, isotonicity agent, buffer and soothing agent for liquid preparations and the like can be mentioned. Where necessary, conventional additives such as preservatives, antioxidants, colorants, sweetening agents, adsorbing agents, wetting agents and the like can be used as appropriate in suitable amounts.

As the excipient, for example, lactose, sucrose, D-mannitol, starch, corn starch, crystalline cellulose, light anhydrous silicic acid and the like can be mentioned.
As the lubricant, for example, magnesium stearate, calcium stearate, talc, colloidal silica and the like can be mentioned.
As the binder, for example, crystalline cellulose, sucrose, D-mannitol, dextrin, hydroxypropylcellulose, hydroxypropylmethylcellulose, polyvinylpyrrolidone, starch, sucrose, gelatin, methylcellulose, carboxymethylcellulose sodium and the like can be mentioned.
As the disintegrant, for example, starch, carboxymethylcellulose, carboxymethylcellulose calcium, sodium carboxymethyl starch, L-hydroxypropylcellulose and the like can be mentioned.
As the solvent, for example, water for injection, alcohol, propylene glycol, macrogol, sesame oil, corn oil, olive oil and the like can be mentioned.
As the solubilizing agents, for example, polyethylene glycol, propylene glycol, D-mannitol, benzyl benzoate, ethanol, trisaminomethane, cholesterol, triethanolamine, sodium carbonate, sodium citrate and the like can be mentioned.
As the suspending agent, for example, surfactant such as stearyltriethanolamine, sodium lauryl sulfate, lauryl aminopropionic acid, lecithin, benzalkonium chloride, benzethonium chloride, glyceryl monostearate and the like; hydrophilic polymer such as polyvinyl alcohol, polyvinylpyrrolidone, carboxymethylcellulose sodium, methylcellulose, hydroxymethylcellulose, hydroxyethylcellulose, hydroxypropylcellulose and the like; and the like can be mentioned.
As the isotonicity agent, for example, glucose, D-sorbitol, sodium chloride, glycerol, D-mannitol and the like can be mentioned.
As the buffer, for example, phosphate buffer, acetate buffer, carbonate buffer, citrate buffer and the like can be mentioned.
As the soothing agent, for example, benzyl alcohol and the like can be mentioned.
As the preservative, for example, paraoxybenzoic acid esters, chlorobutanol, benzyl alcohol, phenethyl alcohol, dehydroacetic acid, sorbic acid and the like can be mentioned.
As the antioxidant, for example, sulfite, ascorbic acid, α-tocopherol and the like can be mentioned.

The mixing ratio of an HER2 inhibitor and a concomitant drug in the combination drug of the present invention can be appropriately selected according to the subject of administration, administration route, disease and the like.
For example, while the content of the HER2 inhibitor in the combination drug of the present invention varies depending on the form of the preparation, it is generally about 0.01 to 100 wt%, preferably about 0.1 to 50 wt%, more preferably about 0.5 to about 20 wt%, relative to the whole preparation.
While the content of the concomitant drug in the combination drug of the present invention varies depending on the form of the preparation, it is generally about 0.01 to 100 wt%, preferably about 0.1 to 50 wt%, more preferably about 0.5 to about 20 wt%, relative to the whole preparation.
While the content of the additive such as a carrier in the combination drug of the present invention varies depending on the form of the preparation, it is generally about 1 to 99.99 wt%, preferably about 10 to about 90 wt%, relative to the whole preparation.
When an HER2 inhibitor and a concomitant drug are separately made into preparations, similar contents can be employed.

These preparations can be produced by a method known *per se*, which is generally used for a preparation step.
For example, an HER2 inhibitor or a concomitant drug can be prepared into an injection by forming an aqueous injection together with a dispersing agent (e.g., Tween 80 (manufactured by Atlas Powder, US), HCO 60 (manufactured by Nikko Chemicals), polyethylene glycol, carboxymethylcellulose, sodium alginate, hydroxypropylmethylcellulose, dextrin and the like), stabilizer (e.g., ascorbic acid, sodium pyrosulfite etc.), surfactant (e.g., polysorbate 80, macrogol etc.), solubilizer (e.g., glycerol, ethanol etc.), buffer (e.g., phosphoric acid and alkali metal salt thereof, citric acid and alkali metal salt thereof etc.), isotonicity agent (e.g., sodium chloride, potassium chloride, mannitol, sorbitol, glucose etc.), pH adjuster (e.g., hydrochloric acid, sodium hydroxide etc.), preservative (e.g., ethyl parahydroxybenzoate, benzoic acid, methyl parahydroxybenzoate, propyl parahydroxybenzoate, benzyl alcohol etc.), dissolving agent (e.g., concentrated glycerol, meglumine etc.), solubilizing agents (e.g., propylene glycol, sucrose etc.), soothing agent (e.g., glucose, benzyl alcohol etc.) and the like, or an oil injection by dissolving, suspending or emulsifying in vegetable oil such as olive oil, sesame oil, cottonseed oil, corn oil and the like, and solubilizing agents such as propylene glycol and the like.

A preparation for oral administration can be produced by adding, for example, an excipient (e.g., lactose, sucrose, starch, corn starch and the like), a disintegrating agent (e.g., starch, calcium carbonate and the like), a binder (e.g., starch, gum arabic, carboxymethylcellulose, polyvinylpyrrolidone, hydroxypropylcellulose, gelatin and the like), a lubricant (e.g., talc, magnesium stearate, polyethylene glycol 6000 and the like) and the like to an HER2 inhibitor or a concomitant drug according to a method known *per se*, and compression molding the mixture, then when desired, coating the molder product by a method known *per se* for the purpose of masking of taste, enteric property or durability, to give a preparation for oral administration. As the coating agent, for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudoragit (methacrylic acid acrylic acid copolymer, manufactured by Rohm, Germany), pigment (e.g., red iron oxide, titanium dioxide, etc.) and the like can be used. As the sugar coating, for example, saccharose, talc, gum arabic, pigment (e.g., red iron oxide, titanium dioxide etc.), polishing agent (e.g., beeswax etc.), and the like can be used. The preparation for oral administration may be any of an immediate-release preparation and a sustained-release preparation.
For example, for production of a suppository, an HER2 inhibitor or a concomitant drug can be formulated into an oily or aqueous solid, semi-solid or liquid suppository according to a method known *per se*. As the oily base to be used for the above-mentioned composition, for example, glycerides of higher fatty acids [e.g., cacao butter, Witepsols (manufactured by Dynamit Nobel, Germany), etc.], medium chain fatty acid [e.g., Miglyols (manufactured by Dynamit Nobel, Germany), etc.], or vegetable oils (e.g., sesame oil, soybean oil, cottonseed oil and the like), and the like are listed. Further, as the aqueous substrate, for example, polyethylene glycols, propylene glycol are listed, and as the aqueous gel substrate, for example, natural gums, cellulose derivatives, vinyl polymers, acrylic acid polymers and the like can be mentioned.
As the above-mentioned sustained-release preparation, sustained-release microcapsule and the like can be mentioned.
A sustained-release microcapsule can be prepared by a method known *per se.*
The HER2 inhibitor is preferably molded into an oral administration preparation such as a solid preparation (e.g., powder, granule, tablet, capsule) and the like, or molded into a rectal administration preparation such as a suppository. Particularly, an oral administration preparation is preferable.
The concomitant drug can be made into the above-mentioned drug form depending on the kind of the drug.

While the dose of the combination drug of the present invention varies depending on the kind of HER2 inhibitor, age, body weight, symptom, dosage form, administration method, administration period and the like, for example, it is generally, as an HER2 inhibitor and a concomitant drug, each within the range of about 0.1 mg - about 500 mg, preferably about 1 mg - about 100 mg, for oral administration, and each about 0.01 mg - about 100 mg, more preferably about 0.1 mg - about 10 mg for parenteral administration, for one patient (adult, body weight about 60 kg). The dose can be administered in 1 - 3 portions a day. Of course, since the dose as described above varies depending on various conditions, amounts smaller than the above-mentioned dosage may sometimes be sufficient, further, amounts over that range sometimes have to be administered.
The amount of the concomitant drug can be set at any value unless side effects are problematical. The daily dosage in terms of the concomitant drug differs depending on the severity of the symptom, age, sex, body weight, sensitivity difference of the subject, administration period, interval, and nature, pharmacy, kind of the pharmaceutical preparation, kind of effective ingredient, and the like, and not particularly restricted, and the amount of a drug is, in the case of oral administration for example, usually from about 0.001 to 2000 mg, preferably from about 0.01 to 500 mg, further preferably from about 0.1 to 100 mg, and in the case of parenteral administration for example, usually from about 0.0001 to 400 mg, preferably from about 0.001 to 200 mg per 1 kg of a mammal and this is usually administered once to 3 times in division a day.

For administration of the combination drug of the present invention, an HER2 inhibitor may be administered after administration of the concomitant drug or the concomitant drug may be administered after administration of an HER2 inhibitor, though they may be administered simultaneously. When administered at a time interval, the interval varies depending on the effective ingredient, dosage form and administration method, and, for example, when the concomitant drug is administered first, a method in which an HER2 inhibitor is administered within time range of from 1 minute to 3 days, preferably from 10 minutes to 1 day, more preferably from 15 minutes to 1 hour, after administration of the concomitant drug is exemplified. When an HER2 inhibitor is administered first, a method in which the concomitant drug is administered within time range of from 1 minute to 1 day, preferably from 10 minutes to 6 hours, more preferably from 15 minutes to 1 hour after administration of an HER2 inhibitor is exemplified.
As the preferable administration method, for example, about 0.001 - 200 mg/kg body weight of a concomitant drug formulated as a parenteral administration preparation is administered by intravenous injection or intramuscular injection and, about 15 min later, about 0.005 - 100 mg/kg body weight of an HER2 inhibitor formulated as an oral administration preparation is orally administered, for a daily dose.

In addition, since compound (I) of the present invention has a thymidine synthase production inhibitory action, it can be used as an active ingredient of a single agent, a therapeutic agent for suppressing cancer growth, or an agent for preventing hormone dependent cancer and hormone dependent cancer from transferring to hormone independent cancer. Compound (I) of the present invention is useful as a pharmaceutical agent since it shows low toxicity (e.g., acute toxicity, chronic toxicity, genetic toxicity, reproductive toxicity, cardiotoxicity, drug interaction, carcinogenicity and the like), high water solubility, and superior stability, in vivo kinetics (absorbability, distribution, metabolism, excretion and the like) and efficacy expression.
That is, compound (I) of the present invention can be used as an active ingredient of a single agent, as well as a safe agent for the prophylaxis or treatment of diseases due to abnormal cell proliferation such as various cancers (particularly, breast cancer (e.g., invasive ductal carcinoma, ductal cancer in situ, inflammatory breast cancer etc.), prostate cancer (e.g., hormone-dependent prostate cancer, non-hormone dependent prostate cancer etc.), pancreatic cancer (e.g., pancreatic duct cancer etc.), gastric cancer (e.g., papillary adenocarcinoma, mucinous adenocarcinoma, adenosquamous carcinoma etc.), lung cancer (e.g., non-small cell lung cancer, small cell lung cancer, malignant mesothelioma etc.), colon cancer (e.g., gastrointestinal stromal tumor etc.), rectal cancer (e.g., gastrointestinal stromal tumor etc.), colorectal cancer (e.g., familial colorectal cancer, hereditary nonpolyposis colorectal cancer, gastrointestinal stromal tumor etc.), small intestinal cancer (e.g., non-Hodgkin lymphoma, gastrointestinal stromal tumor, etc.), esophagus cancer, duodenal cancer, cancer of the tongue, cancer of pharynx (e.g., nasopharyngeal carcinoma, oropharyngeal cancer, hypopharyngeal cancer etc.), salivary gland cancer, brain tumor (e.g., pineal astrocytoma, pilocytic astrocytoma, diffuse astrocytoma, anaplastic astrocytoma etc.), schwannoma, liver cancer (e.g., primary liver cancer, Extrahepatic Bile Duct Cancer etc.), kidney cancer (e.g., renal cell carcinoma, transitional cell cancers of renal pelvis and ureter etc.), cancer of the bile duct, endometrial carcinoma, cancer of the uterine cervix, ovarian cancer (e.g., ovarian epithelial cancer, extragonadal germ cell tumor, ovarian germ cell tumor, ovarian low malignant potential tumor etc.), urinary bladder cancer, urethral cancer, skin cancer (e.g., ocular melanoma, Merkel cell carcinoma etc.), hemangioma, malignant lymphoma, malignant melanoma, thyroid cancer (e.g., medullary thyroid carcinoma etc.), parathyroid cancer, nasal cavity cancer, paranasal sinus cancer, bone tumor s (e.g., osteosarcoma, Ewing's tumor, uterus sarcoma, soft tissue sarcoma etc.), vascular fibroma, retinoblastoma, penile cancer, testis tumor, solid cancer in childhood (e.g., Wilms' tumor, childhood kidney tumor, etc.), Kaposi's sarcoma, Kaposi's sarcoma derived from AIDS, maxillary tumor, fibrous histiocytoma, leiomyosarcoma, rhabdomyosarcoma, leukemia (e.g., acute myeloid leukemia, acute lymphoblastic leukemia etc.) etc.), atherosclerosis, angiogenesis (e.g., angiogenesis associated with growth of solid cancer and sarcoma, angiogenesis associated with tumor metastasis, angiogenesis associated with diabetic retinopathy, etc.), and viral diseases (HIV infection etc.) and the like.

A thymidine synthase production inhibitor containing compound (I) of the present invention shows low toxicity and can be directly used as it is as a pharmaceutical agent or as a pharmaceutical composition containing a pharmaceutically acceptable carrier known *per se* etc. for a mammal (e.g., human, horse, bovine, dog, cat, rat, mouse, rabbit, swine, monkey etc.).
The thymidine synthase production inhibitor of the present invention can be safely administered orally or parenterally (e.g., topical, rectal, intravenous administration etc.), for example, as a pharmaceutical composition obtained by mixing compound (I) with a pharmacologically acceptable carrier according to a method known *per se,* such as tablet (including sugar-coated tablet, film-coated tablet), powder, granule, capsule (including soft capsule), liquid, injection, suppository, sustained-release preparation and the like. Injection can be administered intravenously, intramuscularly, subcutaneously, or by intraorgan administration or direct administration to the lesion.
Examples of the pharmacologically acceptable carrier usable for the production of the thymidine synthase production inhibitor of the present invention include those similar to the pharmacologically acceptable carriers usable for the production of the combination drug of the present invention.

While the content of compound (I) in the thymidine synthase production inhibitor of the present invention varies depending on the form of the preparation, it is generally about 0.01 to 100 wt%, preferably about 0.1 to 50 wt%, more preferably about 0.5 to about 20 wt%, relative to the whole preparation.

These preparations can be produced by a method known *per se*, which is generally used for a preparation formulation step. As for the combination drug of the present invention, for example, methods similar to those mentioned above can be employed.
Compound (I) is preferably formed into an oral administration preparation such as a solid preparation (e.g., powder, granule, tablet, capsule) and the like, or formed into a rectal administration preparation such as a suppository. Particularly, an oral administration preparation is preferable.

While the dose of the thymidine synthase production inhibitor of the present invention varies depending on the kind of compound (I), age, body weight, symptom, dosage form, administration method, administration period and the like, for example, it is generally, as compound (I), within the range of about 0.1 mg - about 500 mg, preferably about 1 mg - about 100 mg, for oral administration, and about 0.01 mg - about 100 mg, more preferably about 0.1 mg - about 10 mg, for parenteral administration, for one patient (adult, body weight about 60 kg). The dose can be administered in 1 - 3 portions a day. Of course, since the dose as described above varies depending on various conditions, amounts smaller than the above-mentioned dosage may sometimes be sufficient, and further, amounts over that range sometimes have to be administered.

### Examples

The production method and use method of the present invention are explained in the following by referring to Examples and Experimental Examples, which are not to be construed as limitative. Other embodiments that fall within the idea and scope of the invention defined in the Claims are also encompassed in the present invention. In Examples and Experimental Examples, compound A means N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutaneamide.

### Example 1

| | | |
|---|---|---|
| (1) | compound A | 8.0 g |
| (2) | lactose | 60.0 g |
| (3) | corn starch | 35.0 g |
| (4) | gelatin | 3.0 g |
| (5) | magnesium stearate | 2.0 g |

A mixture of compound A (8.0 g), lactose (60.0 g) and corn starch (35.0 g) is granulated using 10 wt% aqueous gelatin solution (30 mL, 3.0 g as gelatin) and, by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Example 2

Rapamycin (50 mg) is dissolved in Japanese Pharmacopoeia distilled water for injection (50 mL), and Japanese Pharmacopoeia distilled water for injection is added to 100 mL. This solution is filtered under sterile conditions. The solution (1 mL) is taken, filled in a vial for injection under sterile conditions, freeze-dried and sealed.

### Example 3

PI-103 (50 mg) is dissolved in Japanese Pharmacopoeia distilled water for injection (50 mL), and Japanese Pharmacopoeia distilled water for injection is added to 100 mL. This solution is filtered under sterile conditions. The solution (1 mL) is taken, filled in a vial for injection under sterile conditions, freeze-dried and sealed.

### Example 4

PF-2341066 (50 mg) is dissolved in Japanese Pharmacopoeia distilled water for injection (50 mL), and Japanese Pharmacopoeia distilled water for injection is added to 100 mL. This solution is filtered under sterile conditions. The solution (1 mL) is taken, filled in a vial for injection under sterile conditions, freeze-dried and sealed.

### Example 5

| | | |
|---|---|---|
| (1) | compound A | 8.0 g |
| (2) | rapamycin | 8.0 g |
| (3) | lactose | 60.0 g |
| (4) | corn starch | 35.0 g |
| (5) | gelatin | 3.0 g |
| (6) | magnesium stearate | 2.0 g |

A mixture of compound A (8.0 g), rapamycin (8.0 g), lactose (60.0 g) and corn starch (35.0 g) is granulated using 10 wt% aqueous gelatin solution (30 mL, 3.0 g as gelatin) and by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Example 6

| | | |
|---|---|---|
| (1) | compound A | 8.0 g |
| (2) | PI-103 | 8.0 g |
| (3) | lactose | 60.0 g |
| (4) | corn starch | 35.0 g |
| (5) | gelatin | 3.0 g |
| (6) | magnesium stearate | 2.0 g |

A mixture of compound A (8.0 g), PI-103 (8.0 g), lactose (60.0 g) and corn starch (35.0 g) is granulated using 10 wt% aqueous gelatin solution (30 mL, 3.0 g as gelatin) and by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Example 7

| | | |
|---|---|---|
| (1) | compound A | 8.0 g |
| (2) | PF-2341066 | 8.0 g |
| (3) | lactose | 60.0 g |
| (4) | corn starch | 35.0 g |
| (5) | gelatin | 3.0 g |
| (6) | magnesium stearate | 2.0 g |

A mixture of compound A (8.0 g), PF-2341066 (8.0 g), lactose (60.0 g) and corn starch (35.0 g) is granulated using 10 wt% aqueous gelatin solution (30 mL, 3.0 g as gelatin) and by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Experimental Example 1

### Effect of combined use of compound A and PI3 kinase inhibitor PI-103 (in vitro)

Human breast cancer cell line BT-474 cells were plated on a 96 well plate at 6000 cells/100 µL/well. The next day, compound A alone, PI-103 alone or a mixture of compound A and PI-103 was diluted serially and added thereto. A compound was added and the mixture was left standing in a CO₂ incubator for 5 days. 50 µL of 25% glutaraldehyde solution (Wako) was added to 200 µL of the medium and the mixture was left standing at room temperature for 15 min. Then, the plate was washed once with PBS, 200 µL of PBS was added and 25 µL of 50% trichloroacetic acid was added thereto, and the mixture was left standing at 4°C for 1 hr or longer. The plate was washed 5 times with tap water, and redundant water was removed by flapping the plate against KIMTOWEL. 50 µL of 0.4% (w/v) Sulforhodamine B (SRB, Sigma)-containing 1% (v/v) acetic acid solution was added to each well, and after 15 min, the plate was washed 3 times with 1% acetic acid solution (v/v). The plate was dried well, and 10 mM Tris solution was added. The mixture was stirred well in a plateshaker, and the absorbance at 550 nm was measured (Bio-Rad, Benchmark Plus). The control without a drug was taken as 100% for the calculation (Fig. 1). The combined use of compound A and PI-103 exhibited a strong cell proliferation inhibitory action as compared to single use of each.

### Experimental Example 2

### Effect of combined use of compound A and mTOR inhibitor, rapamycin (in vitro)

Human breast cancer cell line SK-BR-3 cells were plated on a 96 well plate at 2000 cells/100 µL/well. The next day, compound A alone, rapamycin alone or a mixture of compound A and rapamycin was diluted serially and added thereto. A compound was added and the mixture was left standing in a CO₂ incubator for 5 days. 50 µL of 25% glutaraldehyde solution (Wako) was added to 200 µL of the medium and the mixture was left standing at room temperature for 15 min. Then, the plate was washed once with PBS, 200 µL of PBS was added and 25 µL of 50% trichloroacetic acid was added thereto, and the mixture was left standing at 4°C for 1 hr or longer. The plate was washed 5 times with tap water, and redundant water was removed by flapping the plate against KIMTOWEL. 50 µL of 0.4% (w/v) Sulforhodamine B (SRB, Sigma)-containing 1% (v/v) acetic acid solution was added to each well, and after 15 min, the plate was washed 3 times with 1% acetic acid solution (v/v). The plate was dried well, and 10 mM Tris solution was added. The mixture was stirred well in a plateshaker, and the absorbance at 550 nm was measured (Bio-Rad, Benchmark Plus). The control without a drug was taken as 100% for the calculation (Fig. 2). The combined use of compound A and rapamycin exhibited a strong cell proliferation inhibitory action as compared to single use of each.

### Experimental Example 3

### Effect of combined use of compound A and cMet inhibitor, PF-2341066 (in vitro)

Human epidermis cancer cell line A431 cells were plated on a 96 well plate at 2000 calls/100 µL/well. The next day, compound A alone, PF-2341066 alone or a mixture of compound A and PF-2341066 was diluted serially and added thereto. A compound was added and the mixture was left standing in a CO₂ incubator for 5 days. 50 µL of 25% glutaraldehyde solution (Wako) was added to 200 µL of the medium and the mixture was left standing at room temperature for 15 min. Then, the plate was washed once with PBS, 200 µL of PBS was added and 25 µL of 50% trichloroacetic acid was added thereto, and the mixture was left standing at 4°C for 1 hr or longer. The plate was washed 5 times with tap water, and redundant water was removed by flapping the plate against KIMTOWEL. 50 µL of 0.4% (w/v) Sulforhodamine B (SRB, Sigma)-containing 1% (v/v) acetic acid solution was added to each well, and after 15 min, the plate was washed 3 times with 1% acetic acid solution (v/v). The plate was dried well, and 10 mM Tris solution was added. The mixture was stirred well in a plateshaker, and the absorbance at 550 nm was measured (Bio-Rad, Benchmark Plus). The control without a drug was taken as 100% for the calculation (Fig. 3). The combined use of compound A and PF-2341066 exhibited a strong cell proliferation inhibitory action as compared to single use of each.

### Experimental Example 4

### Measurement of expression amount of TS (thymidine) by addition of compound A

Human breast cancer cell line BT-474 (ATCC (American Type Culture Collection) catalog No. HTB-20, Lasfargues EY, In Vitro 15:723-729(1979)), which is a passage cultured HER2 high expressing cell, was treated with trypsin and suspended in RPMI-1640 medium (GibcoInvitrogen) containing 10% bovine fetus serum (GibcoInvitrogen). The cell density of the cell suspension was measured by cell counter Sysmex CDA500, and the cell density was adjusted to 5 x 10⁴ cell/mL using the aforementioned medium. The suspension was dispensed to each well of a 6 well multi-well culture plate (Corning) by 2 mL, and cultured overnight at 37°C under 5% CO₂. Compound A was added to the culture plate to 100 nmol/L. Two days after the addition of the compound, each total RNA was extracted using an RNA easy Mini kit (QIAGEN), and conversed to cDNA using TaqMan Reverse Transcription Reagent (Applied Biosystems). Using this cDNA as a template, the TS expression amount was measured using TaqMan Gene Expression Assays (Applied Biosystems). The expression was detected by GeneAmp7700 (Applied Biosystems), and analyzed by the
ΔΔCt method
(Applied Biosystems) with GAPDH as an internal standard (Fig. 4). Addition of compound A at 100 nmol/L remarkably decreased the TS expression amount.

### Reference Example 1

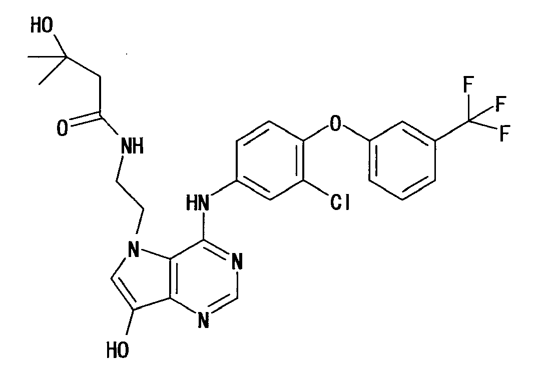

### Preparation of N-{2-[4-({3-chloro-4-[3-(trifluoromethyl)phenox y]phenyl}amino)-7-hydroxy-5H-pyrrolo[3,2-d]pyrimidin-5-yl]ethy 1}-3-hydroxy-3-methylbutaneamide

P450 expressing Escherichia coli transformed cell line BL21star/pETAciBC-50AABP195 colony was inoculated to a 15 mL test tube containing 2 mL of LB medium (1.0% Bacto-Tryptone, 0.5% yeast extract, 1.0% sodium chloride) containing carbenicillin (0.050 mg/mL) and subjected to shaking culture at 25°C for 24 hr. The culture medium (0.5 mL) was inoculated to a 500 mL flask containing 50 mL of LB medium (1.0% Bacto-Tryptone, 0.5% yeast extract, 1.0% sodium chloride) containing carbenicillin (0.050 mg/mL), and three in total of such flask were prepared and subjected to shaking culture at 25°C for 24 hr. The culture medium (10 mL) was inoculated to a 500 mL flask containing 100 mL of M9mix medium (3.39% disodium phosphate, 1.5% potassium dihydrogen phosphate, 0.25% sodium chloride, 0.5% ammonium chloride, 1% casamino acid, 0.002% thymine, 0.1 mM calcium chloride, 0.1 mM iron sulfate) containing carbenicillin (0.050 mg), and 140 in total of such flask were prepared and subjected to shaking culture at 25°C for 24 hr. The thus-obtained culture medium (14 L) was centrifuged at 3500 rpm for 10 min, 3L of CV2 buffer (50 mM potassium phosphate buffer, 2% glycerol, 0.050 mg/mL carbenicillin, 0.1M IPTG) was added to the obtained fungus, and the mixture was uniformly suspended and equally dispended to sixty 500 mL flasks. A 5%(w/v) N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutaneamide solution (0.5 mL) and 1 mL of 25% (w/v) aqueous PMCD solution were each added to a flask. The thus-obtained reaction mixture for conversion was reacted at 28°C for 24 hr. The reaction mixture (3 L) was adjusted to pH 5.6 with 6N aqueous hydrochloric acid, sodium chloride was added in an amount of 20%(w/v) of the reaction mixture and ethyl acetate was added in an amount equal to the reaction mixture. The mixture was stirred in a vortex, and centrifuged in a centrifugation machine at 8,000 rpm for 15 min. The thus-obtained ethyl acetate phase (3 L) was concentrated to dryness. A part of the concentrated dry product was subjected to high performance liquid chromatography analysis. As a result, a dry product (6.58 g) was obtained. 6.4 g therefrom was divided into two (3.2 g each) and applied to silica gel column chromatography (manufactured by Merck, silica gel 60, 150 g) packed with hexane/ethyl acetate (1:9). Thereafter, a fraction containing the title compound was eluted with 0.9 L of ethyl acetate/methanol (70:30). The fraction was concentrated to dryness under reduced pressure to give 1.9 g and 2.0 g of dry product. The obtained dry products were dissolved in dimethyl sulfoxide, and applied to moderate-pressure column chromatography (manufactured by YAMAZEN, cartridge column ODS-S-50C-M (vol=107 mL) or ODS-S-50D-L (vol=294 mL)) each substituted by methanol/10 mM ammonium formate solution (pH 3.0) (30:70 or 50:50). Thereafter, a fraction containing the title compound was eluted with 0.6 L of methanol/10 mM ammonium formate solution (pH 3.0) ((80:20) or (90:10)). This fraction was concentrated under reduced pressure, and methanol was evaporated. The residual solution was freeze-dried and the obtained two portions of freeze-dried powder were combined to give 1.02 g of a freeze-dried powder. The obtained freeze-dried powder was separated by about 100 mg by high performance liquid chromatography (column: CAPCELPAK MGII manufactured by Shiseido Co., Ltd., 50 mm i.d. X 250 mm, mobile phase:acetonitrile/10 mM ammonium acetate (pH 5.0) (50:50), flow rate 50 mL/min, column temperature: room temperature), a fraction containing the title compound was concentrated under reduced pressure, and acetonitrile was evaporated. The residual solution was partitioned by extraction with ethyl acetate, and the ethyl acetate layer was concentrated to dryness to give the title compound (262 mg, recovery rate about 75%) as a brown solid.
¹H NMR (DMSO-d₆) δ 1.12 (6H, s), 2.19 (2H, s), 3.34 - 3.39 (2H, m), 4.41 (2H, t, J=7 Hz), 4.68 (1H, br. s.), 7.20 (1H, s), 7.21 - 7.25 (2H, m), 7.31 (1H, d, J=9 Hz), 7.48 (1H, d, J=8 Hz), 7.62 (1H, t, J=8 Hz), 7.81 (1H, dd, J=9, 2 Hz), 8.05 (1H, d, J=2 Hz), 8.28 (1H, t, J=6 Hz), 8.36 (1H, s), 9.10 (1H, br. s.), 9.17 (1H, br. s.)

### Reference Example 2

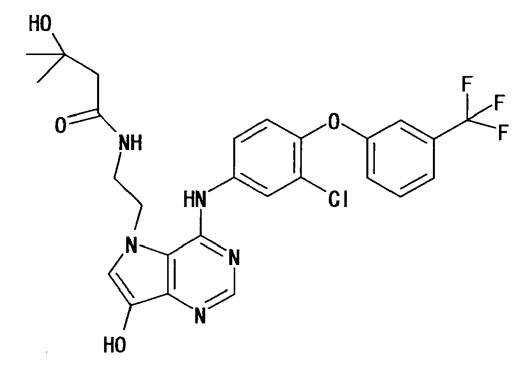

### Preparation of N-{2-[4-({3-chloro-4-[3-(trifluoromethyl)phenoxy]phenyl}amino)-7-hydroxy-5H-pyrrolo[3,2-d]pyrimidin-5-yl]ethyl}-3-hydroxy-3-methylbutaneamide

P450 expressing Escherichia coli transformed cell line BLstarTolC/pETAciBC-50AABP195 colony was inoculated to a 15 mL test tube containing 2 mL of LB medium (1.0% Bacto-Tryptone, 0.5% yeast extract, 1.0% sodium chloride) containing carbenicillin (50 µg/mL) and subjected to shaking culture at 25°C for 24 hr. The culture medium (0.5 mL) was inoculated to a 500 mL flask containing 50 mL of LB medium (1.0% Bacto-Tryptone, 0.5% yeast extract, 1.0% sodium chloride) containing carbenicillin (0.050 mg/mL), and three in total of such flask were prepared and subjected to shaking culture at 25°C for 24 hr. The culture medium (10 mL) was inoculated to a 500 mL flask containing 100 mL of M9 mix medium (3.39% disodium phosphate, 1.5% potassium dihydrogen phosphate, 0.25% sodium chloride, 0.5% ammonium chloride, 1% casamino acid, 0.002% thymine, 0.1 mM calcium chloride, 0.1 mM iron sulfate) containing carbenicillin (0.050 mg), and 140 in total of such flask were prepared and subjected to shaking culture at 25°C for 24 hr. The thus-obtained culture medium (14 L) was centrifuged at 3500 rpm for 10 min, 3.5L of CV2 buffer (50 mM potassium phosphate buffer, 2% glycerol, 0.050 mg/mL carbenicillin, 0.1M IPTG) was added to the obtained fungus, and the mixture was uniformly suspended and equally dispended to seventy 500 mL flasks. A 10%(w/v) N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutaneamide solution (0.5 mL) and 1 mL of 25% (w/v) aqueous PMCD solution were each added to a flask. The thus-obtained reaction mixture for conversion was reacted at 28°C for 24 hr. The reaction mixture (3.5 L) was adjusted to pH 5.5 with 6N aqueous hydrochloric acid, sodium chloride was added in an amount of 20%(w/v) of the reaction mixture and ethyl acetate was added in an amount equal to the reaction mixture. The mixture was stirred in a vortex, and centrifuged in a centrifugation machine at 8,000 rpm for 15 min. The thus-obtained ethyl acetate phase was concentrated to dryness. A part of the concentrated dry product was subjected to high performance liquid chromatography analysis. As a result, a dry product 1 containing the title compound was obtained.
In addition, P450 expressing Escherichia coli transformed cell line BLstarTolC/pETAciBC-50AABP195 colony was inoculated to a 15 mL test tube containing 2 mL of LB medium (1.0% Bacto-Tryptone, 0.5% yeast extract, 1.0% sodium chloride) containing carbenicillin (0.050 mg/mL) and subjected to shaking culture at 25°C for 24 hr. The culture medium (0.5 ml) was inoculated to a 500 mL flask containing 50 mL of LB medium (1.0% Bacto-Tryptone, 0.5% yeast extract, 1.0% sodium chloride) containing carbenicillin (0.050 mg/mL), and subjected to shaking culture at 25°C for 24 hr. The culture medium (10 mL) was inoculated to a 500 mL flask containing 100 mL of M9mix medium (3.39% disodium phosphate, 1.5% potassium dihydrogen phosphate, 0.25% sodium chloride, 0.5% ammonium chloride, 1% casamino acid, 0.002% thymine, 0.1 mM calcium chloride, 0.1 mM iron sulfate) containing carbenicillin (0.050 mg), and 25 in total of such flask were prepared and subjected to shaking culture at 25°C for 24 hr. The thus-obtained culture medium (2.5 L) was centrifuged at 3500 rpm for 10 min, 0.6 L of CV2 buffer (50 mM potassium phosphate buffer, 2% glycerol, 0.050 mg/mL carbenicillin, 0.1M IPTG) was added to the obtained fungus, and the mixture was uniformly suspended and equally dispended to twelve 500 mL flasks. A 5%(w/v) N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutaneamide solution (1 mL) and 2 mL of 25% (w/v) aqueous PMCD solution were each added to a flask. The thus-obtained reaction mixture for conversion was reacted at 28°C for 10 hr. The reaction mixture (0.6 L) was adjusted to pH 5.5 with 6N aqueous hydrochloric acid, sodium chloride was added in an amount of 20%(w/v) of the reaction mixture and ethyl acetate was added in an amount equal to the reaction mixture. The mixture was stirred in a vortex, and centrifuged in a centrifugation machine at 8,000 rpm for 15 min. The thus-obtained ethyl acetate phase was concentrated to dryness. A part of the concentrated dry product was subjected to high performance liquid chromatography analysis. As a result, a dry product 2 containing the title compound was obtained.

The dry product 1 and the dry product 2 were combined (15.2 g), dissolved in ethyl acetate/acetic acid (100:0.01) (200 mL), and the residue was removed. This ethyl acetate/acetic acid solution was concentrated to dryness under reduced pressure to give 10.8 g. This was divided into two (5.4 g each) and applied to silica gel column chromatography (manufactured by Merck, silica gel 60, 150 g) filled with hexane/ethyl acetate (1:9). Thereafter, a fraction containing the title compound was eluted with 0.9 L of ethyl acetate/2-propanol/distilled water (100:10:5). The fraction was concentrated to dryness under reduced pressure, and a dry product (1.6 g) was obtained by the first fractionation, and a dry product (1.2 g) and a dry product (0.3 g) having a high content of the title compound were obtained. The fraction (0.3 g) having a high content of the title compound, which was obtained by the second fractionation, was dissolved in dimethyl sulfoxide, and applied to Sep-Pak vac C18 cartridge (manufactured by Waters, carrier amount 10 g) substituted by methanol/10 mM ammonium formate solution (pH 3.0) (50:50). Thereafter, a fraction containing the title compound was eluted with 60 mL of methanol/10 mM ammonium formate solution (pH 3.0) (75:25). This fraction was concentrated under reduced pressure, and methanol was evaporated. The residual solution was freeze-dried to give 162 mg of a freeze-dried powder. The obtained freeze-dried powder was divided into two and separated by high performance liquid chromatography (column: CAPCELPAK MGII manufactured by Shiseido Co., Ltd., 50 mm i.d. X 250 mm, mobile phase:acetonitrile/10 mM ammonium acetate (pH 5.0) (50:50), flow rate 50 mL/min, column temperature: room temperature), a fraction containing the title compound was concentrated under reduced pressure, and acetonitrile was evaporated. The residual solution was partitioned by extraction with ethyl acetate, and the ethyl acetate layer was concentrated, and substituted by a small amount of ethanol. Distilled water was added, the mixture was concentrated under reduced pressure, and ethanol was evaporated. This solution was freeze-dried to give the title compound (148 mg) as a freeze-dried powder.
¹H NMR (DMSO-d₆) δ 1.12 (6H, s), 2.19 (2H, s), 3.34 - 3.39 (2H, m), 4.41 (2H, t, J=7 Hz), 4.68 (1H, br. s.), 7.20 (1H, s), 7.21 - 7.25 (2H, m), 7.31 (1H, d, J=9 Hz), 7.48 (1H, d, J=8 Hz), 7.62 (1H, t, J=8 Hz), 7.81 (1H, dd, J=9, 2 Hz), 8.05 (1H, d, J=2 Hz), 8.28 (1H, t, J=6 Hz), 8.36 (1H, s), 9.10 (1H, br. s.), 9.17 (1H, br. s.)

### Industrial Applicability

A pharmaceutical agent comprising (1) a HER2 inhibitor having a pyrrolopyrimidine skeleton or pyrazolopyrimidine skeleton, and (2) not less than one pharmaceutical agent selected from an mTOR inhibitor, a PI3 kinase inhibitor and a cMet inhibitor in combination shows a more significant effect than use thereof as a single agent and other combination pharmaceutical agents, and is useful as a safe agent for the prophylaxis or therapeutic of cancer. In addition, a thymidine synthase production inhibitor comprising a compound having a pyrrolopyrimidine skeleton or pyrazolopyrimidine skeleton, which is represented by the aforementioned formula (I), is useful as a single agent or a safe agent for the prophylaxis or therapeutic of cancer.

This application is based on application No. 2008-052615 filed in Japan, the contents of which are incorporated hereinto by reference.

## Claims

1. A pharmaceutical agent comprising (1) a HER2 inhibitor having a pyrrolopyrimidine skeleton or pyrazolopyrimidine skeleton and (2) not less than one pharmaceutical agent selected from an mTOR inhibitor, a PI3 kinase inhibitor and a cMet inhibitor in combination.

2. The pharmaceutical agent of claim 1, wherein the HER2 inhibitor having a pyrrolopyrimidine skeleton or pyrazolopyrimidine skeleton is a compound represented by the formula: 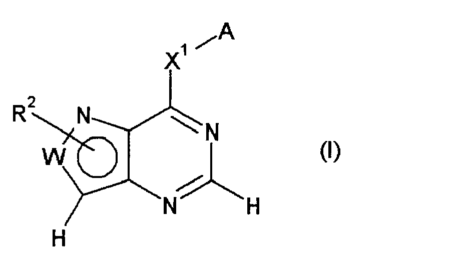 wherein
W is C(R¹) or N,
A is an optionally substituted aryl group or an optionally substituted heteroaryl group,
X¹ is -NR³-Y¹-, -O-, -S-, -SO-, -SO₂- or -CHR³-
wherein R³ is a hydrogen atom or an optionally substituted aliphatic hydrocarbon group, or R³ is optionally bonded to a carbon atom or a hetero atom on the aryl group or the heteroaryl group for A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and
R² is a hydrogen atom or an optionally substituted group bonded via a carbon atom or a sulfur atom,
or R¹ and R², or R² and R³ are optionally bonded to each other to form an optionally substituted ring structure, except compounds represented by the formulas 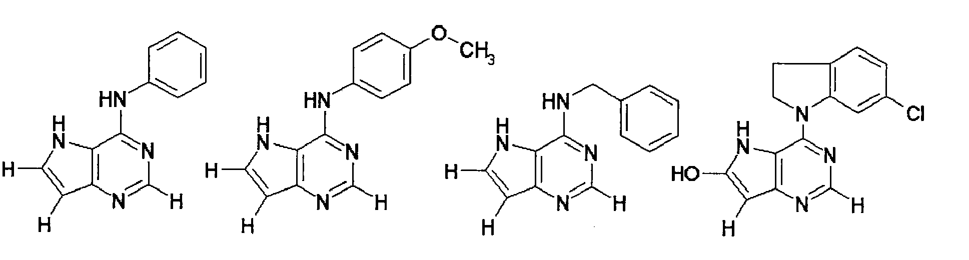 or a salt thereof or a prodrug thereof.

3. The pharmaceutical agent of claim 1, wherein the HER2 inhibitor having a pyrrolopyrimidine skeleton or pyrazolopyrimidine skeleton is a compound represented by the formula: 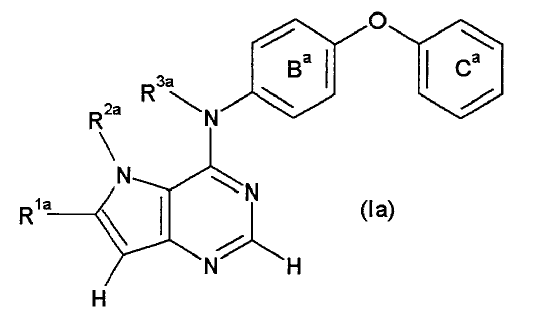 wherein
R^{1a} is a hydrogen atom or an optionally substituted group bonded via a carbon atom, a nitrogen atom or an oxygen atom,
R^{2a} is an optionally substituted group bonded via a carbon atom or a sulfur atom,
or R^{1a} and R^{2a}, or R^{2a} and R^{3a} are optionally bonded to each other to form an optionally substituted ring structure,
R^{3a} is a hydrogen atom or an optionally substituted aliphatic hydrocarbon group, or R^{3a} is optionally bonded to a carbon atom of the adjacent phenyl group to form an optionally substituted ring structure,
B^{a} is an optionally substituted benzene ring, and
C^{a} is an optionally substituted C₆₋₁₈ aryl group, or a salt thereof or a prodrug thereof.

4. The pharmaceutical agent of claim 1, wherein the HER2 inhibitor having a pyrrolopyrimidine skeleton or pyrazolopyrimidine skeleton is N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutaneamide or a salt thereof.

5. The pharmaceutical agent of claim 1, wherein the mTOR inhibitor is rapamycin.

6. The pharmaceutical agent of claim 1, wherein the PI3 kinase inhibitor is PI-103.

7. The pharmaceutical agent of claim 1, wherein the cMet inhibitor is PF2341066.

8. The pharmaceutical agent of claim 1, which is an agent for the prophylaxis or treatment of cancer.

9. The pharmaceutical agent of claim 8, wherein the cancer is breast cancer, ovarian cancer, prostate cancer, lung cancer, pancreatic cancer, kidney cancer, colorectal cancer, small intestinal cancer, esophagus cancer or gastric cancer.

10. A method for the prophylaxis or treatment of cancer in a mammal, comprising administering (1) an effective amount of a HER2 inhibitor having a pyrrolopyrimidine skeleton or pyrazolopyrimidine skeleton, and (2) an effective amount of not less than one pharmaceutical agent selected from an mTOR inhibitor, a PI3 kinase inhibitor and a cMet inhibitor to the mammal.

11. Use of (1) a HER2 inhibitor having a pyrrolopyrimidine skeleton or pyrazolopyrimidine skeleton, and (2) not less than one pharmaceutical agent selected from an mTOR inhibitor, a PI3 kinase inhibitor and a cMet inhibitor, for the production of an agent for the prophylaxis or treatment of cancer.

12. A thymidine synthase production inhibitor comprising a compound represented by the formula: 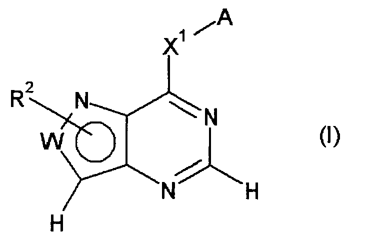 wherein
W is C(R¹) or N,
A is an optionally substituted aryl group or an optionally substituted heteroaryl group,
X¹ is -NR³-Y¹-, -O-, -S-, -SO-, -SO₂- or -CHR³-
wherein R³ is a hydrogen atom or an optionally substituted aliphatic hydrocarbon group, or R³ is optionally bonded to a carbon atom or a hetero atom on the aryl group or the heteroaryl group for A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and
R² is a hydrogen atom or an optionally substituted group bonded via a carbon atom or a sulfur atom,
or R¹ and R², or R² and R³ are optionally bonded to each other to form an optionally substituted ring structure, except compounds represented by the formulas 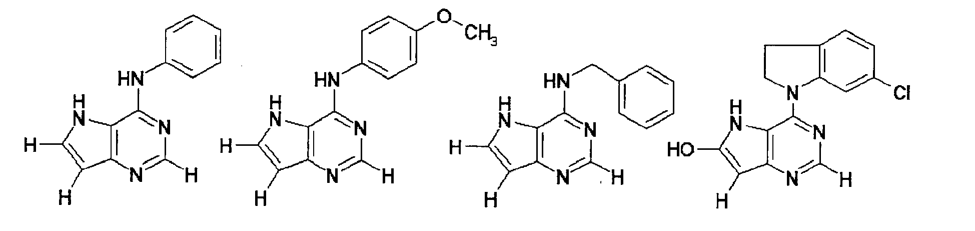 or a salt thereof or a prodrug thereof.

13. A method of inhibiting thymidine synthase production, comprising administering an effective amount of a compound represented by the formula: 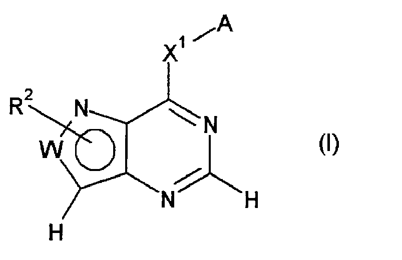 wherein
W is C(R¹) or N,
A is an optionally substituted aryl group or an optionally substituted heteroaryl group,
X¹ is -NR³-Y¹-, -O-, -S-, -SO-, -SO₂- or -CHR³-
wherein R³ is a hydrogen atom or an optionally substituted aliphatic hydrocarbon group, or R³ is optionally bonded to a carbon atom or a hetero atom on the aryl group or the heteroaryl group for A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and
R² is a hydrogen atom or an optionally substituted group bonded via a carbon atom or a sulfur atom,
or R¹ and R², or R² and R³ are optionally bonded to each other to form an optionally substituted ring structure, except compounds represented by the formulas 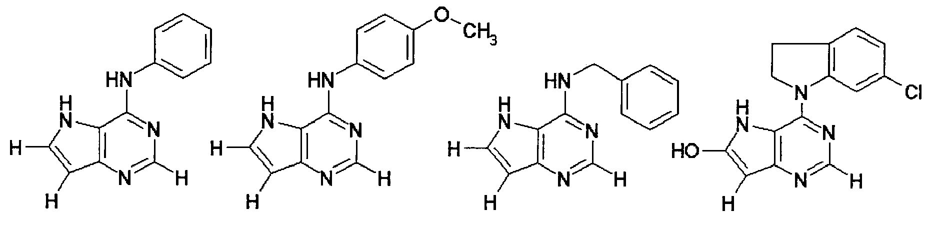 or a salt thereof or a prodrug thereof to a mammal.

14. Use of a compound represented by the formula: 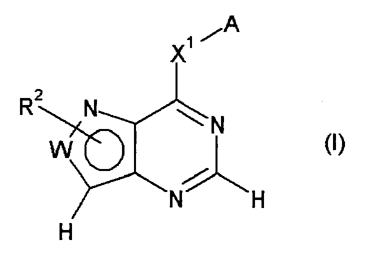 wherein
W is C(R¹) or N,
A is an optionally substituted aryl group or an optionally substituted heteroaryl group,
X¹ is -NR³-Y¹-, -O-, -S-, -SO-, -SO₂- or -CHR³-
wherein R³ is a hydrogen atom or an optionally substituted aliphatic hydrocarbon group, or R³ is optionally bonded to a carbon atom or a hetero atom on the aryl group or the heteroaryl group for A to form an optionally substituted ring structure, and
Y¹ is a single bond or an optionally substituted C₁₋₄ alkylene or an optionally substituted -O-(C₁₋₄ alkylene)-,
R¹ is a hydrogen atom or an optionally substituted group bonded via a carbon atom, a nitrogen atom or an oxygen atom, and
R² is a hydrogen atom or an optionally substituted group bonded via a carbon atom or a sulfur atom,
or R¹ and R², or R² and R³ are optionally bonded to each other to form an optionally substituted ring structure, except compounds represented by the formulas 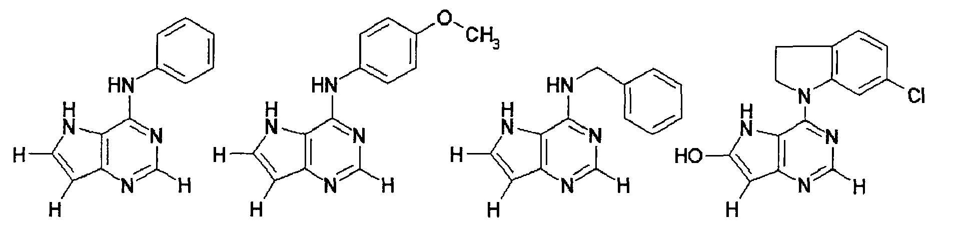 or a salt thereof or a prodrug thereof, for the production of a thymidine synthase production inhibitor.
